(19) Europäisches Patentamt / European Patent Office / Office européen des brevets

(11) EP 2 436 363 B1

(12) # EUROPÄISCHE PATENTSCHRIFT

(45) Veröffentlichungstag und Bekanntmachung des Hinweises auf die Patenterteilung:
**18.01.2017 Patentblatt 2017/03**

(51) Int Cl.:
***A61K 6/00*** *(2006.01)*

(21) Anmeldenummer: **11183338.0**

(22) Anmeldetag: **29.09.2011**

(54) **Zusammensetzung umfassend ein Monomer mit einem polyalicyclischen Strukturelement zum Füllen und/oder Versiegeln eines Wurzelkanals**

Compound comprising a monomer with a polyalicyclic structure element for filling and/or sealing a root canal

Composition comprenant un monomère doté d'un élément structurel polyalicyclique destiné à remplir et/ou sceller un canal radiculaire

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2010 DE 102010041815**

(43) Veröffentlichungstag der Anmeldung:
**04.04.2012 Patentblatt 2012/14**

(73) Patentinhaber: **VOCO GmbH**
**27472 Cuxhaven (DE)**

(72) Erfinder:
- **Blömker, Dr. Tobias**
  **22527 Hamburg (DE)**
- **Stepputtis, Dr. Manfred**
  **27449 Kutenholz (DE)**
- **Maletz, Dr. Reinhard**
  **27472 Cuxhaven (DE)**
- **Plaumann, Manfred Thomas**
  **27472 Cuxhaven (DE)**
- **Fontein, Dr. Nils**
  **27472 Cuxhaven (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**EP-A2- 0 717 976** **DE-A1-102007 029 640**
**US-A- 4 323 348** **US-A- 4 379 695**
**US-B2- 6 734 223**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 436 363 B1

## Beschreibung

Offenbart wird im vorliegenden Text eine härtbare dentale Zusammensetzung umfassend ein Monomer mit einem polyalicyclischen Strukturelement, die zum Füllen und/oder Versiegeln eines Wurzelkanals verwendbar ist. Offenbart wird ferner eine entsprechende Zusammensetzung als Dentalmaterial und ein Verfahren zur Herstellung einer entsprechenden Zusammensetzung. Offenbart werden außerdem polymerisierbare Monomere umfassend mindestens ein polyalicyclisches Strukturelement und bestimmte ethylenische Strukturelemente, die für den Einsatz in einer entsprechenden Zusammensetzung besonders geeignet sind, sowie die Verwendung der offenbarten Monomere in einer entsprechenden Zusammensetzung.

Ziel der definitiven Wurzelkanalfüllung ist die dauerhafte, dreidimensionale, bakteriendichte und hermetische Obturation des aufbereiteten und gereinigten Pulpakavums zum Schutz des Zahnhalteapparats und anatomisch angrenzender Strukturen. Durch den vollständigen Wurzelkanalverschluss wird jeder Transport von Flüssigkeiten unterbunden, so dass im Kanal verbliebene Bakterien keine Möglichkeiten zur weiteren Vermehrung bekommen.

Im Allgemeinen wird eine Wurzelkanalfüllung mit einem semiplastischen Füllmaterial in Kombination mit einem Versiegelungsmaterial vorgenommen. Die Aufgabe des Versiegelungsmaterials ist

- der Ausgleich von Unebenheiten und Inkongruenzen entlang der Kanalwand,

- der Verschluss lateraler Kanäle und offenliegender Dentintubuli, und

- der Aufbau einer dichten Verbindung zwischen dem Füllmaterial und der Wurzelkanalwand.

Die Anforderungen an ein Wurzelkanalversiegelungsmaterial sind dabei äußerst vielfältig und sehr verschieden von einem konventionellen Restaurationsmaterial zum Füllen von Zahnkavitäten.

Das Material sollte leicht in den Wurzelkanal einführbar sein, es sollte in den gesamten Hohlraum einschließlich der feinen Spalten und Nebenkanäle eindringen und diese ausfüllen, es sollte eine exakte und langsame Aushärtungskinetik aufweisen und sollte Volumenstabilität sowie Unlöslichkeit gegenüber Gewebeflüssigkeiten zeigen. Sein Abdichtungsvermögen ist ein entscheidendes Kriterium für die Tauglichkeit des Versiegelungsmaterials, da mit der Hauptfüllmasse ein bakteriendichter Verschluss des Wurzelkanals nicht zu erreichen ist. Das Material sollte zudem röntgenopak und biokompatibel, gewebeverträglich und dauerhaft sein. Es sollte nur wenig schrumpfen, keine Verfärbung des Zahns herbeiführen und, falls nötig, im Zuge einer Revision wieder entfembar sein. Zudem sollte es eine gute Wandständigkeit und gute Adaptation an die Dentinwände aufweisen. Wenn das Wurzelkanalfüll- und/oder -Versiegelungsmaterial in zwei separaten Komponenten (beispielsweise zwei Pasten) vorliegt, die nach Vermischen miteinander aushärten, sollten die beiden Pasten gut miteinander mischbar sein.

Die langsame Aushärtung ist notwendig, damit dem Zahnarzt ausreichend Zeit zur Verfügung steht, eine Röntgenaufnahme zur Kontrolle der Wurzelkanalfüllung anzufertigen und gegebenenfalls eine Ergänzung der Wurzelkanalbehandlung vorzunehmen.

Kompliziert wird ein vollständiger Verschluss des Wurzelkanals durch den Umstand, dass das Wurzelkanalsystem nicht nur aus dem Hauptkanal besteht, sondern auch Verzweigungen, kleine Verästelungen und Seitenkanälchen in unterschiedlichsten Formen aufweist. So können beispielsweise Zerfallsprodukte der erkrankten Pulpa und Abbau- und Stoffwechselprodukte der an der Erkrankung beteiligten Bakterien sowohl über den Hauptkanal in die periapikale Region als auch über Seitenkanälchen ins Desmodont gelangen. Auf diesem Weg kann eine bereits abgeklungene Entzündung wieder in die akute Phase gebracht werden. Da Bakterien und deren Metabolite neben den Seitenkanälchen auch die kleinsten Verästelungen besiedeln und sogar noch weiter bis tief in die Dentinkanälchen eindringen, ist eine Sterilisierung des Kanals trotz gründlicher mechanischer Aufbereitung und sorgfältiger chemischer Desinfektion durch Spülung nicht zu erzielen.

Das Durchsickern kontaminierter Flüssigkeit erfolgt nicht nur vom Kanalsystem nach außen, sondern kann auch von außen in den Kanal hinein geschehen. Überdies können Mikroorganismen, flüssige Speisereste sowie Speichel über die Mundhöhle durch den koronalen Eingang eines unzureichend gefüllten Wurzelkanals in den Kanal gelangen und diesen infizieren.

Einzig eine dicht hergestellte Wurzelkanalfüllung kann die Penetration pathogener Substanzen verhindern und die im Kanal verbliebenen Spezies gegen die Umgebung isolieren, so dass eine Substratzufuhr blockiert ist. In diesem Zusammenhang wurde mehrfach festgestellt, dass in einem dicht verschlossenen Wurzelkanal Bakterien sogar absterben.

Als Material der Wahl zum bakteriendichten Verschluss des Wurzelkanalsystems wird zurzeit als Stand der Technik Guttapercha in Kombination mit einem Versiegelungsmaterial (Sealer) benutzt. Guttapercha wird in den aufbereiteten Wurzelkanal in Form genormter Stifte eingepasst. Die Guttapercha-Stifte bestehen aus Polyisopren. Der Stift und die Kanalwand werden mit dem Sealer beschichtet und der Stift wird dann in den Kanal eingesetzt. Verbliebene

leerräume können durch weitere Stifte oder weitere Mengen an Sealer aufgefüllt werden. Mit entsprechenden Instrumenten werden der vorgeformte Stift und das Versiegelungsmaterial der Kanalwand angepasst.

In der Vergangenheit wurden die unterschiedlichsten Versiegelungs-/Füllungsmaterialien vorgeschlagen.

Die DE 24 20 351 C3 beschreibt die Verwendung einer Zusammensetzung als Wurzelkanalfüllkomponente, die ein hydrophiles, polymerisierbares Material aus Hydroxy-Nieder-Alkylacrylat und/oder Hydroxy-Nieder-Alkylmethacrylat zur Bildung eines Homopolymerisats oder Mischpolymerisats mit einem Anteil von etwa 1 bis 50% an Alkylacrylaten bzw. Methacrylaten und einen aktiven Füllstoff sowie gegebenenfalls ein feinteiliges Röntgenstrahlen-undurchlässiges Material enthält. In den Beispielen der Patentschrift wird in einer Komponente der chemisch härtenden Zusammensetzung als Hydroxy-Nieder-Alkylmethacrylat das Hydroxyethylmethacrylat (HEMA) eingesetzt, während die zweite Komponente, die das Peroxid enthält, eine große Menge eines nichtreaktiven Verdünnungsmittels wie beispielsweise Glyzerindiacetat, umfasst.

Bei der radikalischen Vernetzung von Methacrylat/Acrylatzusammensetzungen entsteht ein dreidimensional verknüpftes Netzwerk. Aufgrund der sehr geringen Größe des Wassermoleküls kann Wasser in die Maschen des Polymerisats eindiffundieren, wo es sich an bestimmten Stellen im Netzwerk anlagert und dort Wasserstoffbrücken, bzw. andere schwach polare Bindungen ausbildet. Je mehr polare Anteile in der Polymermatrix vorhanden sind, umso leichter kann eine weitere Wasseraufnahme erfolgen. Durch die Wasseraufnahme weitet sich das Polymerisat auf und es kann zu einer strukturellen Reorganisation der Polymerketten kommen. Die entstehende Druckspannung kann die Zahnhartsubstanz nachhaltig schädigen. Darüberhinaus können Wassermoleküle empfindliche Strukturelemente des Polymerisats, wie beispielsweise Estergruppen, angreifen und hydrolytisch spalten. Dieser Abbau kann zur völligen Disintegration des Netzwerks führen.

Es ist zu erwarten, dass Zusammensetzungen, wie sie in der DE 24 20 351 C3 beschrieben sind, aufgrund der relativ großen Menge sowohl an HEMA als auch an nichtreaktivem Verdünnermonomer viel Wasser aufnehmen werden. In dem Patentdokument selbst wird auf den stark hydrophilen Charakter der Zusammensetzung hingewiesen. Die hohe Wasseraufnahme, die durch die Wurzelspitze erfolgen soll, soll das Volumen des Polymerisats ausdehnen und so den gesamten Hohlraum des Kanals versiegeln. Wie oben jedoch dargelegt, kann die entstehende Druckspannung die Zahnhartsubstanz eher schädigen und es ist sehr wahrscheinlich, dass durch die Gegenwart hoher Menge an Wasser das Polymerisat irreversibel hydrolytisch aufgebrochen und abgebaut wird.

Im Gegensatz zu den Zusammensetzungen aus DE 24 20 351 C3, die niedermolekulare Methacrylate umfassen, offenbart DE 10 2007 029 640 A1 polymerisierbare Wurzelkanalfüllungs- und Versiegelungsmaterialien, die höhermolekulare Dimethacrylate, Härtungsmittel und röntgenopak-machende Zusätze enthalten. Die eingesetzten höhermolekularen langkettigen Dimethacrylate sollen ein mittleres Molekulargewicht von mehr als 600 aufweisen und in Mengen von 2 -90 Gew.-%, besonders bevorzugt in Mengen von 15 - 30 Gew.-% in den Zusammensetzungen enthalten sein. Bevorzugt werden Dimethacrylatverbindungen mit hydrophilen Eigenschaften wie z.B. $-CH_2-O-$ oder $-CH_2-CH_2-O-$ und/oder OH-, $NH_2$- Gruppen. Besonders bevorzugt sind $-CH_2-CH_2-O-$Einheiten. Höhermolekulare Dimethacrylate mit polyalicyclischem Strukturelement sind in DE 10 2007 029 640 A1 nicht offenbart.

Vorzugsweise werden in DE 10 2007 029 640 A1 Dimethacrylatverbindungen mit mehreren hydrophilen Einheiten verwendet. Bevorzugt sind Verbindungen mit 10 hydrophilen Einheiten, besonders bevorzugt mit 20 hydrophilen Einheiten, ganz besonders bevorzugt welche mit 30 hydrophilen Einheiten. Beispielhaft benannt sind in der Anmeldung ethoxyliertes Bisphenol A Dimethacrylat E (30) mit einem Molekuklargewicht von 1678, ethoxyliertes Bisphenol A Dimethacrylat E (10) mit einem Molekulargewicht von 804, Polyalkylenglykoldimethacrylat EP 100 mit einem Molekulargewicht von 1114 oder Polyethylenglykol 600 Dimethacrylat mit einem Molekulargewicht von 754.

Allen diesen Verbindungen ist eigen, dass sie über hochflexible Molekülketten verfügen, da beispielsweise die Ethoxygruppe um ihre Etherbindung herum ungehindert rotieren kann. Dieser Umstand bedingt, dass die Packung und Anordnung der Molekülketten im Polymerisat nicht homogen und starr, sondern beweglich und offen ist. Es ist daher zu erwarten, dass auch die Zusammensetzungen aus der DE 10 2007 029 640 A1 ebenso wie die aus der DE 24 20 351 C3 große Mengen an Wasser aufnehmen. Dies wird gleichermaßen zu einer schädigenden Druckspannung auf die Zahnsubstanz führen. Weiterhin wird die vom Polymerisat ausgenommene Wassermenge das Netzwerk hydrolytisch spalten und irreversibel abbauen.

Die US 2009/0131552 offenbart ein Wurzelkanalversiegelungsmaterial, das ein lichthärtbares Urethan-monoacrylatoligomer mit einer Acrylat- und einer Hydroxylgruppe an beiden Enden, wenigstens ein di- oder multifunktionelles Acrylat / Methacrylatverdünnungsmonomer und wenigstens einen Initiator umfasst. Das Oligomer ist das Produkt aus Isophorondiisocyanat mit einem Acrylat, das eine Hydroxylgruppe aufweist, in einer ersten Umsetzungsstufe. Hierbei reagiert beispielsweise 1 mol HEMA mit einem mol Isophorondiisocyanat in einer Isocyanat-Alkohol Polyaddition. In einem zweiten Schritt wird die noch am alicyclischen Rest verbliebene Isocyanatgruppe in einer 2. Umsetzungsstufe beispielsweise mit einem Polyesterpolyol wie Polybutyleneadipatglycol abreagiert. Es entsteht so ein Urethanmonomethacrylat mit einem polyalicyclischen Rest. Gegenüber den Strukturen aus der DE 10 2007 029 640 A1 und der DE 24 20 351 C3 liegen in diesem Strukturgerüst somit zum einen Polyurethangruppen vor, die höchst geeignet sind über intermolekulare Wechselwirkungen beständige Überstrukturen auszubilden, die für einen relativ festen Molekülkettenverbund im Polymerisat sorgen, und zum anderen weist dieser Strukturtyp einen alicyclischen Rest auf, der dem Polymerisat hydrophobe Eigenschaften verleiht. Nachteilig an diesem System ist der Umstand, dass das Urethanmonomethacrylat lediglich Molekülketten bilden kann und nicht vemetzbar ist. Da trotz sterischer Hinderung zu erwarten ist, dass der alicyclische Ring seine Konformation ständig ändert, befinden sich auch in diesem System flexible Strukturelemente, die ein Eindringen von Wassermolekülen in das Polymerisat wahrscheinlich machen. Auch dieses System wird somit Wasser in nicht unerheblichem Maße aufnehmen, eine schädigende Druckspannung auf die Zahnsubstanz ausüben und im Laufe kurzer Zeit hydrolytisch abgebaut werden.

Die DE 10 2005 041 115 A1 beschreibt ein Wurzelkanalabdichtungsmaterial, das den durch die radikalische Polymerisation bedingten Volumenschrumpf der härtenden dentalen Masse teilweise ausgleichen oder eliminieren soll. Dies soll mit einer ein Gas während der Aushärtung freisetzenden Komponente erfolgen, so dass sich das Volumen der Zusammensetzung durch Bildung kleiner Gasblasen ausdehnen und den Volumenschrumpf kompensieren soll. Als ein Gas freisetzende Komponente wird beispielsweise ein Säure/Base Paar, wie Zitronensäure/Natriumbicarbonat, eingesetzt. Das Säure/Base Paar wird getrennt in zwei Pasten formuliert. Werden die Pasten vermischt, wird das Bicarbonat durch die Säure unter Bildung von Kohlensäure protoniert, die sich dann unter Bildung von $CO_2$ und Wasser zersetzt. Andere ein Gas freisetzende Komponenten sind beispielsweise Azo(bis)-isobutyronitril, eine Verbindung, die Stickstoff abgeben kann oder die Kombination von Isocyanat und Wasser, wobei bei Reaktion ein Amin und $CO_2$ entstehen und das Amin mit weiterem Isocyanat zu Harnstoffderivaten abreagiert. Problematisch an diesem Konzept ist eine unkontrollierte Gasfreisetzung sowie die Möglichkeit, dass die Gasblasen keine geschlossenen und voneinander isolierten Zellen darstellen, sondern miteinander verbunden oder offen sind und so Bakterien und anderen Kontaminanten einen Weg ins Zahngewebe eröffnen.

In der DE 695 18 030 T2 wird eine Zahnfüllmasse zum Versiegeln des Wurzelkanals auf der Basis Epoxid/Amin vorgestellt. Die Umsetzung eines Diepoxydmonomers mit einem primären Monoamin und/oder disekundären Diamin soll ein thermoplastisches lineares Polymer ergeben, das geeignet sein soll, den Wurzelkanal zu versiegeln. Neben der starken Exothermie der Epoxid/Amin Polyaddition und dem damit einhergehenden hohen Schrumpfverhalten sind sowohl Epoxide wie Amine, die in stöchiometrischen Mengen vorliegen, insbesondere aufgrund ihres toxischen Profils als eher problematische Materialien anzusehen, um im direkten Kontakt mit menschlichem Gewebe zu stehen.

In der US 6,734,223 B2 werden Dentalmassen vorgeschlagen, die vorzugsweise als Wurzelkanalfüllmaterial verwendbar sind und ein polymerisierbares Monomer, einen Polyaminoester bestimmter Struktur, Pigmente, organische und/oder anorganische Füllstoffe, Initiatoren und Stabilisatoren aufweisen. Anstelle der in der DE 695 18 030 T2 als Umsetzungspartner in der Polyaddition eingesetzten primären Monoamine und sekundären Diamine werden hier Polyaminoester, hergestellt aus Mono-, Di- oder Polyaminen und Bis- oder Polyacrylaten, benutzt, die dann in einer stufenweisen Polymerisatiom vorzugsweise mit Di- oder Polyepoxiden oder Di- oder Polyisocyanaten ausgehärtet werden. Als polymerisierbares Monomer wird in US 6,734,223 B2 u.a. 3,(4),8,(9)-Dimethacryloyl(oxymethyl)tricyclo[5.2.1.0$^{2,6}$]decan angegeben.

Einen ähnlichen Ansatz wie in DE 695 18 030 T2 und US 6,734,223 B2 findet man in US 2008/0287566 A1. Als Wurzelkanalversiegelungsmaterial wird das Reaktionsprodukt aus einem Diepoxidoligomer, das eine Mischung aus Bisphenol A Diepoxidoligomeren und/oder Bisphenol F Diepoxidoligimeren umfasst, mit Polyamin/-amidmonomeren beschrieben. Hierbei weisen die Polyaminmonomeren zwei Polyaminregionen auf, die über eine Kohlenwasserstoffregion strukturell miteinander verknüpft sind. Die Kohlenwasserstoffregion, die substituiert sein kann, umfasst wenigstens 28 Kohlenstoffatome. In der Druckschrift wird vorgeschlagen, dieses Material auch zur Vorbeschichtung der Guttapercha-Stifte zu nutzen, um die Bindung zwischen den Stiften und dem Versiegelungsmaterial zu verbessern. Da Guttapercha aus Polyisopren besteht, soll die mehr hydrophobe Kohlenwasserstoffregion im Epoxidpolymerisat eine bessere Kompatibilität zwischen Stift und Versiegelungsmaterial gewährleisten.

EP 1 547 571 B1 beschreibt Zahnwurzelkanalfüllmassen umfassend aminoterminierte Prepolymere.

DE 601 25 374 T2 offenbart Dentalmassen auf der Basis von Bisacrylamiden, die vorzugsweise als Zahnwurzelkanalfüllmaterialien verwendbar sind und eine verbesserte Hydrolysestabilität aufweisen sollen.

In den Druckschriften US 7,320,598 B2, US 6,811,400 B2, US 6,500,004 B2 und US 6,652,282 B2 werden Wurzelkanalversiegelungsmaterialien beansprucht, die eine hohe Haftung zur Zahnhartsubstanz aufweisen sollen. Die Zusammensetzungen enthalten sogenannte Haftmonomere, insbesondere Oxyphosphoralkylmethacrylate. Besonders bevorzugt soll die Verwendung von Bisglyceroldimethacrylatphosphat sein. Weitere geeignete Haftmonomere zur Verwendung in Zusammensetzungen für den Wurzelkanal sollen das Bis-2-hydroxyethylmethacrylatphosphat, Phosphatester von 3-Hydroxypropylmethacrylat und Phosphatester von 4-Hydroxypropylmethacrylat sein.

In US 2008/0200586 werden Versiegelungsmaterialien, u.a. für den Wurzelkanal, beschrieben, die sich ausdehnen oder zusammenziehen sollen sowie die Form verändern können. Ein solches Verhalten soll durch vorzugsweise polymere Teilchen, die zunächst vorbelastet und dann relaxiert werden, ausgelöst werden können. Eine Zusammensetzung, die solche Partikel enthält, soll eine vollständige Abdichtung selbst in einem ungleichmäßig geformten Wurzelkanal bewerkstelligen.

In US 5,646,197 wird ein antimikrobielles Wurzelkanalfüllmaterial auf der Basis von Zinkoxid und hydriertem

Kolophoniumester offenbart.

US 4,449,938 betrifft eine endodontische Füll- und Versiegelungszusammensetzung auf der Basis von Polysiloxanen.

Aus EP 0 864 312 ist ein Kit bekannt, umfassend zwei Komponenten, die nach Vermischen miteinander aushärten und als Wurzelkanalfüllungsmaterial geeignet sind. Das Material härtet in einer Additionsreaktion der beiden Komponenten zu einem Polysiloxan aus, wobei die eine Komponente ein oder mehrere Silikonöle umfasst, die mindestens zwei SiH-Gruppen aufweisen und die andere Komponente ein oder mehrere Silikonöle umfasst, die mindestens zwei Vinylgruppen aufweisen und eine der beiden Komponenten noch zusätzlich einen Katalysator enthält.

DE 1 139 940 A zielt auf eine Kombination von Grundstoffen zur Herstellung von Zahnwurzelfüllmassen, die aus hydroxylendblockierten linearen Diorganopolysiloxanen und Organotriacylsiloxanen besteht.

US 2008/0206716 A1, WO 2008/100451 A2 und WO 2008/100452 A2 beschreiben wasserbasierende Systeme zum Füllen des Wurzelkanals.

Aus US 2008/0299093 A1 ist ein hydraulischer Zement zum Füllen eines Wurzelkanals entnehmbar.

Obwohl es viele verschiedene Systeme zum Füllen und Versiegeln eines Wurzelkanals gibt, sind die Anforderungen an ein solches Material so vielfältig und komplex, dass es bis heute kein Material geschafft hat, sämtliche Anforderungen zufriedenstellend zu erfüllen.

Eine äußerst wichtige Eigenschaft von Versiegelungsmaterialien für den Wurzelkanal ist eine möglichst geringe Wasseraufnahme des Materials. Bei der radikalischen Vernetzung von Methacrylat/Arylatzusammensetzungen entsteht ein dreidimensional verknüpftes Netzwerk. Aufgrund der sehr geringen Größe des Wassermoleküls kann Wasser in die Maschen des Polymerisats eindiffundieren, wo es sich an bestimmten Stellen im Netzwerk anlagert und dort Wasserstoffbrücken, bzw. andere schwach polare Bindungen ausbildet. Je mehr polare Anteile in der Polymermatrix vorhanden sind, umso leichter kann eine weitere Wasseraufnahme erfolgen. Durch die Wasseraufnahme weitet sich das Polymerisat auf und es kann zu einer strukturellen Reorganisation der Polymerketten kommen. Die entstehende Druckspannung kann die Zahnhartsubstanz nachhaltig schädigen. Darüberhinaus können Wassermoleküle empfindliche Strukturelemente des Polymerisats, wie beispielsweise Estergruppen, angreifen und hydrolytisch spalten. Dieser Abbau kann zur völligen Disintegration des Netzwerks führen.

Andererseits ist eine gewisse Polarität der Versiegelungsmaterialien jedoch auch wünschenswert, da die Zahnhartsubstanz hydrophil ist und eine polare Zusammensetzung eine gute Adaptation des Materials am Zahnsubstrat gewährleistet. Durch die Aufbereitung des Wurzelkanals und Entfernung des Endodonts ist der Zahn jedoch tot, so dass die hydrophile Umgebung im Wurzelkanal reduziert ist. Eine gute Benetzbarkeit des Substrats sollte somit auch mit mäßig hydrophilen Strukturen zu erreichen sein.

Wurzelkanalversiegelungsmaterialien sollten einen möglichst geringen Schrumpf während des Aushärtens aufweisen. Als Schrumpf wird die Dichteänderung oder Volumenschwindung der Reaktionsharzmasse bezeichnet. Sie hängt in erster Linie von der Anzahl der abreagierten funktionellen Gruppen ab. Der Schrumpf tritt sowohl im flüssigen Zustand, also ganz zu Beginn der Polymerisation als auch während und nach der Gelierung auf. Der Gesamtschrumpf wird in einen physikalischen und einen chemischen Anteil unterteilt. Während der physikalische Schrumpf richtungsbestimmt ist und räumlich von den Außenbereichen des Polymerisats analog und im Einklang mit dem aufgebauten Temperaturgefälle nach innen zum Mittelpunkt des aushärtenden Formstoffs verläuft, ist der chemische Anteil nicht richtungsorientiert, resultiert einzig aus der Polymerbildung und ist stark von den geometrischen Konformations- und Konfigurationsparametern des neu aufgebauten Makromoleküls abhängig. Hierbei kommt es im Verlauf der Polymerisation zu einer Annäherung der monomeren Bausteine von einem Van-der-Waals Abstand zum Abstand einer kovalenten Bindung. Darüberhinaus ist die Packungsdichte der Polymerketten höher als die Packungsdichte der Monomeren.

Der Schrumpf kann somit dazu führen, dass es zu einer Randspaltenbildung zwischen dem ausgehärteten Komposit und der Zahnwand kommt. Ein solcher Umstand kann zum Eindringen von Bakterien und kontaminierter Flüssigkeiten in den Wurzelkanal führen. Ein erneuter Entzündungsherd wird dann generiert, da ein bakteriendichter Verschluss des Wurzelkanals nicht erreicht wird.

Dem Schrumpf wird im Allgemeinen mit einem hohen Füllstoffgehalt in der Kompositrezeptur entgegengewirkt, da die gängigen dentalen Füllstoffe ihr Volumen nicht ändern. Ein hoher Füllstoffgehalt steht jedoch der Forderung an extrem gutem Fließvermögen des Wurzelkanalfüll- und Versiegelungsmaterials entgegen, da der mit dem Material auszufüllender Wurzelkanal zum einen sehr eng und andererseits von hochkomplexer, verästelter Struktur ist.

Vernetzbare Monomere, die ein polyalicyclisches Strukturelement, insbesondere ein Tricyclo[52.1.0$^{2,6}$]decan (TCD) - Strukturelement, aufweisen, sind als Bestandteile dentaler Zusammensetzungen an sich bereits bekannt.

So werden beispielsweise in DE 29 31 926 die Acryl- oder Methacrylsäureester des oxyalkylierten Bis(hydroxymethyotdcyclo[5.2.1 0.$^{2,6}$]decans als Bindemittel für Dentalmassen vorgeschlagen. Ausgehend vom Umsetzungsprodukt des kommerziell erhältlichen Bis(hydroxymethyl)tricyclo[5.2.1.0$^{2,6}$]decans mit Alkylenoxiden und anschließende Veresterung mit Acryl- oder Methacrylsäure lassen sich auf einfachem Weg Monomere, die ein tricyclisches Kohlenwasserstoffstrukturelement aufweisen, darstellen. Diese Verbindungen sollen vorzugsweise auf dem Dentalgebiet eingesetzt werden, beispielsweise zur Herstellung von Zahnfüllmitteln, Zahnreparaturmaterialien, Überzugsmassen, Versiegelungsmassen für Kavitäten, Kronen-, Brücken- und Verblendmaterialien, Prothesenmaterialien, künstlichen Zähnen und orthodontischen Vorrichtungen. Im Ausführungsteil der DE 29 31 926 werden zahlreiche Beispiele zur Herstellung dentaler Pasten angegeben. Wurzelkanalmaterialien sind weder in der Beschreibung erwähnt, noch gibt es Beispiele für Zusammensetzungen, die zum Einsatz als Wurzelkanalmaterial geeignet wären.

In der DE 28 16 823 werden die Bismethacrylsäureester des Dihydroxymethyltricyclo[5.2.1.0.$^{2,6}$]decans [= Bis(hydroxymethyl)tricyclo[5.2.1.0.$^{2,6}$]decan] als besonders geeignete polymerisierbare Komponenten für Dentalmaterialien beschrieben, da sie besonders dimensionsstabile und harte Polymerisate ergeben. Verwendet werden sie beispielsweise als Zahnfüllmittel, für Präparate zum Aufbau fehlender Zahnteile, als Überzugs- und Versiegelungsmassen, als Grundiermaterialien für Kavitäten, als Kronen-, Brücken- und Verblendmaterialien, als Massen zur Herstellung künstlicher Zähne, als Prothesenmaterialien sowie für orthodontische Vorrichtungen. Wurzelkanalmaterialien sind in dieser Schrift nicht genannt. Die zahlreichen Ausführungsbeispiele beschreiben Dentalmaterialien, die zum Einsatz im Wurzelkanal nicht geeignet sind.

In DE 24 06 557 wird der Bisgylycidylether des Dihydroxymethyltricyclo[5.2.1.0.$^{2,6}$]decans mit 2-Hydroxyethylmethacrylat zum entsprechenden Dimethacrylat umgesetzt. Zusammen mit amorpher Kieselsäure sollen die hier beschriebenen Verbindungen als Zahnfüllmaterialien geeignet sein. Die Patentschrift stellt auf die im Vergleich zu den herkömmlichen Monomeren stark erhöhte Wasseraufnahme der Tricyclodecanderivate ab. Eine solche Eigenschaft soll zu einem besseren Randschlussverhalten des Füllmaterials führen. Wurzelkanalmaterialien werden nicht angegeben.

In DE 35 22 006 A1 und DE 35 22 005 A1 werden Methacrylsäurederivate von Tricyclo[5.2.1.0$^{2,6}$]decanen zur Verwendung in Zahnfüllmassen sowie in Beschichtungsmittel für Zähne beansprucht. Die Monomermischungen können ohne Zusatz von Füllstoffen als Beschichtungsmittel (Zahnlacke) eingesetzt werden. Bei der Verwendung als Zahnfüllmasse soll man den erhaltenen Monomermischungen im Allgemeinen Füllstoffe zusetzen. Um einen hohen Füllgrad erreichen zu können, sollen Monomermischungen, die eine Viskosität im Bereich von 60 bis 10000 mPas besitzen, besonders vorteilhaft sein. Wurzelkanalmaterialien werden nicht beschrieben. Die Zusammensetzungen der Ausführungsbelspiele sind nicht für einen Einsatz im Wurzelkanal geeignet.

In DE 37 03 080 A1 werden u.a. Methacrylsäureester beschrieben, die ein räumlich zentral angeordnetes Tricyclo[5.2.1.0.$^{2.8}$]decanstrukturelement aufweisen und als Monomere für Dentalwerkstoffe, beispielsweise als Füllungsmaterialien für Zähne, Beschichtungsmittel für Zähne und Komponenten für die Herstellung von Zahnersatz, bevorzugt Kunststoffzähne, verwendet werden können. Materialien für den Wurzelkanal oder Zusammensetzungen, die im Wurzelkanal eingesetzt werden können, werden nicht angegeben.

In DE 37 03 130 A1 und DE 37 03 120 A1 werden Urethangruppen enthaltende Methacrylsäurederivate von Tricyclo[5.2.1.0.2$^{2,6}$]decanen oder Bicyclo[2.2.1]heptanen als Monomere für Zahnbeschichtungen, Zahnfüllmassen und zur Herstellung von Kunststoffzähnen vorgeschlagen. Wurzelkanalmaterialien werden nicht beschrieben. Die Ausführungsbeispiele betreffen Zusammensetzungen, die nicht für einen Einsatz im Wurzelkanal geeignet sind.

In DE 10 2005 053 775 A1 wird ein dünnfließendes Kompositmaterial beschrieben, das eine Reduzierung der Schrumpfkraft bei Zahnfüllungen herbeiführen soll und als Kavitätenliner Verwendung findet. Bevorzugt eingesetzte Monomere sind u.a. das Bis(methacryloyloxymethyl)tricyclo[5.2.1$^{2,6}$]decan und das Bis(acryloyloxymethyl)tricyclo[5.2.1.0$^{2,6}$]decan. Wurzelkanalmaterialien werden nicht beschrieben. Die Zusammensetzungen der Ausführungsbeispiele sind nicht geeignet im Wurzelkanal eingesetzt zu werden.

DE 10 2007 034 457 A1 betrifft Dentalmaterialien mit geringer Schrumpfspannung und hoher Biegefestigkeit, die als Monomerbestandteile Bis(methacryloyloxymethyl)tricyclo[5.2.1.0.$^{2,6}$]decan und Bis(acryloyloxymethyl)tricyclo[5.2.1.0.$^{2,6}$]decan enthalten und als Füllungswerkstoffe verwendbar sind. Die dentalen Zusammensetzungen weisen einen sehr hohen Füllstoffgehalt auf, der mit für die extrem hohen Werte der Biegefestigkeit verantwortlich ist. Solche Zusammensetzungen sind für Anwendungen im Wurzelkanal nicht geeignet, da sie zum einen aufgrund ihres hohen Füllstoffgehalts nicht in den Kanal eingeführt werden können und zum anderen eine solche Festigkeit aufweisen, dass sie im Zuge einer Revision aus dem Kanal nicht leicht entfernt werden können.

In DE 10 2006 060 983 A1 wird die Verwendung Urethangruppen enthaltender Acrylsäureester des Tricyclo[5.2.1.0.$^{2,6}$]decans in dentalen Kompositen beschrieben. Die Gegenwart des Acrylsäureesters soll die Zytotoxizität der Monomerenmischung gegenüber einer Mischung herkömmlicher Monomere herabsenken.

WO 03/035013 A1 und DE 602 16 951 T2 betreffen Dentalkleberzusammensetzungen zum Binden von Dental-Restaurlerungsmitteln an Dentin und/oder Zahnschmelz. In diesen Dokumenten wird unter anderem die Herstellung von 3,(4),8,(9)-Bis(2-propenamidomethyl)tricyclo[5.2.1.0]$^{2,6}$-decan beschrieben.

Aus dem Stand der Technik ist der Einsatz von Monomeren mit einem polylalicyclischen Strukturelement für Materialien zur Versiegeln und/oder Füllen des Wurzelkanals nicht entnehmbar.

Man unterscheidet die Materialien zur Wurzelkanalversiegelung auf der Basis von Methacrylaten und/oder Acrylaten beispielsweise nach der Art ihrer Aushärtung, die entweder durch Licht- und/oder Autopolymerisation erfolgen kann. Vorzugsweise werden sie dualhärtend eingestellt, da die Lichtquanten der Polymerisationslampe nicht tief genug in den Wurzelkanal eindringen können. Darüberhinaus ist bei dieser Anwendung eine möglichst vollständige Konversionsrate notwendig, damit keine nicht umgesetzten und somit freien Monomere, die bekanntermaßen toxische Eigenschaften aufweisen können, über die Wurzelspitze in lebendes Gewebe eindringen und dort zu Entzündungen führen können. Redoxinduzierte autopolymerislerbare Systeme weisen eine Nachhärtung auf und ihre Umsetzungsrate ist höher als die Konversionsrate rein photovernetzbarer Zusammensetzungen. Zur Wurzelkanalversiegelung sind somit dualhärtbare Systeme klinisch bevorzugt.

Bei Anwendung der Kombination aus Photohärtung und chemischer Autopolymerisation wird die Netzwerkdichte des Polymerisats an der der Polymerisationslampe zugewandten Seite, also zur Mundhöhle hin, größer sein als an der gegenüberliegenden Seite, zur Wurzelkanalspitze hin, da der duale Härtungsmodus am koronalen Eingang des Wurzelkanals am effektivsten wirksam ist. Dieser Effekt ist äußerst wichtig, da der Verschluss des Wurzelkanals zum Mundraum am kritischsten ist.

Relevant in diesem Zusammenhang sind auch die Auswahl der Komponenten der Zusammensetzung und die Kinetik beim Härten, z.B. die kinetische Einstellung des Redoxsystems. Es muss sichergestellt sein, dass sowohl eine für den Zahnarzt akzeptable, d.h. genügend lange Verarbeitungszeit des Materials sichergestellt ist, als auch ein möglichst vollständiger Umsatz der Reaktanden in der Tiefe des Wurzelkanals gewährleistet ist. Das Redoxsystem muss also so beschaffen sein, dass das Polymerisat eine hinreichende innere Festigkeit aufbaut und einen dichten Verschluss des Kanals erzeugt, allerdings darf die Festigkeit nicht zu hoch sein, denn im Falle einer Revision oder bei Verwendung als provisorisches Material muss es mit zahnärztlichen Instrumenten wieder leicht aus dem Kanal entfembar sein.

Primäre Aufgabe der Erfindung war es, eine dentale Zusammensetzung zur Verfügung zu stellen, die sich zum Füllen und/oder Versiegeln eines Wurzelkanals eignet und möglichst viele der folgenden Eigenschaften aufweist:

- eine hohe Wasserbeständigkeit (geringe Wasseraufnahme) und geringe Löslichkeit,
- gute Röntgensichtbarkeit,
- gute Adaptation an den Kavitätenwänden des Zahns,
- sehr gute mechanische Eigenschaften (insbesondere Biegefestigkeit und E-Modul), die sicherstellen, dass der Wurzelkanal- fest verschlossen bleibt, die Zusammensetzung im ausgehärteten Zustand jedoch im Falle einer Revision auch wieder gut entfembar ist,
- eine ausreichende Fließfähigkeit (Flow), um die komplizierten Geometrien der Wurzelkanäle möglichst vollständig auszufüllen.

Dabei sollte die Zusammensetzung ein solches Schrumpfverhalten zeigen, dass die Bildung von Randspalten (wegen der zu vermeidenden Bakterienpenetration) vermieden wird und ein ausreichend dichter Verschluss des Kanals gewährleistet ist.

Die Zusammensetzung soll ferner eine hinreichend lange Verarbeitungszeit aufweisen (ca. 30 bis 120 Minuten ab Herstellung der Zusammensetzung, z.B. unmittelbar nach Vermischen der Komponenten).

Offenbart wird im vorliegenden Text eine härtbare dentale Zusammensetzung bestehend aus oder umfassend:

(a) eine Monomerenkomponente umfassend oder bestehend aus

(a1) ein, zwei oder mehr polymerisierbare Monomere mit einer Molmasse von weniger als 4000 g/mol ausgewählt aus der Gruppe bestehend aus Verbindungen (Monomere) der Struktur $Q(YZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $YZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,
- b ist eine natürliche Zahl ausgewählt aus der Gruppe der natürlichen Zahlen 1, 2, 3 und 4,
- jedes Z bedeutet ein Strukturelement, das unabhängig von etwaigen weiteren Strukturelementen Z ausgewählt ist aus der Gruppe bestehend aus

$$-O-(C=O)-CH=CH_2,\ -O-(C=O)-C(CH_3)=CH_2.$$

$-(C=O)-CH=CH_2$, $-(C=O)-C(CH_3)=CH_2$,

$-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4.

- jedes Y bedeutet ein Strukturelement, welches in der Struktur $Q(YZ_e)_b$ das polyalicyclische Strukturelement Q mit e Strukturelementen Z verbindet, wobei jedes Y unabhängig von etwaigen weiteren Strukturelementen Y gewähft ist oder entfällt,

(a2) ein, zwei oder mehr weitere polymerisierbare Monomere aus der Gruppe bestehend aus Acrylaten und Methacrylaten, vorzugsweise der Gruppe der Methacrylate.

(b) ein oder mehrere Initiatoren und/oder Katalysatoren, und
(c) eine Füllstoffkomponente bestehend aus oder umfassend

(c1) einen, zwei oder mehrere röntgenopake Füllstoffe, sowie gegebenenfalls ein oder mehrere weitere Additive.

Offenbart wird auch eine härtbare dentale Zusammensetzung oder umfassend:

(a) eine Monomerenkomponente umfassend oder bestehend aus

(a1) ein, zwei oder mehr polymerisierbare Monomere mit einer Molmasse von weniger als 4000 g/mol ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,

- b ist eine natürliche Zahl ausgewählt aus der Gruppe der natürlichen Zahlen 1, 2, 3 und 4,

- jedes Z bedeutet ein Strukturelement, das unabhängig von etwaigen weiteren Strukturelementen Z ausgewählt ist aus der Gruppe bestehend aus

$-O-(C=O)-CH=CH_2$, $-O-(C=O)-C(CH_3)=CH_2$,

$-(C=O)-CH=CH_2$, $-(C=O)-C(CH_3)=CH_2$,

$-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jeder Index x bedeutet unabhängig von etwaigen weiteren Indizes x 0 oder 1,

- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,

(b) ein oder mehrere Initiatoren und/oder Katalysatoren, und

(c) eine Füllstoffkomponente bestehend aus oder umfassend

(c1) einen, zwei oder mehrere röntgenopake Füllstoffe,

sowie gegebenenfalls ein oder mehrere sonstige Additive.

Der Gegenstand der vorliegenden Erfindung wird in den Ansprüchen definiert.

Sämtliche nachfolgenden Ausführungen betreffend die Verbindungen der Struktur $Q(YZ_e)_b$ (wobei jedes Y unabhängig von etwaigen weiteren Strukturelementen Y gewählt ist oder entfällt) und die im Zusammenhang mit diesen Verbindungen angegebenen bevorzugten bzw. besonders bevorzugten Ausgestaltungen der vorliegenden Erfindung gelten entsprechend für die Verbindungen der Struktur $Q(Y_xZ_e)_b$ (wobei jeder Index x unabhängig von etwaigen weiteren Indizes x 0 oder 1 bedeutet), und umgekehrt.

Eine erfingdungsgemäß einzusetzende Verbindung der Struktur $Q(Y_xZ_e)_b$ umfasst ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten $Y_xZ_e$ ersetzt sind (wie oben beschrieben), und optional ein, zwei oder mehr der nicht durch Substituenten $Y_xZ_e$ substituierten Wasserstoffatome durch Alkylgruppen, Alkoxygruppen, Halogenatome oder Trifluormethylgruppen substituiert sind. Das polyalicyclische Strukturelement Q wird konstituiert durch Kohlenstoff-Ring-Atome. Kohlenstoffatome außerhalb der Ringe sind Bestandteil von Substituenten.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", tricyclisch", tetracyclisch", "pentacyclisch" und "hexacyclisch" entsprechen dabei der IUPAC-Nomenklatur.

Vorzugsweise bedeutet jedes Y ein Strukturelement, welches in der Struktur $Q(Y_xZ_e)_b$ mit x = 1 das polyalicyclische Strukturelement Q mit e Strukturelementen Z verbindet, wobei jedes Y unabhängig von etwaigen weiteren Strukturelementen Y gewählt ist.

Die erfindungsgemäßen Zusammensetzungen sind aufgrund ihrer überaus geringen Wasseraufnahme und ihrer überaus geringen Löslichkeit sowie ihrer Verarbeitungseigenschaften ganz hervorragend als Wurzelkonalversiegelungs- und -füllmaterialien geeignet.

Zudem sind Adaptation der erfindungsgemäßen Zusammensetzungen an den Kanalwänden sowie die Flow-Eigenschaften ausgezeichnet.

Die erfindungsgemäße Zusammensetzung ist vorzugsweise lichthärtbar, bevorzugt dualhärtend.

Die Summe der Zahlenwerte des Index b und des Index e beträgt vorzugsweise 3, 4, 5, 6, 7 oder 8.

Unter (Meth)acryl ist im Rahmen des vorliegenden Textes sowohl Acryl als auch Methacryl zu verstehen.

Vorzugsweise weisen die polymerisierbare Monomeren der Struktur $Q(YZ_e)_b$, (wie oben definiert und wobei jedes Y unabhängig von etwaigen weiteren Strukturelementen Y gewählt ist oder entfällt) der Komponente (a1) eine Molmasse von weniger als 3000 g/mol auf, bevorzugt von weniger als 2000 g/mol, weiter bevorzugt von weniger als 1500 g/mol.

Die Monomerenkomponente (a) umfasst oder besteht aus

(a1) ein, zwei oder mehr polymerisierbaren Monomeren wie oben definiert und

(a2) ein, zwei oder mehr weiteren polymerisierbaren Monomeren aus der Gruppe bestehend aus Acrylaten und Methacrylaten, vorzugsweise Acrylaten und Methacrylaten mit einem Polyether-Strukturelement, bevorzugt mit einem Polyetherpolyol-Strukturelement,

wobei das Verhältnis der Masse der Komponente (a1) zur Masse der Komponente (a2) im Bereich von 3 : 1 bis 1 : 3, bevorzugt im Bereich von 2 : 1 bis 1 : 2, weiter bevorzugt im Bereich von 3 : 2 bis 2 : 3 liegt.

Bevorzugt ist eine erfindungsgemäße Zusammensetzung (wie oben definiert), wobei die Monomerehkomponente (a)

(a1) ein, zwei oder mehr polymerisierbare Monomere wie oben zu (a1) definiert, und
(a2) ein, zwei oder mehr weitere polymerisierbare Monomere aus der Gruppe bestehend aus Acrylaten und Methacrylaten mit einem Polyether-Strukturelement enthält, wobei keines dieser weiteren polymerisierbaren Monomere eines der Struktur $Q(Y_xZ_e)_b$ gemäß der obigen Definition zu (a1) ist,

wobei das Verhältnis der Gesamtmasse der (a1) polymerisierbaren Monomere gemäß der obigen Definition zu (a1) zur Gesamtmasse der (a2) ein, zwei oder mehr weiteren polymerisierbaren Monomeren aus der Gruppe bestehend aus Acrylaten und Methacrylaten mit einem Polyether-Strukturelement, wobei keines der weiteren polymerisierbaren Monomere eines der Struktur $Q(Y_xZ_e)_b$ gemäß der obigen Definition zu (a1) ist , im Bereich von 2 : 1 bis 1 : 2, weiter bevorzugt im Bereich von 3 : 2 bis 2 : 3 liegt.

Jede Verbindung, die unter die Definition von Komponente (a1) bzw. (a2) fällt, ist für die Zwecke quantitativer Betrachtungen der Komponente (a1) bzw. (a2) zuzuordnen.

In einer bevorzugten erfindungsgemäßen Zusammensetzung umfasst die Monomerenkomponente (a),

- Komponente (a1) in einer Gesamtmenge von 5 bis 48,5 Gew.-%, vorzugsweise von 8 bis 30 Gew.-%, bevorzugt von 10 bis 20 Gew.-%, und/oder
- Komponente (a2) in einer Gesamtmenge von 5 bis 48,5 Gew.-%, vorzugsweise von 8 bis 30 Gew.-%, bevorzugt von 10 bis 20 Gew.-%,

jeweils bezogen auf die Gesamtmasse der Zusammensetzung.

Anders ausgedrückt ist eine erfindungsgemäße Zusammensetzung (wie oben definiert) bevorzugt, wobei die Monomerenkomponente (a),

- als Komponente (a1) eine Gesamtmenge im Bereich von 5 bis 48,5 Gew.-%, vorzugsweise von 8 bis 30 Gew.-%, bevorzugt von 10 bis 20 Gew.-%, polymerisierbare Monomere mit einer Molmasse von weniger als 4000 g/mol umfasst, ausgewählt aus der Gruppe bestehend aus Verbindungen (Monomere) der Struktur $Q(Y_xZ_e)_b$ gemäß der obigen Definition zu (a1), und/oder
- als Komponente (a2) eine Gesamtmenge im Bereich von 5 bis 48,5 Gew.-%, vorzugsweise von 8 bis 30 Gew.-%, bevorzugt von 10 bis 20 Gew.-% an ein, zwei oder mehr weiteren polymerisierbaren Monomeren umfasst, ausgewählt aus der Gruppe bestehend aus Acrylaten und Methacrylaten mit einem Polyether-Strukturelement, vorzugsweise der Gruppe der Methacrylate mit einem Polyether-Strukturelement, wobei keines der weiteren polymerisierbaren Monomere eines der Struktur $Q(Y_xZ_e)_b$ gemäß der obigen Definition zu (a1) ist,

jeweils bezogen auf die Gesamtmasse der Zusammensetzung

Weiter bevorzugt als Monomere der Komponente (a2) sind Polyethylenglykoldi(meth)acrylate mit 4 bis 10 Ethylenoxideinheiten.

Erfindungsgemäße Zusammensetzungen, insbesondere in einer der vorstehend oder nachfolgend als bevorzugt bzw. besonders bevorzugt gekennzeichneten Ausgestaltungen, weisen eine gute Fließfähigkeit (Flow) und im ausgehärteten Zustand einen geringen Schrumpf, eine gute Haftung, eine hohe Hydrolysebeständigkeit, eine geringe Wasseraufnahme, eine geringe Löslichkeit und eine geeignete mechanische Festigkeit (insbesondere Biegefestigkeit und E-Modul) auf. Die genannten Eigenschaften sind insbesondere im Bereich der Dentaltechnik wichtig, insbesondere zum Füllen und/oder Versiegeln eines Wurzelkanals.

Bevorzugt ist eine erfindungsgemäße Zusammensetzung bestehend aus oder umfassend:

(a) eine Monomerenkomponente umfassend oder bestehend aus

(a1) in einer Gesamtmenge von 8 bis 30 Gew.-%, vorzugsweise von 10 bis 20 Gew.-%,

(a2) in einer Gesamtmenge von 8 bis 30 Gew.-%. vorzugsweise von 10 bis 20 Gew.-%,

(b) ein oder mehrere Initiatoren und/oder Katalysatoren, (c) eine Füllstoffkomponente umfassend oder bestehend aus

(c1) einen oder mehrere röntgenopake Füllstoffe in einer Gesamtmenge im Bereich von 30 bis 75 Gew.-%, bevorzugt im Bereich von 40 bis 70 Gew.-%, weiter bevorzugt im Bereich von 50 bis 70 Gew.-%, besonders bevorzugt im Bereich von 55 bis 65 Gew.-%,

sowie gegebenenfalls ein oder mehrere weitere Additive, wobei die Gewichtsprozentangaben jeweils auf die Gesamtmasse der Zusammensetzung bezogen sind.

Weiter bevorzugt ist eine erfindungsgemäße Zusammensetzung bestehend aus oder umfassend:

(a) eine Monomerenkomponente umfassend oder bestehend aus

(a1) in einer Gesamtmenge von 10 bis 20 Gew.-%, wobei die polymerisierbaren Monomeren der Struktur $Q(YZ_e)_b$ (wie oben definiert und wobei jedes Y unabhängig von etwaigen weiteren Strukturelementen Y gewählt ist oder entfällt) eine Molmasse von weniger als 2000 g/mol aufweisen,
(a2) in einer Gesamtmenge von 10 bis 20 Gew.-%, wobei Komponente (a2) Polyethylenglykoldi(meth)acrylate mit 4 bis 10 Ethylenoxideinheiten umfasst oder aus diesen besteht,

(b) ein oder mehrere Initiatoren und/oder Katalysatoren, vorzugsweise gewählt aus der Gruppe bestehend aus Photoinitiatoren, Hydroperoxiden und/oder Thioharnstoffderivaten,

(c) eine Füllstoffkomponente umfassend oder bestehend aus

(c1) einen oder mehrere röntgenopake Füllstoffe in einer Gesamtmenge im Bereich von 40 bis 70 Gew.-%, bevorzugt im Bereich von 50 bis 70 Gew.-%,

sowie gegebenenfalls ein oder mehrere weitere Additive, wobei die Gewichtsprozentangaben jeweils auf die Gesamtmasse der Zusammensetzung bezogen sind.

Besonders bevorzugt ist eine erfindungsgemäße Zusammensetzung bestehend aus oder umfassend:

(a) eine Monomerenkomponente umfassend oder bestehend aus

(a1) in einer Gesamtmenge von 10 bis 20 Gew.-%, wobei die polymerisierbaren Monomeren der Struktur $Q(YZ_e)_b$ (wie oben definiert und wobei jedes Y unabhängig von etwaigen weiteren Strukturelementen Y gewählt ist oder entfällt) eine Molmasse von weniger als 1500 g/mol aufweisen,

(a2) in einer Gesamtmenge von 10 bis 20 Gew.-%, wobei Komponente (a2) Polyethylenglykoldi(meth)acrylate mit 4 bis 10 Ethylenoxideinheiten umfasst oder aus diesen besteht,

(b) ein oder mehrere Initiatoren und/oder Katalysatoren, vorzugsweise gewählt aus der Gruppe bestehend aus Photoinitiatoren, Hydroperoxiden und/oder Thioharnstoffderivaten,

(c) eine Füllstoffkomponente umfassend oder bestehend aus

(c1) einen oder mehrere röntgenopake Füllstoffe in einer Gesamtmenge im Bereich von 50 bis 70 Gew.-%, bevorzugt im Bereich von 55 bis 65 Gew.-%,

(c2) einen oder mehrere nicht röntgenopake Füllstoffe,

sowie vorzugsweise ein, zwei oder mehr weitere Additive ausgewählt aus den Komponenten (d), (e) und (f)

(d) ein oder mehrere Molekulargewichtsregler, vorzugsweise ausgewählt aus der Gruppe bestehend aus α-Terpinen, β-Terpinen, γ-Terpinen, α-Phellandren, β-Phellandren und Terpinolen, Linolsäure, Linolensäure sowie substituierten oder nicht substituierten Cyclohexadienen, vorzugsweise in einer Gesamtmenge im Bereich 0,025 bis 0,75 Gew.-%,

(e) ein oder mehrere Wirkstoffe ausgewählt aus der Gruppe bestehend aus remineralisierenden, therapeutischen, devitalisierenden, desinfizierenden, entzündungshemmenden, antibakteriellen und/oder kariostatischen Wirkstoffen,

(f) ein oder mehrere haftfördemde Additive, vorzugsweise in einer Gesamtmenge im Bereich 0,5 bis 2,5 Gew.-%,

sowie gegebenenfalls ein oder mehrere weitere Additive,
wobei die Gewichtsprozentangaben jeweils auf die Gesamtmasse der Zusammensetzung bezogen sind.

Selbstverständlich gilt für Substanzen, die aufgrund ihrer Struktur gleichzeitig unter die Definition verschiedener Komponenten einer erfindungsgemäßen Zusammensetzung fallen, dass diese Substanzen für die Zwecke quantitativer Betrachtungen jeweils sämtlichen dieser Komponenten zugeordnet werden. Sofern beispielsweise eine erfindungsgemäße Zusammensetzung ein oder mehr weitere Additive der Komponente (f) enthält, und dieses Additiv gleichzeitig unter die Definition von Komponente (a1) oder (a2) fällt, ist dieses Additiv für die Zwecke quantitativer Betrachtungen sowohl der Komponente (a1) bzw. (a2) als auch der Komponente (f) zuzuordnen.

Erfindungsgemäße Zusammensetzungen umfassend einen Molekulargewichtsregler (Komponente (d)) weisen weiter verbesserte mechanische Werte auf, insbesondere wenn dieses Zusammensetzungen zusätzlich einen oder mehrere nicht röntgenopake nicht agglomerierte nanoskalige Füllstoffe (Komponente (c2-a), siehe unten) umfassen. Daneben kann der Wert der Löslichkeit und/oder der Wasseraufnahme dadurch sehr niedrig eingestellt werden, bei gleichzeitig gutem Fließverhalten.

Femer hat sich gezeigt, dass eine erfindungsgemäße Zusammensetzung, insbesondere eine zum Füllen und/oder Versiegeln eines Wurzelkanals geeignete erfindungsgemäße Zusammensetzung, im ausgehärteten Zustand

eine sehr geringe Wasseraufnahme aufweist, vorzugsweise weniger als 50 $\mu g/mm^3$, vorzugsweise weniger als 40 $\mu g/mm^3$ und bevorzugt weniger als 35 $\mu g/mm^3$, besonders bevorzugt weniger als 30 $\mu g/mm^3$. Die Wasseraufnahme wurde dabei gemäß ISO 4049 bestimmt.

Nach Wasserlagerung einer erfindungsgemäßen Zusammensetzung wurden bei den mikroskopischen Untersuchungen keine Randspalten zwischen der Wurzelkanalfüllung und der Wurzelkanalwand beobachtet.

Zudem hat sich gezeigt, dass eine erfindungsgemäße Zusammensetzung, insbesondere eine zum Füllen und/oder Versiegeln eines Wurzelkanals geeignete erfindungsgemäße Zusammensetzung, eine gute Fließfähigkeit (Flow) aufweist, vorzugsweise einen Flow im Bereich von 20 bis 40 mm, im Bereich von 25 bis 35 mm. Der Flow wurde dabei gemäß ISO 6876 bestimmt.

Durch den Zusatz des oder der röntgenopaken Füllstoffe als Bestandteil der Komponente (c1) erhält eine erfindungsgemäße Zusammensetzung eine ausreichende Röntgenopazität. Die Röntgenopazität wird üblicherweise in Aluminiumäquivalenten gemessen und in mm (Al) angegeben (siehe hierzu auch die Beispiele).

Vorzugsweise beträgt die Röntgenopazität einer erfindungsgemäßen Zusammensetzung zumindest 3 mm (Al), vorzugsweise liegt sie im Bereich von 3 bis 20 mm (Al), bevorzugt im Bereich von 4 bis 12 mm (Al). Die Messung der Röntgenopazität erfolgt gemäß ISO 4049.

Obwohl Wurzelkanalversiegelungs- und/oder -füllungsmaterialien wieder leicht aus dem Wurzelkanal entfembar sein sollen, ist eine möglichst abschlussdichte Haftung des Materials am Dentin gewünscht.

Zudem hat sich gezeigt, dass eine zum Füllen und/oder Versiegeln eines Wurzelkanals geeignete erfindungsgemäße Zusammensetzung regelmäßig Haftwerte im Bereich von 1 bis 4 MPa aufweist, vorzugsweise im Bereich von 2 bis 4 MPa, besonders bevorzugt im Bereich von 2 bis 3,8 MPa.

Ein Haftwert im Bereich 1 von 4 MPa ist für den Einsatzzweck im Wurzelkanal, d.h. als Wurzelkanalfüll- und/oder -versiegelungsmaterial regelmäßig ausreichend.

Es hat sich gezeigt, dass eine bevorzugte erfindungsgemäße Zusammensetzung, insbesondere eine zum Füllen und/oder Versiegeln eines Wurzelkanals geeignete erfindungsgemäße Zusammensetzung, eine Biegefestigkeit im Bereich von 5 bis 30 MPa aufweist, vorzugsweise eine Biegefestigkeit im Bereich von 5 bis 15 MPa.

Es hat sich ferner gezeigt, dass eine bevorzugte erfindungsgemäße Zusammensetzung, insbesondere eine zum Füllen und/oder Versiegeln eines Wurzelkanals geeignete erfindungsgemäße Zusammensetzung, ein Elastizitätsmodul (E-Modul) im Bereich von 50 bis 800 MPa aufweist, vorzugsweise im Bereich von 75 bis 300 MPa, bevorzugt ein E-Modul im Bereich von 80 bis 125 MPa.

Für ein Wurzelkanalversiegelungs- und/oder -füllungsmaterial ist eine Biegefestigkeit im Bereich von 5 bis 20 MPa und ein E-Modul von etwa 100 MPa als ideal anzusehen.

Die erfindungsgemäßen Zusammensetzungen sind vorzugsweise so beschaffen, dass sie sich gut aus Zahnstrukturen entfernen lassen und sich somit besonders zum Füllen und/oder Versiegeln des Wurzelkanals eignen.

Eine erfindungsgemäße härtbare dentale Zusammensetzung ist daher vorzugsweise zur Verwendung als Wurzelkanalfüll- und/oder-versiegelungsmaterial geeignet.

Bestandteil (a): Monomerenkomponente

Innerhalb einer erfindungsgemäßen Zusammensetzung besteht die Funktion der Monomerenkomponente (a) darin, eine Matrix zu bilden. Diese Matrix wird gebildet durch Polymerisation, insbesondere radikalische Polymerisation, von ein, zwei oder mehr Monomeren der Struktur $Q(YZ_e)_b$ (wie oben definiert und wobei jedes Y unabhängig von etwaigen weiteren Strukturelementen Y gewählt ist oder entfällt) der Komponente (a1), vorzugsweise zusammen mit einem oder mehreren weiteren Monomeren der Komponente (a2). Die Monomerenkomponente (a) ist vorzugsweise eine Monomerenmischung, die Komponente (a1) sowie Komponente (a2) umfasst oder aus diesen besteht.

Das polyalicyclische Strukturelement Q der Komponente (a1) sorgt dabei für ein sterisch starres und hydrophobes Rückgrat, das oder die Monomere der Komponenten (a2) sorgen für eine gezielte Schwächung der mechanischen Eigenschaften (insbesondere Biegefestigkeit und E-Modul).

Eine Kombination von Komponente (a1) und Komponente (a2) erlaubt ein Feineinstellen der Eigenschaften einer erfindungsgemäßen Zusammensetzung. Eine bewusst herbeigeführte Schwächung der mechanischen Eigenschaften (insbesondere Biegefestigkeit und E-Modul) einer erfindungsgemäßen Zusammensetzung (im ausgehärteten Zustand) wird primär durch die Auswahl und die Menge der eingesetzten Bestandteile und deren Komponenten erreicht, so dass eine erfindungsgemäße Zusammensetzung im ausgehärteten Zustand im Falle einer Revision auch wieder gut aus dem Wurzelkanal entfembar ist. Diese Schwächung kann insbesondere durch die bevorzugten Monomere der Komponente (a2) erreicht werden und/oder durch Einarbeitung eines oder mehrerer Molekulargewichtsregler der Komponente (f) (siehe unten).

Komponente (a1): ein, zwei oder mehr Monomere der Struktur $Q(YZ_e)_b$ mit mindestens einem polyalicyclischen Strukturelement

Komponente (a1) bilden ein, zwei oder mehr Monomere der oben definierten Struktur $Q(YZ_e)_b$ (wie oben definiert und wobei jedes Y unabhängig von etwaigen weiteren Strukturelementen Y gewählt ist oder entfällt), wobei Z vorzugsweise ein Strukturelement bedeutet, das unabhängig von etwaigen weiteren Strukturelementen Z ausgewählt ist aus der Gruppe bestehend aus $-O-(C=O)-CH=CH_2$, $-O-(C=O)-C(CH_3)=CH_2$, $-(C=O)-CH=CH_2$ oder $-(C=O)-C(CH_3)=CH_2$ Bevorzugt sind Verbindungen der Struktur $Q(YZ_e)_b$ (wie oben definiert und wobei jedes Y unabhängig von etwaigen weiteren Strukturelementen Y gewählt ist oder entfällt), wobei Z ausgewählt ist aus der Gruppe bestehend aus $-O0-(C=O)-CH=CH_2$, $-O-(C=O)-C(CH_3)=CH_2$, d.h. solche Verbindungen der Struktur $Q(YZ_e)_b$ (wie oben definiert und wobei jedes Y unabhängig von etwaigen weiteren Strukturelementen Y gewählt ist oder entfällt), die ein, zwei oder mehr Acrylat- und/oder Methacrylatgruppen aufweisen, vorzugsweise zwei oder mehr Acrylat- und/oder Methacrylatgruppen.

Die mit den erfindungsgemäß zu verwendenden Monomeren der Komponente (a1) erhältlichen Polymere und Erzeugnisse weisen eine ausgeprägte Hydrophobie auf, die sich u.a. in einer sehr geringen Wasseraufnahme der Polymere und Erzeugnisse zeigt. Daneben zeichnen sich die unter Verwendung der erfindungsgemäß zu verwendenden Monomere der Komponente (a1) erhältlichen Polymere durch eine geeignete mechanische Stabilität aus, die sich u.a. in einer geeigneten Biegefestigkeit der Polymere zeigt. Die efindungsgemäß zu verwendenden Monomere der Komponente (a1), insbesondere gemäß den besonders bevorzugten Ausgestaltungen und Ausführungsformen, lassen sich zu Polymeren verarbeiten, die im ausgehärteten Zustand sowohl eine geringe Wasseraufnahme als auch eine geeignete Biegefestigkeit aufweisen.

Die Monomere der Komponente (a1) sind mit den weiteren Monomeren der Komponente (a2) copolymerisierbar, wobei die ausgehärteten Polymere bzw. Formstoffe einen geringen Schrumpf, eine gute Haftung auf verschiedenen Untergründen, eine hohe Hydrolysebeständigkeit, eine geringe Wasseraufnahme und eine geeignete mechanische Festigkeit aufweisen. Die genannten Eigenschaften sind insbesondere im Bereich der Dentaltechnik wichtig.

Insbesondere die bevorzugten und besonders bevorzugten erfindungsgemäß zu verwendenden Verbindungen der Komponente (a1) ermöglichen einen hohen Vemetzungsgrad und sind ferner vorzugsweise radikalisch vernetzbar. Wegen ihrer hochfunktionalisierten Struktur weisen sie eine hohe Vemetzungs- und Polymerisationswahrscheinlichkeit auf.

Bevorzugte erfindungsgemäß einzusetzende Verbindungen sind solche, wobei Q ein polyalicyclisches Strukturelement bedeutet, vorzugsweise ein gesättigtes polyalicyclisches Strukturelement, das ausgewählt ist aus der Gruppe bestehend aus bicyclischen und tricyclischen Kohlenwasserstoffresten, wobei vorzugsweise keines der nicht durch Substituenten $YZ_e$ (wie oben definiert und wobei jedes Y unabhängig von etwaigen weiteren Strukturelementen Y gewählt ist oder entfällt) substituierten Wasserstoffatome dieses polyalicyclischen Strukturelements Q substituiert ist.

Sofern eine erfindungsgemäß einzusetzende verbindung zwei oder mehrere polyalicyclische Strukturelemente umfasst, können diese identisch oder verschieden sein.

Besonders bevorzugt sind erfindungsgemäß einzusetzende Monomere $Q(YZ_e)_b$ (wie oben definiert und wobei jedes Y unabhängig von etwaigen weiteren Strukturelementen Y gewählt ist oder entfällt), deren polyalicyclisches Strukturelement Q sich von einem der folgenden tricyclischen Kohlenwasserstoffe ableitet: Tricyclo[52.1.$0^{2,6}$]decan (TCD), Tricyclo[5.2.1.$0^{2,6}$]dec-3-en oder Tricyclo[3.3.1.$1^{3,7}$]decan (Adamantan), d.h. bevorzugt sind erfindungsgemäß einzusetzende Verbindungen, die ein TCD - Gerüst, ein Tricyclo[5.2.1.$02^{2,6}$]dec-3-en - Gerüst oder ein Adamantan-Gerüst aufweisen.

Bei den genannten besonders bevorzugten erfingdungsgemäß einzusetzenden Verbindungen, in denen das Strukturelement Q einen Tricycio[5.2.1.$0^{2,6}$]decan-Rest, einen Tricyclo[5.2.1.$0^{2,6}$]deo-3-en-Rest, einen Tricyclo[3.3.$1^{3,7}$]decan-Rest oder einen Bicyclo[2.2.1]heptan-Rest bedeutet, handelt es sich vorzugsweise um solche mit einem Tricyclo[5.2.1.$0^{2,6}$]decan-Gerüst, einem Tricyclo[5.2.1.$0^{2,6}$]dec-3-en-Gerüst, einem Tricyclo[3.3.1.$1^{3,7}$]decan-Gerüst bzw. einem Bicyclo[2.2.1]heptan-Gerüst, bei dem jeweils keines der nicht durch Substituenten $YZ_e$ (wobei jedes Y unabhängig von etwaigen weiteren Strukturelementen Y gewählt ist oder entfällt) substituierten Wasserstoffatome dieses polyalicyclischen Strukturelements Q substituiert ist.

Besonders bevorzugte erfindungsgemäß einzusetzende Verbindungen sind solche, wobei das Strukturelement Q einen Tricyclo[5.2.1.$0^{2,6}$]decan-Rest, einen Tricyclo[52.1.$0^{2,6}$]dec-3-en-Rest, einen Tricyclo[3.3.1.$1^{3,7}$]decan-Rest oder einen Bicyclo[2.2.1]heptan-Rest bedeutet, ganz besonders bevorzugt bedeutet das Strukturelement Q einen Tricyclo[52.1.$0^{2,6}$]decan-Rest, einen Tricyclo[5.2.1.$0^{2,6}$]dec-3-en-Rest.

Bevorzugt eingesetzt werden Methacrylsäure- oder Acrylsäureester mit einem Tricyclo[5.2.1.$0^{2,6}$]-decan- oder Tricyclo[5.2.1.$0^{2,6}$]-decen-Strukturelement ausgewählt aus der Gruppe bestehend aus

- 8,9-Bis(acryloxymethyl)tricyclo[5.2.1.$0^{2,6}$]decan

- 8,9-Bis(methacryloyloxymethyl)tricyclo[5.21.$0^{2,6}$]decan
- 8,9-[Bis(2-vinyloxyethyl)oxymethyl]tricyclo[5.2.1.$0^{2,6}$]dec-3-en
- 8,9-[Bis(2-vinyloxyethy(oxymethyl]tricyclo[5.2.1.$0^{2,6}$]decan
- 8-Hydroxymethyl-9-(2-vinyloxyethyl)oxymethyl]tricyclo[5.2.1.$0^{2,6}$]dec-3-en
- 9-Hydroxymethyl-8-(2-vinyloxyethyl)oxymethyl]tricyclo[5.2.1.$0^{2,6}$]dec-3-en
- 8,9-Bis(acryloyloxymethyl)tricyclo[5.2.1.$0^{2,6}$]dec-3-en
- 8,9-Bis(methacryloyloxymethyl)tricyclo[5.2.1.$0^{2,6}$]dec-3-en
- Diacrylsäure- oder Dimethacrylsäureester von Verbindungen ausgewählt aus der Gruppe bestehend aus:
- 3,8-Dihydroxymethyl- tricyclo[5.2.1.$0^{2,6}$]decan
- 3,9-Dihydroxymethyltricydo- [5.2.1.$0^{2,6}$]decan
- 4,8-Dihydroxymethyltricyclo[5.2.1.$0^{2,6}$]decan
- 3,8-Dihydroxytricyclo[5.2.1.$0^{2,6}$]decan
- 3,9-Dihydroxytricyclo-[5.2.1.$0^{2,6}$]decan
- 4,8-Dihydroxytricydo[5.2.1.$0^{2,6}$]decan
- Methacrylsäure- oder Acrylsäureester von Verbindungen aus der Gruppe bestehend aus:
- Poly(hydroxymethyl-tricyclo[5.2.1.$0^{2,6}$]decanyl-siloxanen
- oxyalkyliertes Bis(hydroxymethy))tricyclo[5.2.1.$0^{2,6}$]decan
- oxyalkyliertes Bishydroxytricyclo[5.2.1.$0^{2,6}$]decan
- Urethan- oder Hamstoffgruppen enthaltende Methacrylsäure- oder Acrylsäureester von Verbindungen ausgewählt aus der Gruppe bestehend aus:
- 3,8-Dihydroxymethyl-tricyclo[5.2.1.$0^{2,6}$]decan
- 4,8-Dihydroxymethyl-tricyclo[5.2.1,$0^{2,6}$]decan
- 3,9-Dihydroxymethyl-tricyclo[5.2.1.$0^{2,6}$]decan
- 4,9-Dihydroxymethyl-tricyclo[5.2.1,$0^{2,6}$]decan

Hierbei kann in den genannten Verbindungen Wasserstoff am Tricyclo[5.2.1.$0^{2,6}$]-decan-oder Tricyclo[5.2.1.$0^{2,6}$]-decen-Rest durch Alkylgruppen (dabei vorzugsweise C1-C4-Alkyl, Alkoxygruppen (dabei vorzugsweise C1-C4-Alkoxy), Halogenatome (dabei vorzugsweise F) oder Trifluormethylgruppen substituiert sein.

Eine bevorzugte erfindungsgemäße Zusammensetzung umfasst ein, zwei oder mehr Verbindungen der Struktur $Q(YZ_e)_b$ (wie oben definiert und wobei jedes Y unabhängig von etwaigen weiteren Strukturelementen Y gewählt ist oder entfällt), die einen Tiicyclo[5.2.1$0^{2,6}$]-decan-Rest oder einen Tricyclo[3.3.1.$1^{3,7}$]decan-Rest aufweisen, wobei Z vorzugsweise ausgewählt ist aus der Gruppe bestehend aus -O-(C=O)-CH=$CH_2$ und -O-(C=O)-C($CH_3$)=$CH_2$, bevorzugt bedeutet Z die Gruppe -O-(C=O)-C($CH_3$)=$CH_2$.

Viele der vorstehend aufgelisteten radikalisch polymerisierbaren Methacrylsäure- oder Acrylsäureester mit einem TCD-Strukturelement sind aus dem Stand der Technik bekannt.

In eigenen Untersuchungen hat sich gezeigt, dass insbesondere mit den vorstehend oder nachstehend ge-

nannten Monomeren $Q(YZ_e)_b$ (wie oben definiert und wobei jedes Y unabhängig von etwaigen weiteren Strukturelementen Y gewählt ist oder entfällt) der Komponente (a1) mit einem Tricyclo[5.2.1.0$^{2,6}$]-decan-Strukturelement der Komponente (a1) Zusammensetzungen mit einem guten Anfließverhalten an der Wurzelkanalwand und geringer Wasseraufnahme (vorzugsweise weniger als 40 $\mu g/mm^3$, bevorzugt weniger als 35 $\mu g/mm^3$) erhältlich sind.

Y ist vorzugsweise ein Strukturelement, welches in der Struktur $Q(Y_xZ_e)_b$ das polyalicyclische Strukturelement Q mit e Strukturelementen Z verknüpft und ein Strukturelement enthält oder aus diesem besteht, das ausgewählt ist aus der Gruppe bestehend aus

$-CH_2-$ ,

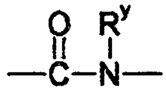
und
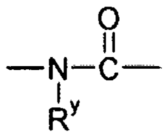

wobei $R^y$ einen sonstigen Rest der Verbindung bedeutet und

wobei die jeweils im Formelbild links angeordnete Bindung dem Strukturelement Q und die rechts angeordnete Bindung dem Strukturelement Z näher ist.

Der sonstige Rest $R^y$ einer erfindungsgemäß einzusetzenden Verbindung der Struktur $Q(Y_xZ_e)_b$ Ist vorzugsweise ausgewählt aus der Gruppe bestehend aus Wasserstoff, linearen, verzweigten oder Ringe umfassenden Strukturelementen mit 1 bis 50 C-Atomen und 0 bis 12 Heteroatomen, wobei die gegebenenfalls vorhandenen Heteroatome vorzugsweise gewählt sind aus der Gruppe bestehend aus N und O.

Der sonstige Rest $R^y$ ist dabei bevorzugt ausgewählt aus der Gruppe bestehend aus Wasserstoff, linearen, verzweigten oder Ringe umfassenden Strukturelementen mit 1 bis 40 C-Atomen und 0 bis 10 Heteroatomen, wobei die gegebenenfalls vorhandenen Heteroatome vorzugsweise gewählt sind aus der Gruppe bestehend aus N und O.

Der sonstige Rest $R^y$ ist dabei besonders bevorzugt ausgewählt aus der Gruppe bestehend aus Wasserstoff, linearen, verzweigten oder Ringe umfassenden Strukturelementen mit 1 bis 35 C-Atomen und 1 bis 10 Heteroatomen, wobei die gegebenenfalls vorhandenen Heteroatome vorzugsweise gewählt sind aus der Gruppe bestehend aus N und O.

Y ist dabei bevorzugt ein Strukturelement, welches ein Strukturelement enthält oder aus diesem besteht, ausgewählt aus der Gruppe bestehend aus

$-CH_2-$

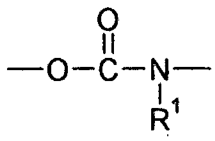
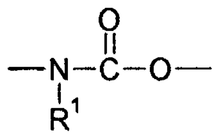
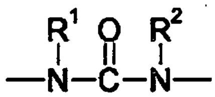

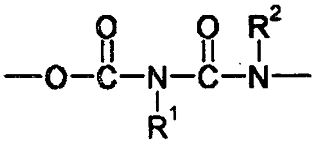
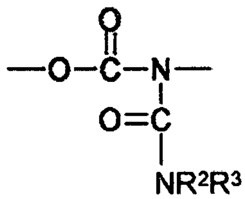
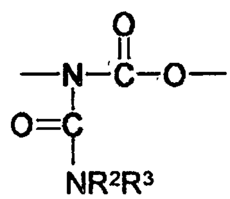

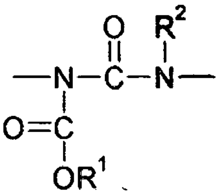
,
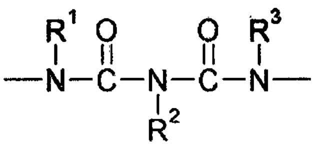
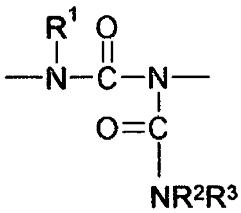

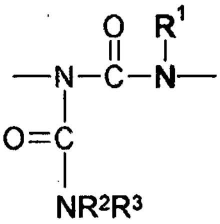 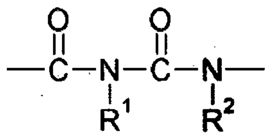 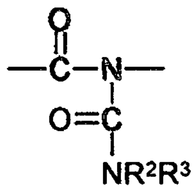

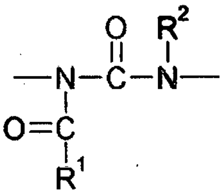 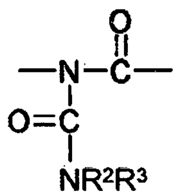

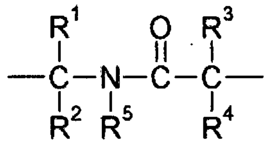 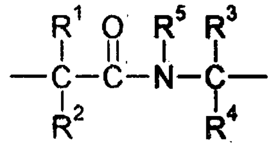

wobei $R^1$, $R^2$, $R^3$, $R^4$ und $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Die oben genannten sonstigen Reste $R^1$, $R^2$, $R^3$, $R^4$ bzw. $R^5$ einer erfindungsgemäß einzusetzenden Verbindung der Struktur $Q(Y_xZ_e)_b$ sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$, $R^3$, $R^4$ bzw. $R^5$ sind dabei, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$, $R^3$, $R^4$ bzw. $R^5$ sind dabei, jeweils unabhängig voneinander, besonders bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

In mit veigleichsweise geringem Aufwand synthetisierbaren erfindungsgemäß einzusetzenden Verbindungen der Struktur $Q(Y_xZ_e)_b$ ist Y ein Strukturelement, welches ein Strukturelement enthält oder aus diesem besteht, das ausgewählt ist aus der Gruppe bestehend aus

$-CH_2-$ 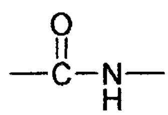 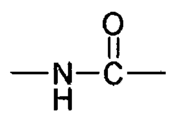

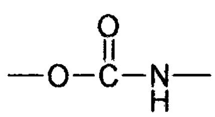 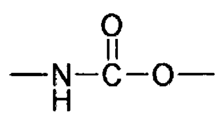 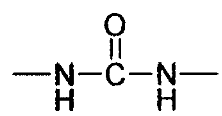

wobei die jeweils im Formelbild links angeordnete Bindung dem Strukturelement Q und die rechts angeordnete Bindung dem Strukturelement Z näher ist.

Die erfindungsgemäß einzusetzenden Verbindungen der Struktur $Q(Y_xZ_e)_b$ können gemäß dem Fachmann bekannten Herstellungsverfahren erhalten werden.

Erfindungsgemäß einzusetzende Verbindungen der Struktur $Q(Y_xZ_e)_b$ mit einem Amid-Strukturelement können

beispielsweise durch Umsetzung von (i) einer Edukt-Verbindung mit einer Isocyanatgruppe und (ii) einer Edukt-Verbindung mit einer Carbonsäuregruppe erhalten werden.

Erfindungsgemäße einzusetzende Verbindungen der Struktur $Q(Y_xZ_e)_b$ mit einem Urethan-Strukturelement können beispielsweise durch Umsetzung von (i) einer Edukt-Verbindung mit einer Isocyanatgruppe und einer Edukt-Verbindung mit einer Alkoholgruppe erhalten werden.

Erfindungsgemäße einzusetzende Verbindungen der Struktur $Q(Y_xZ_e)_b$ mit einem Harnstoff-Strukturelement können beispielsweise durch Umsetzung von (i) einer Edukt-Verbindung mit einer isocyanatgruppe und (ii) einer Edukt-Verbindung mit einer Aminogruppe erhalten werden.

Erfindungsgemäße einzusetzende Verbindungen der Struktur $Q(Y_xZ_e)_b$ mit einem Allophanat-Strukturelement können beispielsweise durch Umsetzung von (i) einer Edukt-Verbindung mit einer Urethangruppe und (ii) einer Edukt-Verbindung mit einer Isocyanatgruppe erhalten werden.

Erfindungsgemäße einzusetzende Verbindungen der Struktur $Q(Y_xZ_e)_b$ mit einem Biuret-Strukturelement können beispielsweise durch Umsetzung von (i) einer Edukt-Verbindung mit einer Hamstoffgruppe und (ii) einer Edukt-Verbindung mit einer Isocyanatgruppe erhalten werden.

Erfindungsgemäße einzusetzende Verbindungen der Struktur $Q(Y_xZ_e)_b$ mit einem *N*-Acylharnstoff-Strukturelement können beispielsweise durch Umsetzung von (i) einer Edukt- Verbindung mit einer Amidgruppe und (ii) einer Edukt-Verbindung mit einer Isocyanatgruppe erhalten werden.

In einer bevorzugten Ausgestaltung einer erfindungsgemäßen Zusammensetzung ist Komponente (b1) so gewählt, dass diese umfasst oder besteht aus Bis(methacryloyloxymethyl)tricyclo[5.2.1.02$^{2,6}$]decan und/oder Bis(acryloyloxymethyotricyclo[5.2.1.0$^{2,6}$]decan.

In erfindungsgemäße Zusammensetzungen sind die Methacrylsäureester wegen ihrer höheren biologischen Verträglichkeit gegenüber den entsprechenden Acrylsäureestem bevorzugt, d.h. dass Z in Verbindungen der Struktur $Q(YZ_e)_b$ (wie oben definiert und wobei jedes Y unabhängig von etwaigen weiteren Strukturelementen Y gewählt ist oder entfällt) bevorzugt $-O-(C=O)-C(CH_3)=CH_2$ bedeutet.

Weiterhin bevorzugte Verbindungen (Monomere) der Struktur $Q(YZ_e)_b$ (wie oben definiert und wobei jedes Y unabhängig von etwaigen weiteren Strukturelementen Y gewählt ist oder entfällt) der Komponente (a1) sind solche mit ein, zwei, drei, vier oder mehrfunktionellen Gruppen ausgewählt aus der Gruppe bestehend aus Urethan, Harnstoff, *N*-Acylhamstoff, Allophanat, Biuret und Amid, wobei die Amidfunktion nicht unmittelbar mit einem N-Atom, einem O-Atom oder einer Carbonylgruppe verknüpft ist.

EP 1 238 993 beschreibt ein Verfahren zur Herstellung von Acylharnstoff-Gruppen enthaltenden Polyisocyanaten sowie Gemische hiervon sowie deren Verwendung als Ausgangskomponente bei der Herstellung von Polyurethan-Kunststoffen.

EP 0 209 700 A2 und DE 35 22 005 beschreiben (Meth)acrylsäure-Derivate bestimmter Tricyclodecane mit zweibindigen Brückengliedern aus der Gruppe der Urethane bzw. Harnstoffe, welche im Dentalbereich eingesetzt werden können.

EP 0 000 194 A1 (entsprechend US 4,160,080) beschreibt Polyisocyanate, die Allophanatgruppen enthalten. Diese Allophanatpolyisocyanate können zur Herstellung von Polyurethan-Schaumstoffen, Elastomeren, Duromeren, Beschichtungen, Verklebungen und Lackierungen eingesetzt werden.

EP 0 682 012 B1 betrifft ein Verfahren zur Herstellung von hellfarbigen lichtechten Allophanatgruppen aufweisenden (cyclo)aliphatischen Polyisocyanaten, durch Umsetzung von Urethangruppen aufweisenden organischen Verbindungen mit organischen Polyisocyanaten mit (cyclo)aliphatisch gebundenen Isocyanatgruppen in Gegenwart von Zinn(II)-salzen. Die in EP 0 682 012 B1 beschriebenen Polyisocyanate können als Aufbaukomponente bei der Herstellung von Polyurethankunststoffen verwendet werden.

EP 1 727 846 B1 offenbart ein Verfahren zur Herstellung von Allophanatgruppen enthaltenden Bindemitteln, die unter Einwirkung aktinischer Strahlung mit ethylenisch ungesättigten Verbindungen unter Polymerisation reagierende Gruppen und gegebenenfalls NCO-reaktive Gruppen aufweisen.

EP 0 712 840 B1 betrifft ein Verfahren zur Herstellung von bestimmten Allophanatgruppen aufweisenden Polyisocyanaten durch Umsetzung unter Allophanatbildung von Urethangruppen aufweisenden Verbindungen. Die Verbindungen gemäß EP 0 712 840 B1 können als Bindemittel oder Bindemittelkomponente in Beschichtungsmitteln verwendet werden.

EP 0 867 457 B1 offenbart ein ethylenisch ungesättigtes Polyurethan, das im Wesentlichen frei von Isocyanatgruppen ist, welches das Reaktionsprodukt eines ethylenisch ungesättigten Polyisocyanats, das Allophanatgruppen und $\beta,\gamma$-ethylenisch ungesättigte Ethergruppen enthält, mit einer hydroxyfunktionellen, ethylenisch ungesättigten Verbindung ist, wobei das ethylenisch ungesättigte Polyisocyanat durch Allophanatierung des Urethangruppen-enthaltenden Reaktionsprodukts eines organischen Diisocyanats mit einem $\beta,\gamma$-ethylenisch ungesättigten Etheralkohol hergestellt wird, wobei der $\beta,\gamma$-ethylenisch ungesättigte Etheralkohol eine Verbindung umfasst, die aus der aus Glycerindiallylether, Trimethylolpropandiallylether und Pentaerythrittriallylether bestehenden Gruppe ausgewählt ist. Die in EP 0 867 457 B1 offenbarten ethylenisch ungesättigten Polyurethane mit Allophanatgruppen können als Bindemittel in Einkomponenten-Beschichtungszusammensetzungen verwendet werden.

DE 10 2007 040 240 A1 und EP 1 645 582 A1 beschreiben jeweils Verfahren zur Herstellung von strahlenhärtenden Allophanaten durch Umsetzung von isocyanatgruppenhaltigen Verbindungen und hydroxyfunktionellen Verbindungen, wobei das Verhältnis von NCO-Gruppen zu den OH-Gruppen 1,45 : 1,0 bis 1,1 : 1,0 beträgt. Gemäß DE 10 2007 040 239 A1 werden unter Einsatz von bestimmten Mischungen enthaltend Hydroxyethylacrylat und Hydroxypropylacrylat als hydroxyfunktionellen Verbindungen entsprechende strahlenhärtenden Allophanate erhalten. Die strahlenhärtenden Allophanate gemäß dieser drei Dokumente können zur Herstellung von Beschichtungen und Lacken sowie Klebstoffen, Druckfarben, Gießharzen, Dentalmassen, Schlichten, Photoresisten, Stereolfthographiesystemen, Harzen für Verbundwerkstoffe und Dichtungsmassen verwendet werden.

DE 10 2004 060 285 A1 betrifft strahlungshärtbare Zusammensetzungen auf Basis eines Dicidolgemisches (enthaltend zwei oder drei isomere 3,8-, 4,8- und/oder 5,8-Bis(hydroxymethyl)tricyclo[5.2.1.0$^{2,8}$]decane) mit mindestens einer Verbindung, welche mindestens eine ethylenisch ungesättigte Gruppierung mit gleichzeitig mindestens einer gegenüber Dicidol reaktive Gruppierung aufweist, wobei diese Verbindung ein Umsetzungsprodukt aus Hydroxyalkyl(meth)acrylat und Diisocyanat sein kann. Die Zusammensetzungen gemäß DE 10 2004 060 285 A1 können verwendet werden als strahlungsinduziert härtende Beschichtungsstoffe, Klebstoffe, Laminierungen, Druckfarben und Tinten, Polituren, Lasuren, Pigmentpasten, Spachtelmassen, Kosmetikartikel, Verpackungsmaterialien und/oder Dicht- und/oder Dämmstoffe.

WO 2006/063891 A1 offenbart radikalisch polymerisierbare Verbindungen, im Wesentlichen enthaltend das Umsetzungsprodukt eines Dicidolgemisches und mindestens einer Verbindung, welche mindestens eine ethylenisch ungesättigte Gruppierung mit gleichzeitig mindestens einer gegenüber Dicidol reaktive Gruppierung aufweist. Die Anwendungsgebiete entsprechen den in DE 10 2004 060 285 A1 genannten.

US 6,670,499 B1 beschreibt vom Adamantan abgeleitet Diurethane. Die in US 6,670,499 beschriebenen Verbindungen eignen sich als Intermediate für den Einsatz im dentalen Bereich oder zur Herstellung von optischen Materialien (wie beispielsweise Linsen).

Auf dem Gebiet der Dentaltechnik besteht ein ständiger Bedarf an weiteren schrumpfungsarmen radikalisch polymerisierbaren Monomeren. Daher war es eine weitere Aufgabe der vorliegenden Erfindung, neue radikalisch polymerisierbare Monomere bereitzustellen, die in einer erfindungsgemäßen Zusammensetzung, insbesondere in einer erfindungsgemäßen dentalen Zusammensetzung, als Bestandteil der Komponente (a1) eingesetzt werden können.

Offenbart wird insoweit eine Verbindung der Struktur $Q(YZ_e)_b$ (wie oben definiert und wobei jedes Y unabhängig von etwaigen weiteren Strukturelementen Y gewählt ist) mit ein, zwei, drei, vier oder mehr funktionellen Gruppen ausgewählt aus der Gruppe bestehend aus *N*-Acylharnstoff, Allophanat, Biuret und Amid, wobei die Amidfunktion nicht unmittelbar mit einem N-Atom, einem O-Atom oder einer Carbonylgruppe verknüpft ist, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $YZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,

  $-(C=O)-CH=CH_2$ und $-(C=O)-C(CH_3)=CH_2$,

  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(YZ_e)_b$ das polyalicyclische Strukturelement Q mit e Strukturelementen Z verbindet;

wobei die Verbindung ein erstes Umsetzungsprodukt ist aus einer ersten Reaktion von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,
wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;

- m ist eine natürliche Zahl ausgewählt aus der Gruppe der natürlichen Zahlen von 0 bis 10,

- jeder index n ist eine natürliche Zahl, die unabhängig von etwaigen weiteren Indizes n ausgewählt ist aus der Gruppe bestehend aus 0 und 1,
  oder
  die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Aus dem oben Gesagten ergibt sich, dass offenbarte Verbindungen, die eine Amidgruppe (wie definiert) enthaltend, diese Amidgruppe nicht Bestandteil einer Urethangruppe ist.

Weiter bevorzugt sind offenbarte Verbindungen der Struktur $Q(YZ_e)_b$ (wie oben definiert und wobei jedes Y unabhängig von etwaigen weiteren Strukturelementen Y gewählt ist) der Komponente (a1) mit ein, zwei, drei, vier oder mehr funktionellen Gruppen ausgewählt aus der Gruppe bestehend *N*-Acylharnstoff, Allophanat, Biuret und Amid, wobei die Amidfunktion nicht unmittelbar mit einem N-Atom, einem O-Atom oder einer Carbonylgruppe verknüpft ist, wobei das Amid wiederum vorzugsweise (Meth)acrylamid bedeutet.

In bevorzugten offenbarten Verbindungen der Struktur $Q(YZ_e)_b$ (wie oben definiert und wobei jedes Y unabhängig von etwaigen weiteren Strukturelementen Y gewählt ist) erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

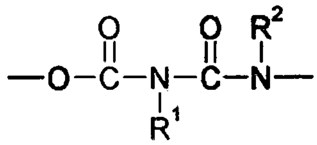
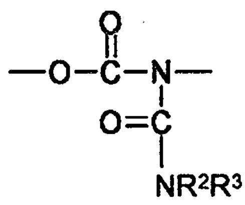
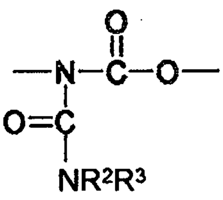

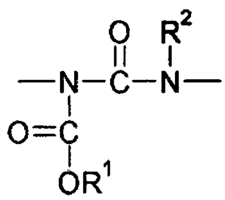
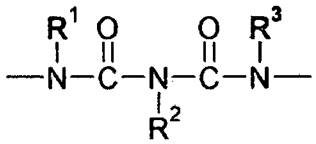
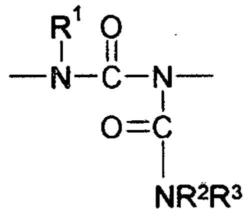

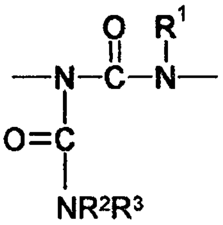
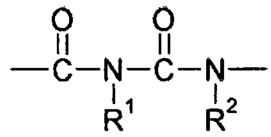
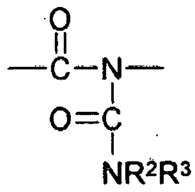

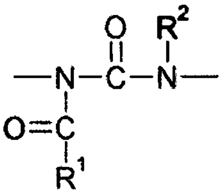
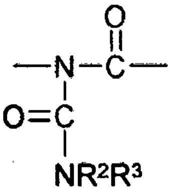

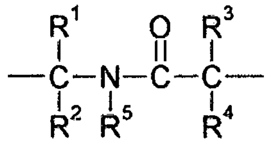
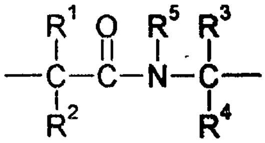

wobei $R^1$, $R^2$, $R^3$ $R^4$, $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Die weitere Aufgabe wird ebenfalls gelöst durch neue Verbindungen der Struktur $Q(Y_xZ_e)_b$ mit x = 1 mit ein, zwei, drei, vier oder mehr funktionellen Gruppen, welche ausgewählt sind aus der Gruppe bestehend aus *N*-Acylharnstoff, Allophanat und Biuret, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $YZ_e$ substituierten Wasserstoffatome dieses polyalicyclischen Strukturelements Q durch Alkylgruppen, Alkoxygruppen, Halogenatome oder Trifluormethylgruppen substituiert ist bzw. sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

  $-O-(C{=}O)-CH{=}CH_2$, $-O-(C{=}O)-C(CH_3){=}CH_2$,

  $-(C{=}O)-CH{=}CH_2$, $-(C{=}O)-C(CH_3){=}CH_2$,

  $-CH{=}CH_2$, $-C(CH_3){=}CH_2$ und $-O-CH{=}CH_2$,

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(Y_xZ_e)_b$ mit x = 1 das polyalicyclische Strukturelement Q mit e Strukturelementen Z verknüpft und ein Strukturelement enthält oder aus diesem besteht, ausgewählt aus der Gruppe bestehend aus

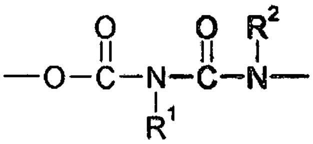
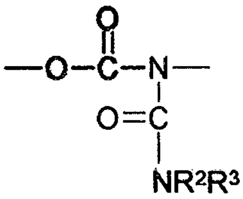
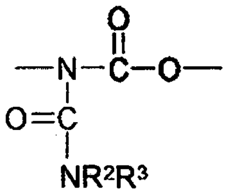

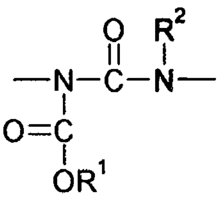
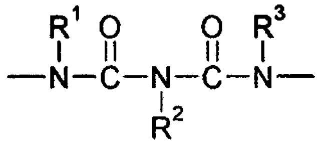
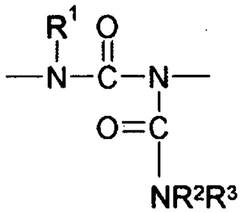

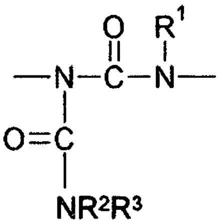
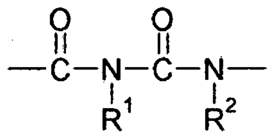
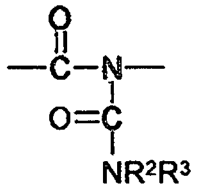

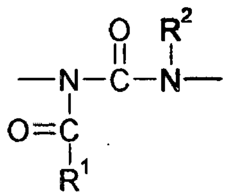
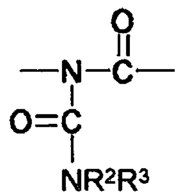

wobei $R^1$, $R^2$ und $R^3$ sonstige Reste der Verbindung bedeuten, wobei die jeweils im Formelbild links angeordnete Bindung dem Strukturelement Q und die rechts angeordnete Bindung dem Strukturelement Z näher ist.

Diese offenbarten Verbindungen sind in besonderer Weise als Monomere für den Einsatz in erfindungsgemäßen Zusammensetzungen geeignet.

Vorzugsweise umfasst eine solche offenbarte Verbindung der Struktur $Q(Y_xZ_e)_b$ mit x = 1 zwei, drei, vier oder mehr funktionelle Gruppen ausgewählt aus der Gruppe bestehend aus *N*-Acylharnstoff, Allophanat und Biuret.

In einer bevorzugten Ausgestaltung bedeutet jeder Index e eine natürliche Zahl, die unabhängig von etwaigen weiteren Indizes e ausgewählt ist aus der Gruppe der natürlichen Zahlen 2, 3 und 4.

Die oben genannten sonstigen Reste $R^1$, $R^2$ bzw. $R^3$ einer offenbarten neuen Verbindung sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$ bzw. $R^3$ einer offenbarten neuen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$ bzw. $R^3$ einer offenbarten neuen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

Eine offenbarte neue Verbindung $Q(Y_xZ_e)_b$ mit x = 1, vorzugsweise eine erfindungsgemäße Verbindung $Q(Y_x\text{-}Z_e)_b$ mit x = 1 wie sie vorstehend oder nachfolgend als bevorzugt bezeichnet ist, ist vorzugsweise herstellbar durch Umsetzung eines ersten Umsetzungsproduktes, welches das Umsetzungsprodukt ist aus einer ersten Reaktion von

A) einer Verbindung der Struktur $QG_b$, worin jedes G eine reaktive Gruppe bedeutet, die unabhängig von weiteren

Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,
wobei gilt:

- R bedeutet jeweils unabhängig von etwaigen weiteren R ein Wasserstoffatom oder einen Alkylrest;

- m ist eine natürlichen Zahl ausgewählt aus der Gruppe der natürlichen Zahlen von 0 bis 10,

- jeder Index n ist eine natürliche Zahl, die unabhängig von etwaigen weiteren Indizes n ausgewählt ist aus der Gruppe bestehend aus 0 und 1,

wobei die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
Eine offenbarte Verbindung gemäß einer bevorzugten Ausgestaltung ist ein zweites Umsetzungsprodukt aus einer Reaktion von dem obigen ersten Umsetzungsprodukt mit

C) einer weiteren Verbindung gemäß A) oder B), wobei jede weitere Verbindung gemäß A) bzw. B) dieselbe Bedeutung hat wie in der ersten Reaktion,
und/oder
wobei die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

In einer bevorzugten Ausführungsform ist m = 0. Dies gilt für sämtliche Aspekte der vorliegenden Erfindung.

In bevorzugten offenbarten Verbindungen erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

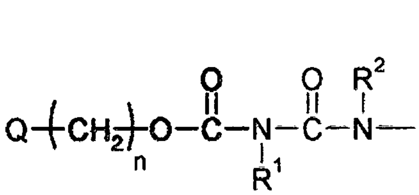

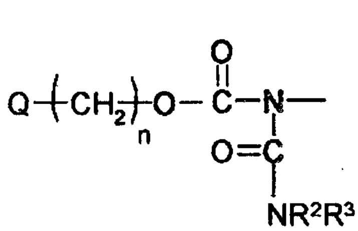

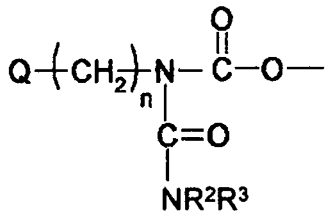

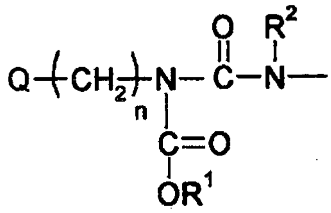

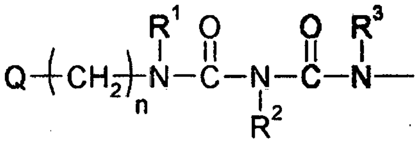

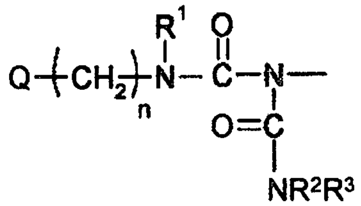

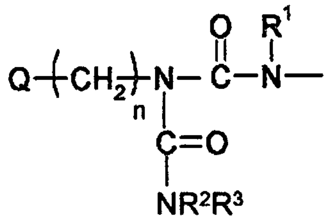

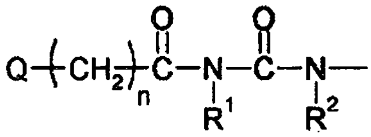

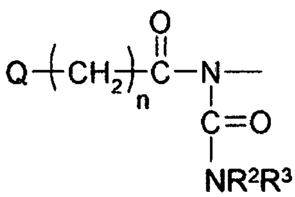

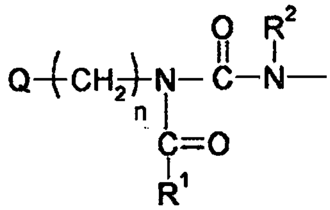

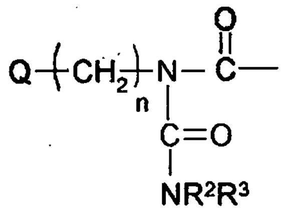

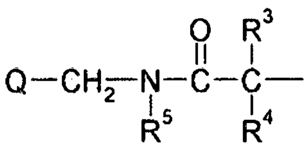

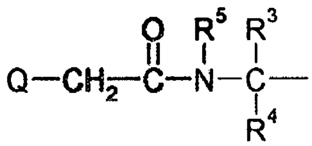

wobei $R^1$, $R^2$, $R^3$ $R^4$, $R^5$ sonstige Reste der Verbindung bedeuten und Q sowie der index n die oben angegebene Bedeutung haben.

Die jeweils im Formelbild rechts angeordnete Bindung ist dem Strukturelement Z näher.

In einer bevorzugten Ausgestaltung umfasst eine neue offenbarte Verbindung $Q(Y_xZ_e)_b$ mit x = 1, vorzugsweise eine offenbarte Verbindung $Q(Y_xZ_e)_b$ mit x = 1 wie sie vorstehend oder nachfolgend als bevorzugt bezeichnet ist, ein oder mehrere Strukturelemente ausgewählt aus der Gruppe bestehend aus

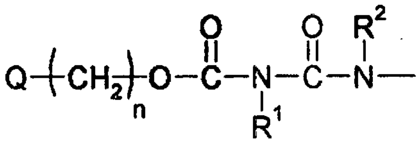

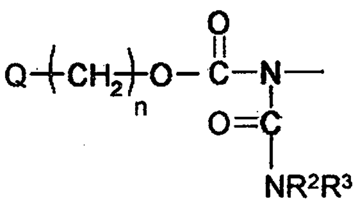

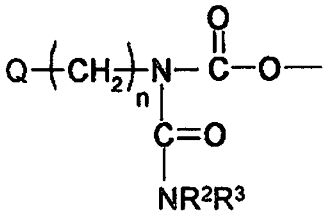
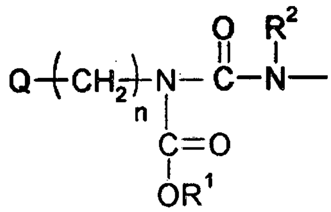
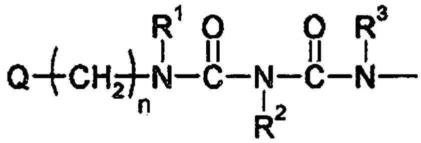
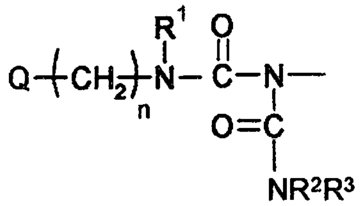
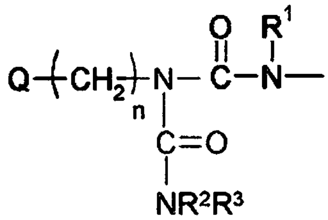
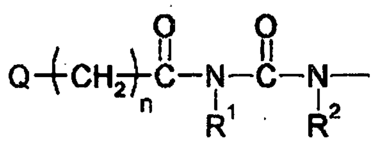
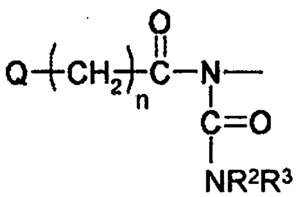
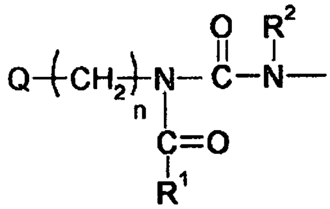
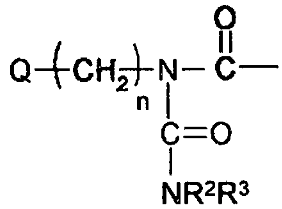

wobei $R^1$, $R^2$ und $R^3$ sonstige Reste der Verbindung bedeuten (und vorzugsweise die oben genannte bevorzugte Bedeutung haben) und Q die oben genannte Bedeutung hat und der Index n ausgewählt ist aus der Gruppe bestehend aus 0 und 1.

Wie oben bereits erwähnt sind bevorzugte neue offenbarte Verbindungen solche, wobei Q ein gesättigtes polyalicyclisches Strukturelement bedeutet, das ausgewählt ist aus der Gruppe bestehend aus bicyclischen und tricyclischen Kohlenwasserstoffresten, wobei vorzugsweise keines der nicht durch Substituenten $YZ_e$ (wobei jedes Y unabhängig von etwaigen weiteren Strukturelementen Y gewählt ist) substituierten Wasserstoffatome dieses polyalicyclischen Strukturelements Q substituiert ist.

Besonders bevorzugte offenbarte neue Verbindungen sind solche, wobei das Strukturelement Q einen Tricyclo[5.2.1.$0^{2,6}$]decan-Rest, einen Tricyclo[5.2.1.$0^{2,6}$]dec-3-en-Rest, einen Tricyclo[3.3.1.$1^{3,7}$]decan-Rest oder einen Bicyclo[2.2.1]heptan-Rest bedeutet.

Bevorzugt sind offenbarte neue Verbindungen, in denen

(i) das Strukturelement Z = $-O-(C=O)-C(CH_3)=CH_2$ bedeutet, wobei die funktionellen Gruppen Allophanat-, Biuret- oder Acylharnstoff-Gruppen sind, da mit diesen Verbindungen besonders gute Ergebnisse erzielt wurden, und/oder

(ii) das Strukturelement Q einen Tricyclo[5.2.1.$0^{2,6}$]decan-Rest bedeutet.

Weiter bevorzugt sind offenbarte neue Verbindungen, in denen das Strukturelement Z = $-O-(C=O)-C(CH_3)=CH_2$ bedeutet, wobei die funktionellen Gruppen Allophanat-, Biuret-oder Acylharnstoft-Gruppen sind und das Strukturelement Q einen Tricyclo[5.2.1.$0^{2,6}$]decan-Rest bedeutet.

Bevorzugt sind offenbarte neue Verbindungen, in denen sämtliche vorhandenen lichthärtbaren Gruppen dem Strukturelement Z entsprechen.

Bevorzugt sind offenbarte neue Verbindungen, in denen sämtliche vorhandenen endständigen polymerisierbaren Gruppen dem Strukturelement Z entsprechen.

Eine offenbarte neue Verbindung kann neben lichthärtbaren Gruppen des Strukturelements Z auch weitere polymerisierbare, vorzugsweise endständige polymerisierbare, Gruppen umfassen, die nicht lichthättbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.

Weiter bevorzugte offenbarte neue Verbindungen sind solche, in denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

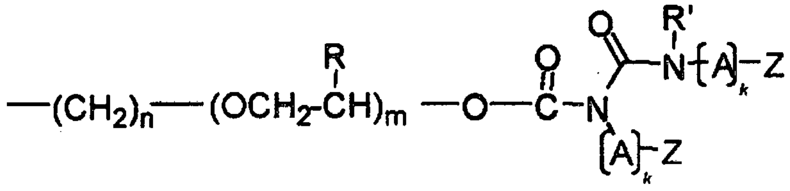

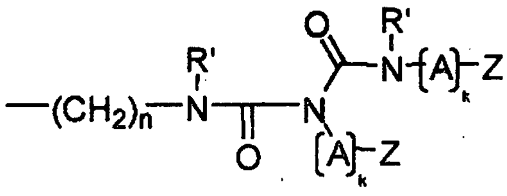

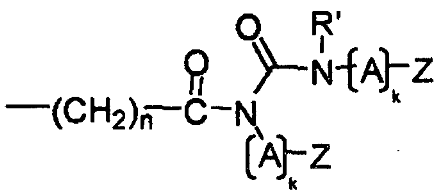

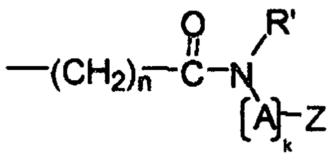

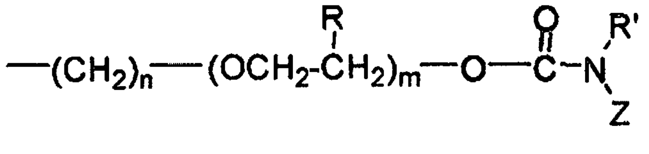

wobei Z, R, m sowie n die obige Bedeutung haben und worin ferner gilt:

- jedes A bedeutet ein zweibindiges organisches Brückenglied,
- jeder index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement $(C{=}O)\text{-}NH\text{-}(A)_k\text{-}Z$, wobei A, Z und k wiederum die obigen Bedeutungen haben.

In einer bevorzugten Ausführungsform ist m = 0.

Ebenfalls bevorzugte offenbarte Verbindungen sind solche, bei denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

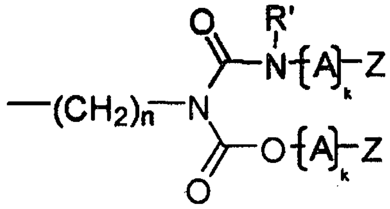

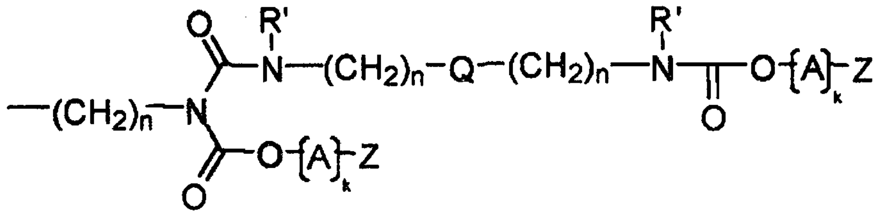

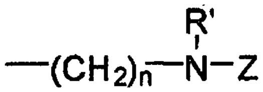

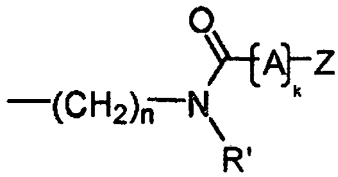

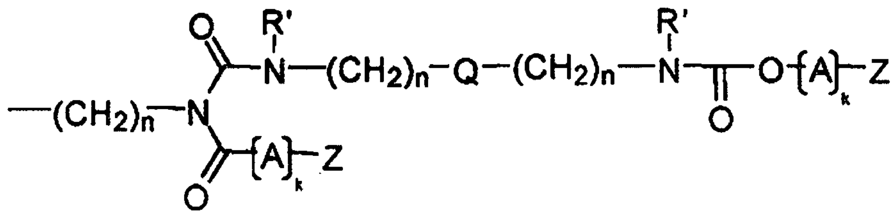

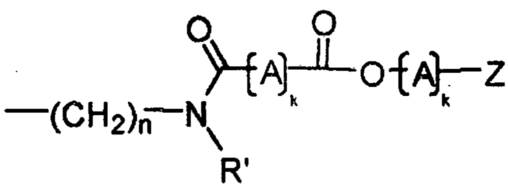

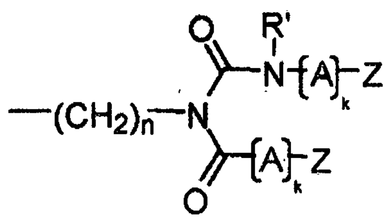

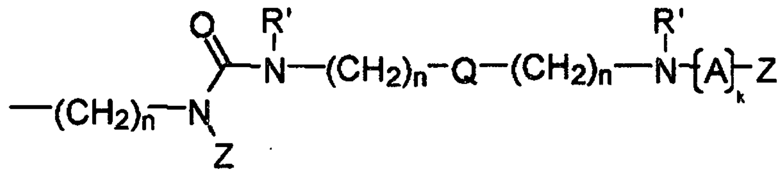

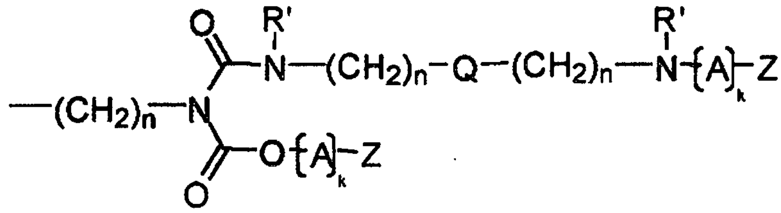

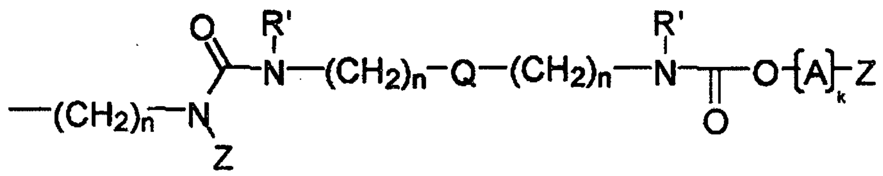

wobei jedes Q unabhängig von etwaigen weiteren Strukturelementen Q die obige Bedeutung hat und wobei Z, A, k und R' sowie n die oben genannte Bedeutung haben.

In einer bevorzugten Ausgestaltung betrifft die vorliegende Erfindung eine offenbarte Verbindung $Q(Y_xZ_e)_b$ mit x = 1, vorzugsweise eine offenbarte Verbindung $Q(Y_xZ_e)_b$ mit x = 1 wie sie vorstehend oder nachfolgend als bevorzugt bezeichnet ist, wobei
mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind,
aus der Gruppe bestehend aus

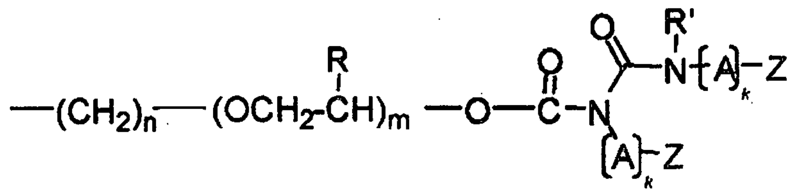

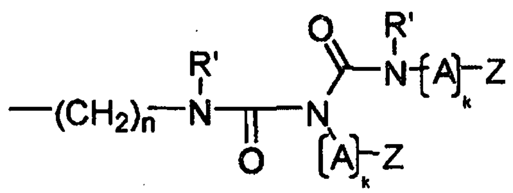

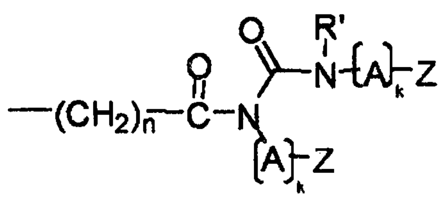

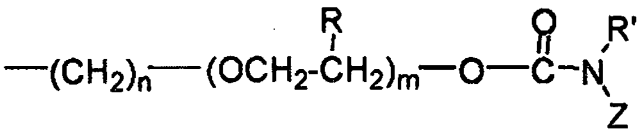

wobei Z, R, m sowie n die obige Bedeutung haben und worin ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$Z, wobei A, Z und k wiederum die obigen Bedeutungen haben.

In einer bevorzugten Ausgestaltung betrifft die vorliegende Erfindung eine offenbarte neue Verbindung $Q(Y_xZ_e)_b$ mit x = 1, vorzugsweise eine offenbarte Verbindung $Q(Y_xZ_e)_b$ mit x = 1 wie sie vorstehend oder nachfolgend als bevorzugt bezeichnet ist, wobei
mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind,
aus der Gruppe bestehend aus

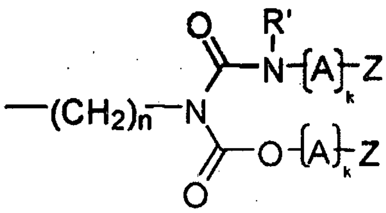

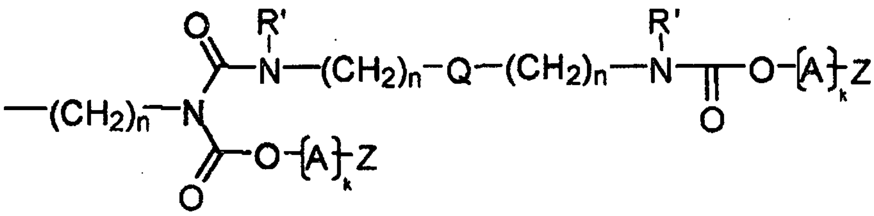

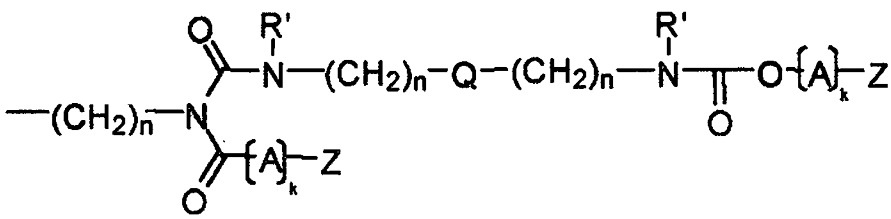

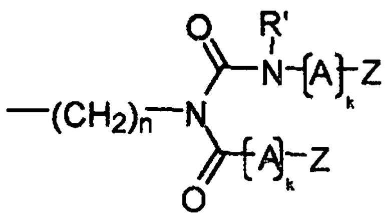

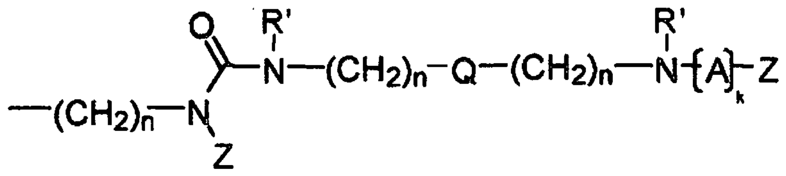

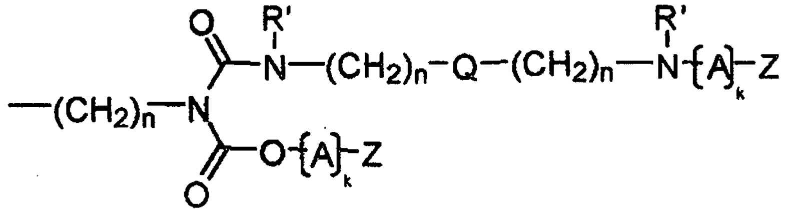

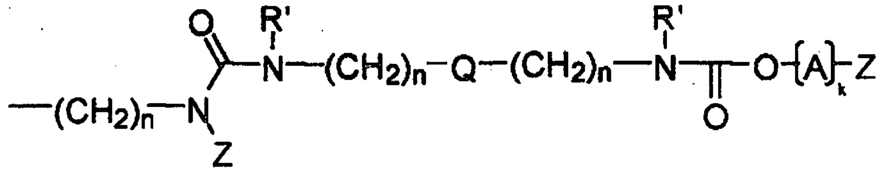

wobei jedes Q unabhängig von etwaigen weiteren Strukturelementen Q die obige Bedeutung hat, und wobei Z und n die oben genannte Bedeutung haben und wobei ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement $(C=O)-NH-(A)_k-Z$, wobei A, Z und k wiederum die obigen Bedeutungen haben.

Dabei wiederum bevorzugt sind offenbarte einzusetzende Verbindungen, in denen jedes Strukturelement A unabhängig von etwaigen weiteren Strukturelementen A ausgewählt ist aus der Gruppe bestehend aus linearen, verzweigten oder Ringe umfassenden zweibindigen organischen Brückengliedern mit 1 bis 25 C-Atomen und gegebenenfalls 1 bis 10, vorzugsweise 1 bis 5 Heteroatomen, wobei die gegebenenfalls vorhandenen Heteroatome vorzugsweise gewählt sind aus der Gruppe bestehend aus N und O.

Dabei wiederum bevorzugt ist eine offenbarte Verbindung, in der jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, vorzugsweise mit 1 bis 5 Heteroatomen, wobei die gegebenenfalls vorhandenen Heteroatome vorzugsweise gewählt sind aus der Gruppe bestehend aus N und O.

Weiter bevorzugt sind Verbindungen, in denen jedes Strukturelement A unabhängig von etwaigen weiteren Strukturelementen A ausgewählt ist aus der Gruppe bestehend aus $C_1$-$C_{20}$-Alkylen, $C_1$-$C_{20}$-Heteroalkylen, $C_3$-$C_{20}$-Cycloalkylen, $C_4$-$C_{20}$-Cycloalkylalkylen, $C_2$-$C_{20}$-Alkenylen, $C_3$-$C_{20}$-Cycloalkenylen, $C_4$-$C_{20}$-Cycloalkenylalkylen, $C_4$-$C_{20}$-Cycloalkenylenalkylen, $C_3$-$C_{25}$-Arylen, $C_2$-$C_{25}$-Heteroarylen, $C_4$-$C_{25}$-Arylalkylen, $C_4$-$C_{25}$-Arylenalkylen, $C_4$-$C_{25}$-Arylheteroalkylen, $C_4$-$C_{25}$-Arylenheteroalkylen.

In bevorzugten Ausgestaltungen umfasst Strukturelement A eine oder mehrere der folgenden Atome oder Atomgruppen:

$$-O-,\ -O-Ar^1-CR^8F^7-Ar^2-O-,\ -NR^8-,\ -N-(C=O)-,\ -NH-(C=O)-O-,\ -NH-C(=O)-NH-$$

wobei gilt:

$Ar^1$ und $Ar^2$ bedeuten unabhängig voneinander einen gegebenenfalls substituierten, dabei vorzugsweise einen ein oder mehrfach mit C1-C4-Alkylresten substituierten, aromatischen Ring, dabei wiederum bevorzugt einen Phenyl-

ring,

$R^6$, $R^7$ und $R^8$ bedeuten unabhängig voneinander Wasserstoff oder einen C1-C8-Rest, dabei vorzugsweise einen C1-C4-Alkylrest, dabei wiederum bevorzugt Methyl oder Ethyl.

Offenbart wird ferner ein Verfahren zur Herstellung einer offenbarten Verbindung $Q(YZ_e)_b$ (wobei jedes Y unabhängig von etwaigen weiteren Strukturelementen Y gewählt ist) oder einer Mischung, umfassend zumindest eine solche offenbarte Verbindung $Q(YZ_e)_b$, mit folgenden Schritten:

In einer ersten Reaktion Umsetzen von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$, vorzugsweise einer Verbindung der Struktur $QG_b$, worin jedes G eine reaktive Gruppe bedeutet, die unabhängig von weiteren Gruppen G ausgewählt ist aus der Gruppe bestehend aus $-NH_2$, $-CH_2NH_2$, -OH, $-CH_2OH$, -NCO, $-CH_2NCO$, und - COOH,
mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH
zu einem ersten Umsetzungsprodukt,
gegebenenfalls in einer zweiten Reaktion Umsetzen des ersten Umsetzungsproduktes mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
zu einem zweiten Umsetzungsprodukt
und gegebenenfalls in einer dritten Reaktion Umsetzen des zweiten Umsetzungsproduktes mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

wobei Q, b, Y, Z, und e jeweils die oben genannte Bedeutung haben, und wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;
- m ist eine natürliche Zahl ausgewählt aus der Gruppe der natürlichen Zahlen von 0 bis 10,
- jeder Index n ist eine natürliche Zahl, die unabhängig von etwaigen weiteren Indizes n ausgewählt ist aus der Gruppe bestehend aus 0 und 1,

wobei das Verhältnis der Gesamtzahl von NCO-Gruppen zu der Gesamtzahl von - $NH_2$, -OH und -COOH in der Gesamtzahl von Verbindungen gemäß A) und B) in der ersten, gegebenenfalls zweiten und gegebenenfalls dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

Ein bevorzugtes Verfahren zur Herstellung einer offenbarten Verbindung $Q(Y_xZ_e)_b$ mit x = 1, vorzugsweise einer offenbarten Verbindung $Q(Y_xZ_e)_b$ mit x = 1 wie sie vorstehend oder nachfolgend als bevorzugt bezeichnet ist, oder einer Mischung umfassend zumindest eine solche Verbindung $Q(Y_xZ_e)_b$, ist ein Verfahren mit folgenden Schritten:

In einer ersten Reaktion Umsetzen von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-C_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH
zu einem ersten Umsetzungsprodukt,
in einer zweiten Reaktion Umsetzen des ersten Umsetzungsproduktes mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
zu einem zweiten Umsetzungsprodukt
und gegebenenfalls in einer dritten Reaktion Umsetzen des zweiten Umsetzungsproduktes mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

wobei Q, b, Y, Z, e, M, R, m und n jeweils die oben genannte Bedeutung haben, und
wobei das Verhältnis der Gesamtzahl von NCO-Gruppen zu der Gesamtzahl von $-NH_2$, - OH und -COOH in der Gesamtzahl von Verbindungen gemäß A) und B) in der ersten, der zweiten und gegebenenfalls der dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

In einem Verfahren zur Herstellung einer offenbarten Verbindung $Q(Y_xZ_e)_b$ mit x = 1, vorzugsweise einer offenbarten Verbindung $Q(Y_xZ_e)_b$ mit x = 1 wie sie vorstehend oder nachfolgend als bevorzugt bezeichnet ist, oder einer Mischung umfassend zumindest eine solche Verbindung $Q(Y_xZ_e)_b$, liegt das Verhältnis der Gesamtzahl von umgesetzten NCO-Gruppen zu der Gesamtzahl von umgesetzten - $NH_2$, -OH und -COOH in der Gesamtzahl von Verbindungen gemäß A) und B) in der ersten, gegebenenfalls zweiten und gegebenenfalls dritten Reaktion bevorzugt im Bereich von 1,1 : 1 bis 5 : 1, weiter bevorzugt im Bereich von 1,25 : 1 bis 4 : 1, besonders bevorzugt im Bereich von 1,5 : 1 bis 3 : 1, und am meisten bevorzugt im Bereich von 2 : 1 bis 2,5 : 1.

Vorzugsweise erfolgt die Reaktion zu dem ersten Umsetzungsprodukt, zu dem zweiten Umsetzungsprodukt und/oder zu dem dritten Umsetzungsprodukt in Gegenwart eines Katalysators.

Bevorzugte Katalysatoren sind dabei tertiäre Amine oder Lewis Säuren, dabei wiederum bevorzugt Metallsalze höherer Fettsäuren, insbesondere Dibutylzinndilaurat oder Zinn(II)octoat.

Die Menge des Katalysators liegt dabei vorzugsweise im Bereich von 0,01 bis 2 Gew.-%, bevorzugt von 0,08 bis 1 Gew.-%, bezogen auf die Gesamtmenge der Reaktanden gemäß A) und B) sowie gegebenenfalls C) und gegebenenfalls D).

Die Reaktion zu dem ersten Umsetzungsprodukt, zu dem zweiten Umsetzungsprodukt und/oder zu dem dritten Umsetzungsprodukt erfolgt vorzugsweise in einem Temperaturbereich von 0 bis 160°C, bevorzugt im Bereich von 30 bis 140°C und besonders bevorzugt im Bereich von 60 bis 120°C. Die Umsetzung wird vorzugsweise bei Normaldruck (1013 mbar) ausgeführt.

Offenbart wird ferner eine Mischung umfassend ein, zwei oder mehr unterschiedliche offenbarten Verbindungen, herstellbar nach einem vorstehend offenbarten Verfahren.

Offenbart wird ferner die Verwendung einer offenbarten Verbindung, vorzugsweise in einer der als bevorzugt oder besonders bevorzugt gekennzeichneten Ausgestaltungen, zur Herstellung einer dentalen Zusammensetzung, vorzugsweise zur Herstellung einer offenbarten Zusammensetzung.

Offenbart wird ferner offenbarten die Verwendung einer Verbindung, vorzugsweise in einer der als bevorzugt oder besonders bevorzugt gekennzeichneten Ausgestaltungen, in einer dentalen Zusammensetzung.

Offenbart wird ferner die Verwendung einer offenbarten Verbindung, vorzugsweise in einer der als bevorzugt oder besonders bevorzugt gekennzeichneten Ausgestaltungen, in einer zum Füllen- und/oder Versiegeln eines Wurzelkanals geeigneten Zusammensetzung, vorzugsweise in einer der als bevorzugt oder besonders bevorzugt gekennzeichneten Ausgestaltungen.

Zur Herstellung der offenbarten Verbindungen können vorzugsweise Hydroxylverbindungen von (Meth)acrylaten eingesetzt werden, wobei auch Gemische von Acrylaten und Methacrylaten verwendet werden können. Bevorzugt sind (zum Einsatz als Reaktionspartner gemäß Komponente B), C) und/oder D)):

Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat., Hydroxybutyl(meth)acrylat, 4-Hydroxybutyl(meth)acrylat, Hydroxypentyl(meth)acrytat. 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritol-tri(meth)acrylat, wie Dipentaerythrit, wie beispielsweise Dipentaerythritol-penta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Zur Herstellung der offenbarten Verbindungen können als Komponente B) auch Isocyanate eingesetzt werden. Bevorzugt sind dabei Mono- und Diisocyanate.

Bevorzugte Diisocyanate sind gewählt aus der Gruppe bestehend aus Cyclohexandiisocyanat, Methylcyclohexandiisocyanat, Ethylcyclohexandiisocyanat, Propylcyclohexandiisocyanat, Methyldiethylcyclohexandiisocyanat, Phenylendiisocyanat, Toluylendiisocyanat, Bis(isocyanatophenyl)methan, Propandiisocyanat, Butandiisocyanat, Pentandiisocyanat, Hexandiisocyanat, wie Hexamethylendiisocyanat oder 1,5-Diisocyanato-2-methylpentan, Heptandiisocyanat, Octandiisocyanat, Nonandiisocyanat, wie 1,6-Diisocyanato-2,4,4-trimethylhexan oder 1,6-Diisocyanato-2,2,4-trimethylhexan, Nonantriisocyanat, wie 4-Isocyanatomethyl-1,8-octandiisocyanat, Decandi- und triisocyanat. Undekandi- und -triisocyanat, Dodecandi- und -triisocyanat, Isophorondiisocyanat, Dicyclohexylmethan-4,4'-diisocyanat, Isocyanatomethylmethylcyclohexylisocyanat, 1,3-Bis(isocyanatomethyl)cyclohexan oder 1,4-Bis(isocyanatomethyl)cyclohexan.

Bevorzugte Monoisocyanate sind (Meth)acryloylisocyanat und (Meth)acryl-C2-C8-alkylisocyanate (d.h. (Meth)acrylalkylisocyanate mit Alkylspacern, die über 2 bis 8, besonders bevorzugt 2 bis 6 Kohlenstoffatome verfügen), dabei wiederum bevorzugt ist (Meth)acrylethylisocyanat (2-Isocyanatoethyl(meth)acrylat).

Außerdem haben sich als Komponente B) Monoisocyanate vorteilhaft erwiesen, die Umsetzungsprodukte sind aus Amino- bzw. Hydroxyalkyl(meth)acrylaten, deren Alkylspacer über 1 bis 12, bevorzugt 2 bis 8, besonders bevorzugt 2 bis 6 Kohlenstoffatome verfügen, und Diisocyanaten.

Vorzugsweise wird dazu ein vorstehend genanntes Diisocyanat äquimolar mit einer (oben als bevorzugt gekennzeichneten) Amino- oder Hydroxylalkylverbindung eines (Meth)acrylats umgesetzt, wobei die Hydroxylalkylverbindungen wiederum vorzugsweise gewählt sind aus der Gruppe bestehend aus Hydroxyethyl(meth)acrylat, Hydroxypropyl(meth)acrylat, Hydroxybutyl(meth)acrylat, Hydroxypentyl(meth)acrylat, Hydroxyhexyl(meth)acrylat.

Als Beispiele seien die Umsetzungsprodukte im molaren Verhältnis von 1 : 1 von Hydroxyethylmethacrylat mit Isophorondüsocyanat, Dicyclohexylmethan-4,4'-diisocyanat oder Hexamethylendiisocyanat genannt.

Im Folgenden wird die Erfindung zunächst für Monomere umfassend tricyclische Struk-turelemente Q am Beispiel von Tricyclo[5.2.1.$0^{2,6}$]decan (TCD) - Derivaten im Detail erläutert.

1.) Ausgehend vom Bis(hydroxymethyl)tricyclo[5.2.1.$0^{2,8}$]decan (TCO-Diol)

Bis(hydroxymethyntricyclo[5.2.1.$0^{2,6}$]decan ist kommerziell erhältlich, beispielsweise als Dicidolgemisch der isomeren Verbindungen 3,8-Bis(hydroxymethyl)tricyclo[5.2.1.$0^{2,6}$]decan und 4,8-Bis(hydroxymethyl)tricyclo[5.2.1.$0^{2,6}$]decan sowie 3,9-Bis(hydroxymethyl)tricyclo[5.2.1.$0^{2,6}$]decan und 4,9-Bis(hydroxymethyl)tricyclo[5.2.1.$0^{2,6}$]decan.

Die Bis(hydroxymethyl)tricyclo[5.2.1.$0^{2,6}$]decane können, ausgehend von Dicyclopentadien (Tricyclo[5.2.1.$0^{2,6}$]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.$0^{2,6}$]decan. Je nach Synthesepfad können gezielt an unterschiedlichen Positionen substituierte Bis(hydroxymethyl)tricyclo[5.2.1.$0^{2,6}$]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.$0^{2,6}$]decan herstellbar ist. Die DE 103 52 260 B3 beschreibt dagegen Verfahren zur Herstellung von 3(4), 8(9)-Bis(hydroxymethyl)tricyclo[5.2.1.$0^{2,6}$]decan. Die Notation der Positionen der Hydroxymethylgruppen 3(4), 8(9) bedeutet 3 oder 4, 8 oder 9.

Das im Handel erhältliche und als Ausgangsverbindung zur Herstellung erfindungsgemäß einzusetzender Monomere einsetzbare Bis(hydroxymethyl)tricyclo[5.2.1.$0^{2,6}$]decan enthält somit Hydroxymethylgruppen sowohl an den Positionen 3 oder 4 als auch in den Stellungen 8 oder 9. Es ist nun möglich, durch Anlagerung von Alkylenoxiden, im

Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die Umsetzung der 3(4), 8(9)-Bis(hydroxymethyl)tricyclo[5.2.1.$0^{2,6}$]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.$0^{2,6}$]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

Das erhaltene Reaktionsprodukt (Formel (1)) von 2-Isocyanatoethylmethacrylat mit 3(4), 8(9)-Bis(hydroxymethyl)tricyclo[5.2.1.$0^{2,6}$]decan weist in einer Formulierung nach Aushärtung einen niedrigen Reaktionsschrumpf auf.

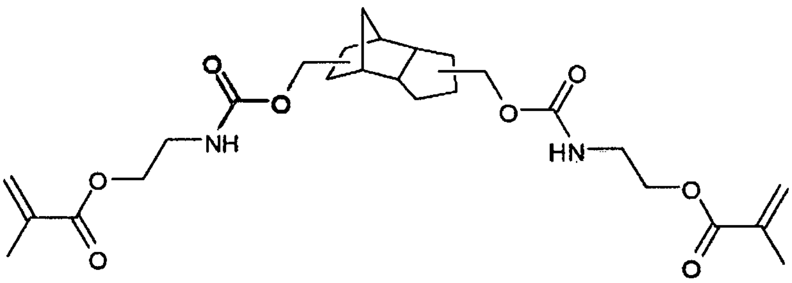

Formel (1)

Das Urethan der Formel (1) besitzt noch zwei reaktionsfähige Wasserstoffatome am Stickstoff, die nun in einer zweiten Umsetzungsstufe mit überschüssigem Isocyanat weiter abreagiert werden unter Bildung einer offenbarten Verbindung. Es bildet sich hierbei zunächst das Allophanat der Formel (2) als tetrafunktionalisierte, radikalisch vernetzbare Verbindung. Auch dieses Monomer weist wiederum noch umsetzungsfähige Wasserstoffatome am Stickstoff auf, die bei Reaktion mit weiterem Isocyanat das hexafunktionalisierte, radikalisch härtbare Allophanat der Formel (3) bilden.

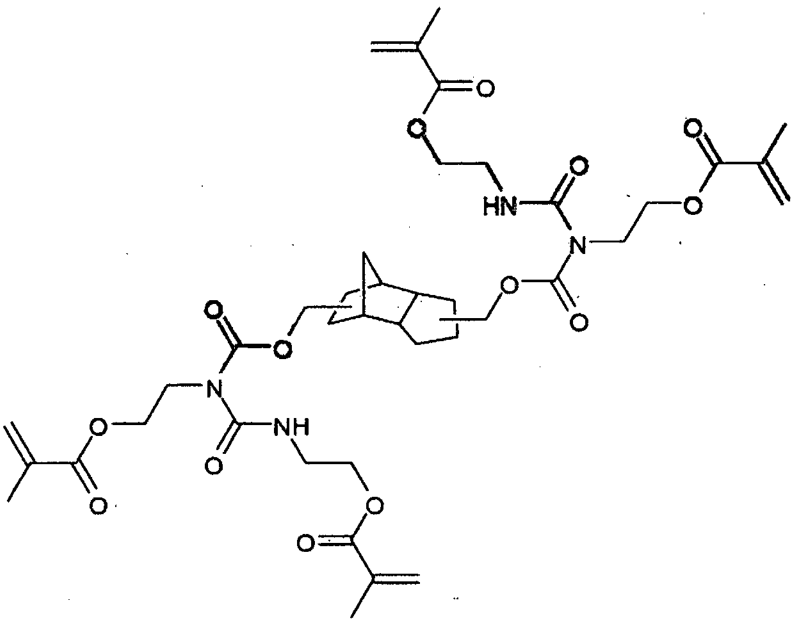

Formel (2)

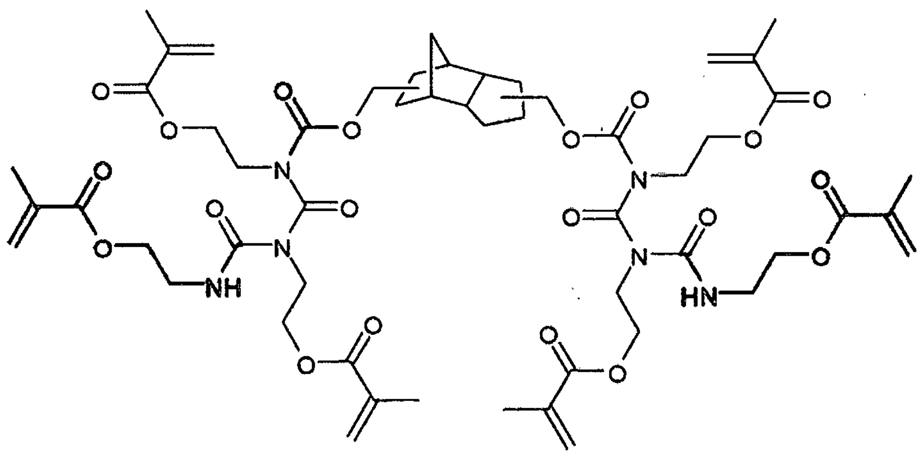

Formel (3)

Alternativ kann das 3(4), 8(9)-Bis(hydroxymethyl)tricyclo[5.2.1.0$^{2,6}$]decan auch mit Methacryloylisocyanat zur Reaktion gebracht werden. Methacryloylisocyanat ist kommerziell oder durch Umsetzung von Methacrylamid mit Oxalylchlorid erhältlich, wie in der EP 0 143 613 B1 beschrieben. Durch Umsetzung von 3(4), 8(9)-Bis(hydroxymethyl)tricyclo[5.2.1.0$^{2,6}$]decan mit Methacryloylisocyanat wird eine Verbindung der Formel (4) erhalten:

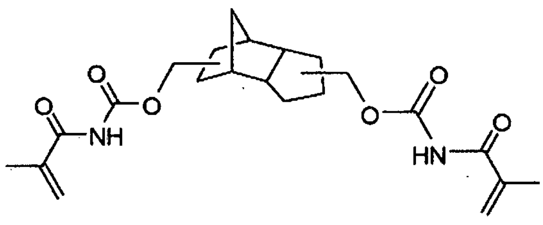

Formel (4)

Die verbleibenden reaktiven Wasserstoffatome am Stickstoff der Verbindung der Formel (4) können anschließend wiederum in Isocyanatreaktionen zu Allophanaten umgesetzt werden. Beispielhaft sei hier das Reaktionsprodukt mit 2-Isocyanatoethylmethacrylat (Formel (5)) gezeigt.

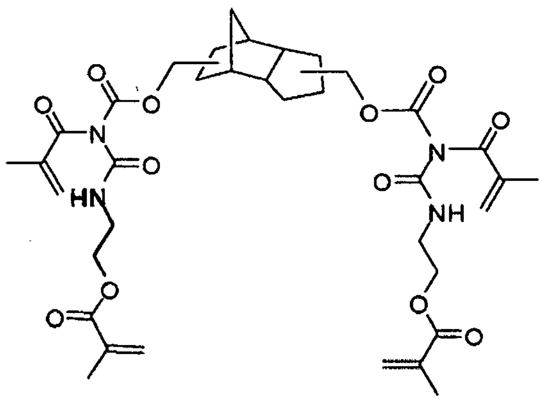

Formel (5)

2.) Ausgehend von 3(4), 8(9)-Bis(Carbonsäure)tricyclo[5.2.1 $0^{2,6}$]decan

Das 3(4), 8(9)-Bis(Carbonsäure)tricyclo[5.2.1.$0^{2,6}$]decan ist durch einfache Oxidation des kommerziell erhättlichen 3(4), 8(9)-Bis(formyl)tricyclo[5.2.1.$0^{2,8}$]decans herstellbar. Umsetzung der Dicarbonsäure mit 2-Isocyanatoethylmethacrylat ergibt das Amid der Formel (8):

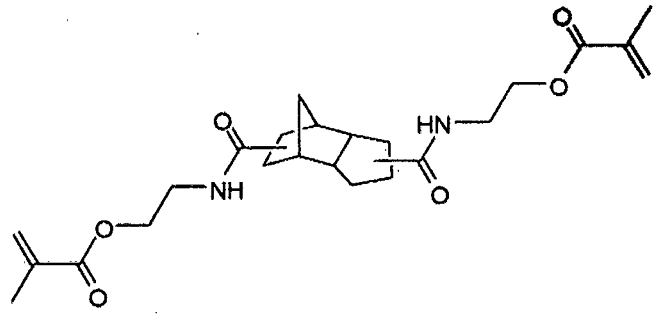

Formel (8)

Weitere Umsetzung der beiden reaktionsfähigen Amid-Wasserstoffatome des Amids der Formel (8) mit 2-Isocyanatoethylmethacrylat ergibt den Acylharnstoff der Formel (9).

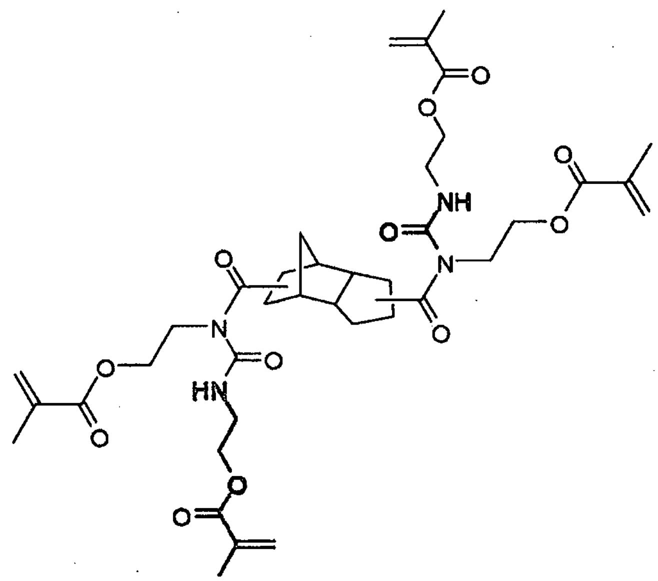

Formel (9)

Wird 3(4), 8(9)-Bis(Carbonsäure)tricyclo[5.2.1.$0^{2,6}$]decan mit Methacryloylisocyanat umgesetzt, ergibt sich das Imid der Formel (10). Die reaktiven Wasserstoffatome am Stickstoff können auch hier weiter in Isocyanatreaktionen umgesetzt werden.

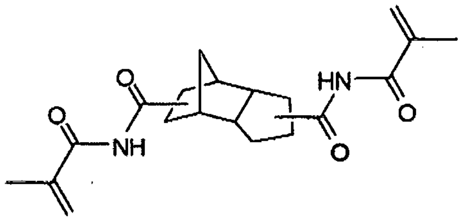

Formel (10)

3.) Ausgehend von 3(4), 8(9)-Bis(isocyanatomethyl)tricyclo[5.2.1.$0^{2,6}$]decan

Das 3(4), 8(9)-Bis(isocyanatomethyl)tricyclo[5.2.1.$0^{2,6}$]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Die Durchführung der zweiten Umsetzungsstufe der Isocyanat-Alkoholreaktion kann nicht nur ausgehend vom Tricyclodecandiol mit dem Isocyanatoethylmethacrylat, sondern auch ausgehend vom Tricyclodecandiisocyanat und Hydroxyethylmethacrylat eingeleitet werden. Durch stöchiometrische Umsetzung der beiden Reaktanden erhält man das Urethan der Formel (11).

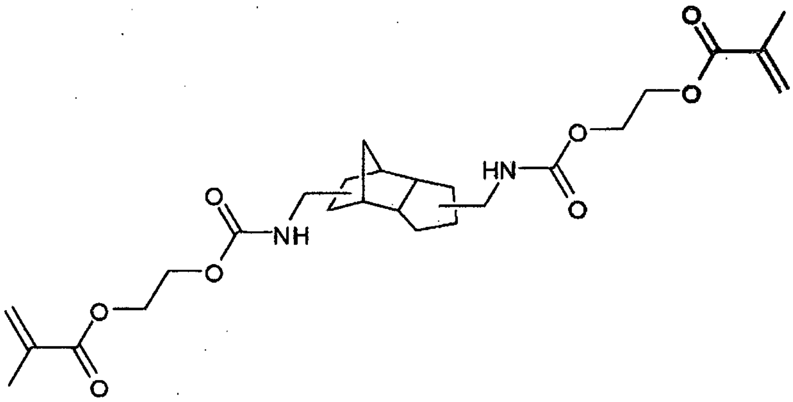

Formel (11)

Auch dieses Carbamat (Formel (11)) weist zwei reaktionsfähige Wasserstoffatome am Stickstoff auf, die mit einem Überschuss an Bis(isocyanatomethyl)tricyclo[5.2.1.$0^{2,6}$]decan zu dem Diisocyanat der Formel (12) weiter umgesetzt werden kann.

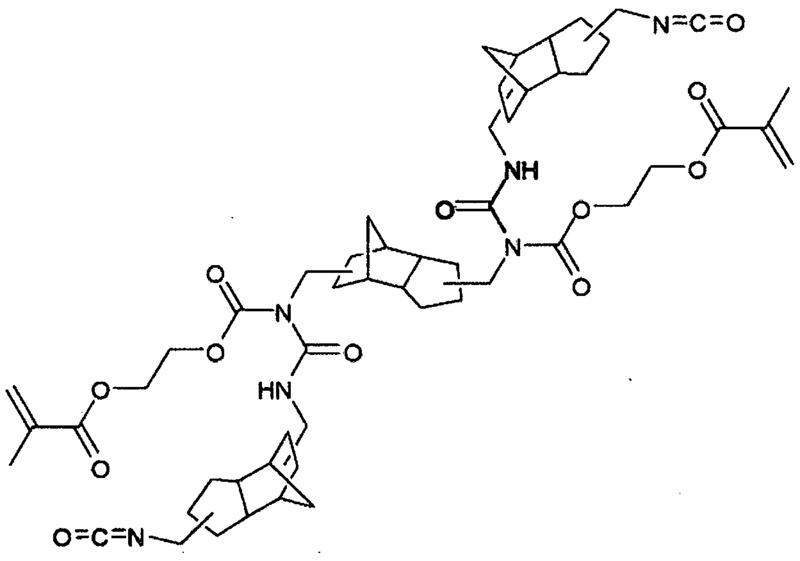

Formel (12)

Umsetzung des Allophanat-Diisocyanats (Formel (12)) mit Methacrylsäure liefert die Verbindung der Formel (13).

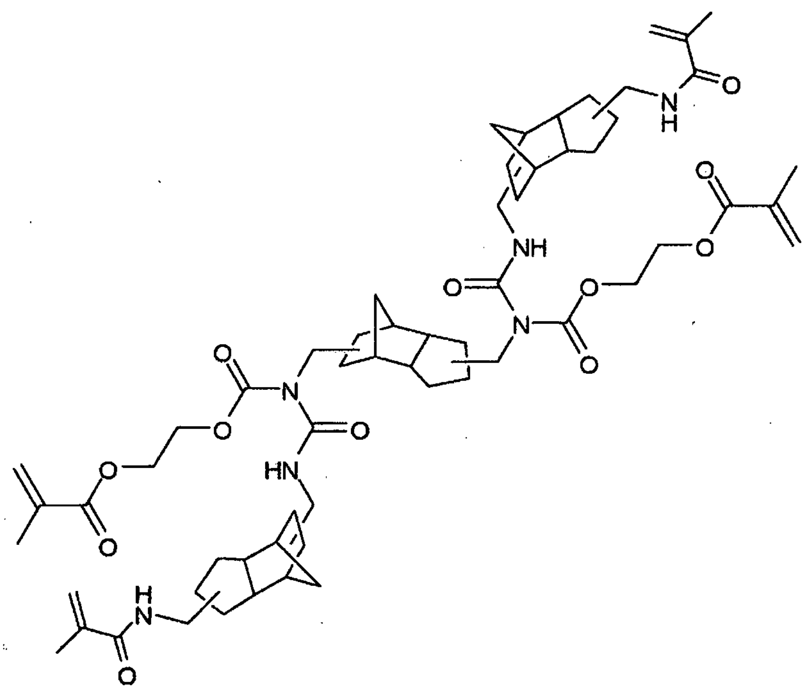

Formel (13)

Anstelle von Hydroxyethylmethacrylat können in den oben exemplarisch dargelegten Umsetzungen auch andere Hydroxylverbindungen von (Meth)acrylaten eingesetzt werden, wobei auch Gemische von Acrylaten und Methacrylaten verwendet werden können. So kann - analog dem obigen Beispiel - 3(4), 8(9)-Bis(isocyanatomethyl)-tricyclo[5.2.1.$0^{2,6}$]decan umgesetzt werden. Dabei bevorzugte Hydroxylverbindungen von (Meth)acrylaten sind die oben explizit genannten.

Diese Verbindungen weisen sowohl (Meth)acrylatgruppen als auch Hydroxygruppen auf. Letztere können mit Isocyanatgruppen in der oben für die Reaktion zwischen Hydroxyethylmethacrylat und 3(4), 8(9)-Bis(isocyanatomethyl)-tricyclo[5.2.1.$^{2,6}$]decan beschriebenen Weise reagieren. So lässt sich in einem einzigen Reaktionsschritt ein hoher Funktionalisierungsgrad erreichen.

3(4), 8(9)-Bis(isocyanatomethyl)tricyclo[5.2.1.0$^{2,6}$]decan kann mit 2-Carbonsäureethylmethacrylat zum entsprechenden Amid der Formel (16) umgesetzt werden.

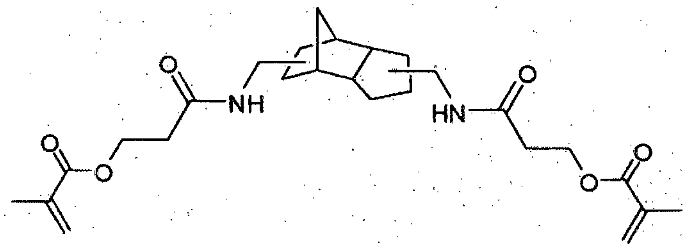

Formel (16)

Umsetzung des Amids der Formel (16) mit 2-Isocyanatoethylmethacrylat liefert den Acylharnstoff der Formel (17).

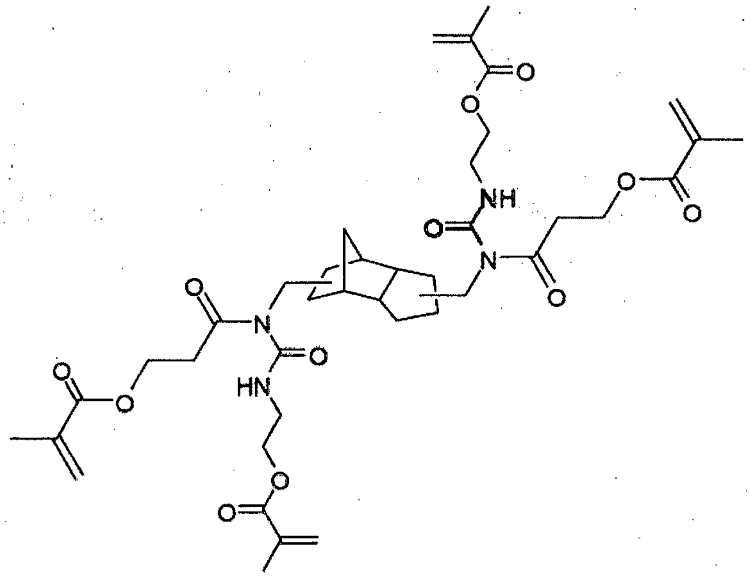

Formel (17)

Das Amid der Formel (16) kann auch mit einem Überschuss an 3(4), 8(9)-Bis(isocyanatomethyl)tricyclo[5.2.1.0$^{2,6}$]decan zum entsprechenden Isocyanat umgesetzt werden, wobei das gebildete Isocyanat mit Hydroxyethylmethacrylat weiter zum vernetzbaren Monomer der Formel (18) weiter umgesetzt wird.

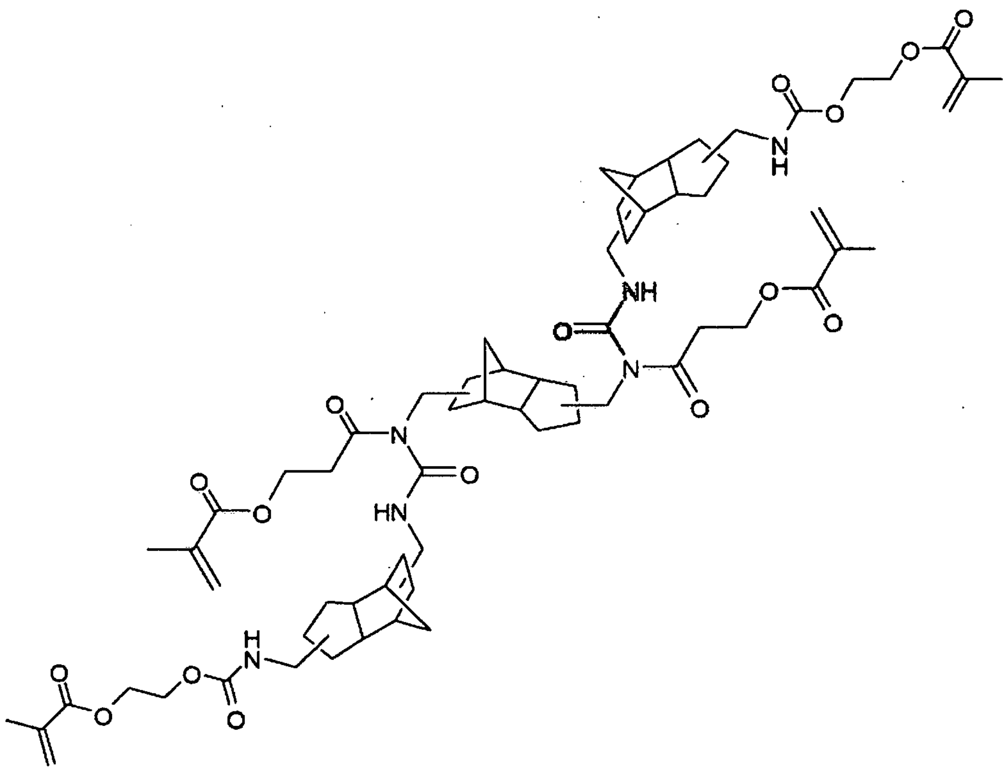

Formel (18)

Wird 3(4), 8(9)-Bis(isocyanatomethyl)tricyclo[5.2.1.0$^{2,6}$]decan mit 2-Methacryloyloxyethylhydrogensuccinat umgesetzt, so erhält man das Amid der Formel (19), das weiter mit 2-Isocyanatoethylmethacrylat zum Acylharnstoff der Formel (20) abreagiert wird.

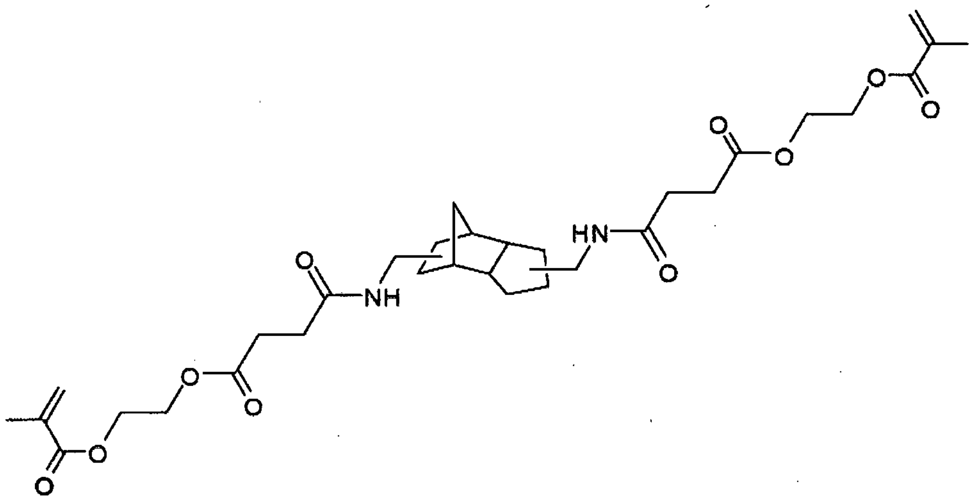

Formel (19)

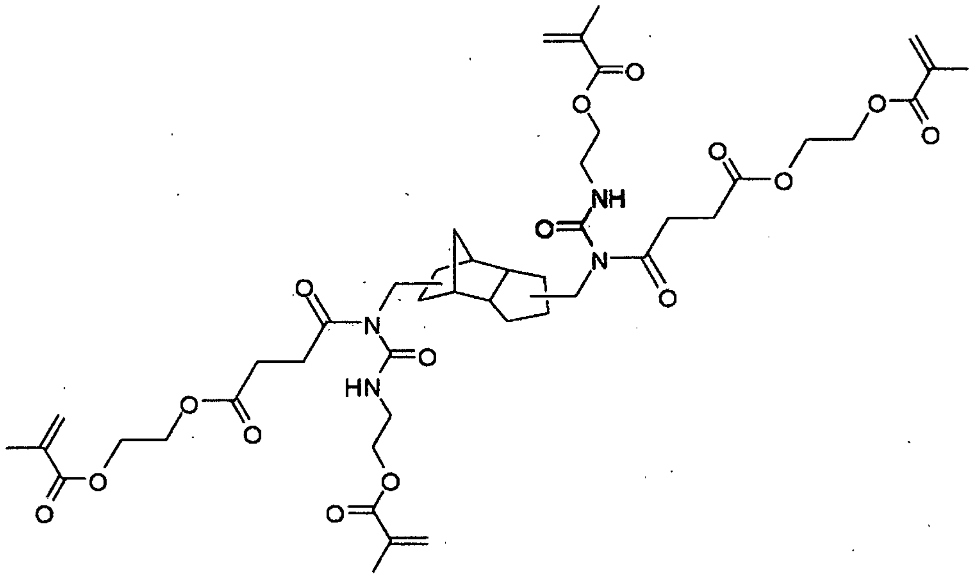

Formel (20)

Weitere geeignete Carbonsäuremethacrylate können durch Reaktionen von Di- oder Tetracarbonsäuremono- oder dianhydrid mit geeigneten OH-funktionalisierten, härtbaren Verbindungen wie beispielsweise 2-Hydroxyethylmethacrylat erhalten werden.

4.) Ausgehend von 3(4), 8(9)-Bis(Aminomethyl)tricyclo[5.2.1.$0^{2.6}$]decan

Das 3(4), 8(9)-Bis(Aminomethyl)tricyclo[5.2.1.$0^{2.6}$]decan ist an sich bekannt oder kann beispielsweise durch Umsetzung der entsprechenden Tosylate mit Ammoniak hergestellt werden. Reaktion des 3(4), 8(9)-Bis(Aminomethyl)tricyclo[5.2.1.$0^{2.6}$]decans mit 2-Isocyanatoethylmethacrylat ergibt die aus der EP 0 209 700 A2 bekannte Hamstoffverbindung der Formel (26).

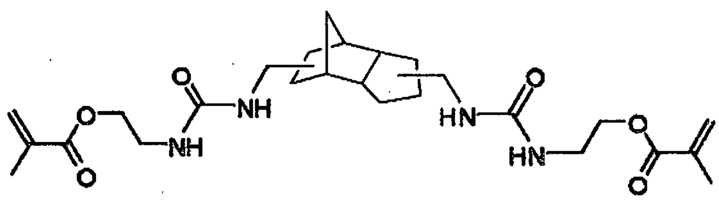

Formel (26)

Auch hier befinden sich noch aktive, reaktionsfähige Wasserstoffatome am Stickstoff, die mit einem Überschuss an Isocyanat beispielsweise zum Biuret der Formel (27) reagieren.

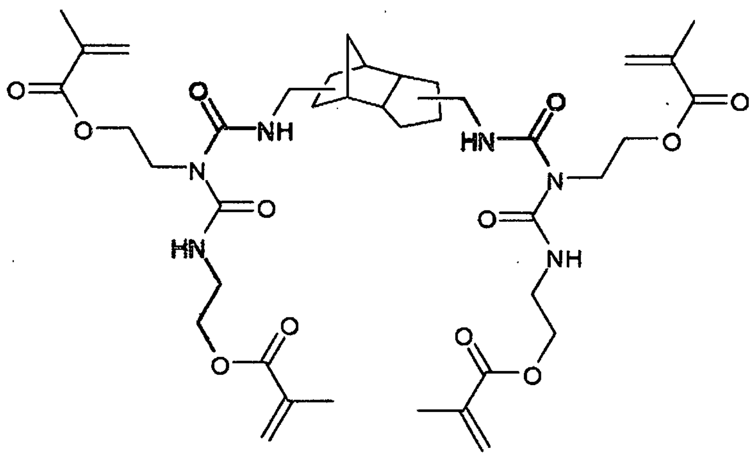

Formel (27)

Das 3(4), 8(9)-Bis(Aminomethyl)tricyclo[5.2.1.0$^{2,6}$]decan kann auch mit Methacryloylisocyanat zum entsprechenden Acylharnstoff umgesetzt werden. Die weitere Reaktion der verbleibenden reaktiven am Stickstoff befindlichen Wasserstoffatome mit Methacryloylisocyanat liefert das Biuret der Formel (28).

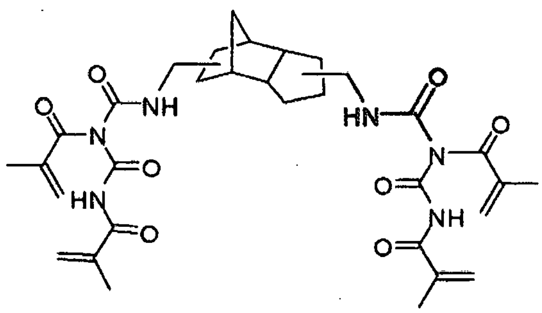

Formel (28)

Analog zu den vorstehend beschriebenen Monomeren, welche ein vom Tricyclo[5.2.1.0$^{2,6}$]decan abgeleitetes polyalicyclisches Strukturelement Q umfassen, können auch Monomere hergestellt werden, welche ein vom Tricyclo[3.3.1.1$^{3,7}$]decan (Adamantan) abgeleitetes polyalicyclisches Strukturelement Q umfassen. Als Beispiele seien folgende Reaktionsprodukte gezeigt:

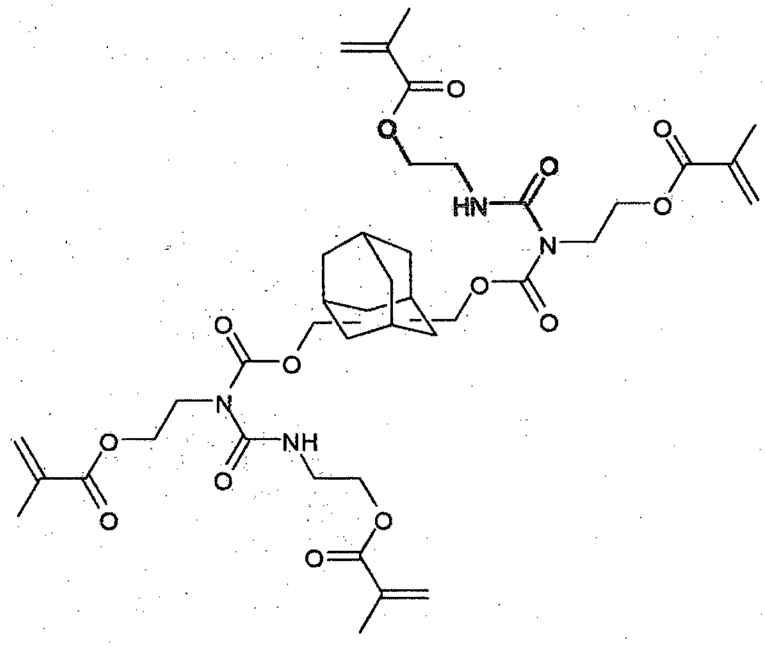

Formel (29)

Die Umsetzung der Verbindung der Formel (11) mit Diisocyanatoadamantan [(Bis(isocyanatomethyl)tricyclo[3.3.1.1$^{3,7}$]decanl liefert ein offenbartes Monomer, dessen Molekül zwei voneinander verschiedene polyalicyclische Strukturelemente Q umfasst, wie die nachfolgende Strukturformel der Verbindung der Formel (69) zeigt.

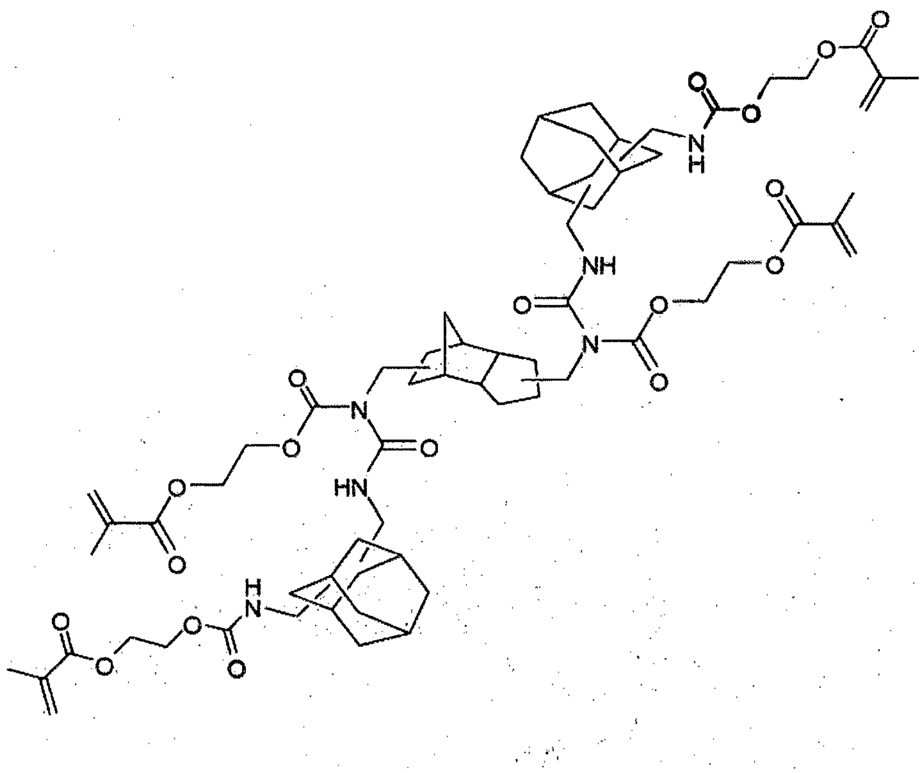

Formel (69)

Komponente (a2): weitere radikalisch polymerisierbare Monomere aus der Gruppe bestehend aus Acrylaten und Methacrylaten, vorzugsweise der Gruppe der Methacrylate

Den nicht zu Komponenten (a1) zählbaren optionalen Bestandteil der matrixbildenden Monomerenmischung bilden radikalisch polymerisierbare Monomere ausgewählt aus der Gruppe bestehend aus Acrylaten und Methacrylaten.
Die radikalisch polymerisierbaren Monomere der Komponente (a2) weisen vorzugsweise mindestens zwei ethylenische Gruppen auf.
In einer bevorzugten erfindungsgemäßen Zusammensetzung umfasst Monomerenkomponente (a2) oder besteht Monomerenkomponente (a2) aus

(a2) einem oder mehreren Polyalkylenglykoldi(meth)acrylaten, vorzugsweise aus einem oder mehreren Polyethylenglykoldi(meth)acrylaten.

Erfindungsgemäß bevorzugt ist eine Zusammensetzung, wobei die Monomerenkomponente (a2) ein oder mehrere Polyalkylenglykoldi(meth)acrylate mit 4 bis 10 Alkylenoxideinheiten enthält oder aus diesen besteht, vorzugsweise ein oder mehrere Polyethylenglykoldi(meth)acrylate mit 4 bis 10 Ethylenoxideinheiten enthält oder aus diesen besteht.
Bevorzugte Polyalkylenglykoldi(meth)acrylate entsprechen dabei der Formel

$$CH_2=CH(R^b)-C(=O)-O-[-(CH_2)_q-CH(R^8)-O-]_xC(=O)-CH(R^c)=CH_2$$

wobei gilt

$R^e$ ist Wasserstoff oder C1-C4-Alkyl,
$R^b$ und $R^c$ sind jeweils unabhängig voneinander Wasserstoff oder Methyl, der Index q ist eine natürliche Zahl ausgewählt aus der Gruppe der natürlichen Zahlen von 1 bis 5, vorzugsweise 1, 3 oder 4,
und
der Index x ist eine natürliche Zahl ausgewählt aus der Gruppe der natürlichen Zahlen von 2 bis 18, vorzugsweise von 3 bis 12.

Vorzugsweise ist der Index q = 1.
Weiter bevorzugte Polyalkylenglykoldi(meth)acrylate entsprechen daher der Formel

$$CH_2=CH(R^b)-C(=O)-O-[-CH_2-CH(R^8)-O-]_xC(=O)-CH(R^c)=CH_2$$

wobei

$R^1$, $R^b$ und $R^c$ sowie x jeweils die oben angegebene Bedeutung haben.

Vorzugsweise ist der Index x eine natürliche Zahl ausgewählt aus der Gruppe der natürlichen Zahlen von 4 bis 10.
$R^a$ ist vorzugsweise Wasserstoff oder Methyl.
Besonders bevorzugt als Monomere der Komponente (a2) sind Polyethylenglykoldi(meth)acrylate mit 4 bis 10 Ethylenoxideinheiten (d.h. Monomere der oben angegebenen Formel, in denen $R^a$ Wasserstoff und x = 4 - 10 ist).
Ganz besonders bevorzugt als Monomere der Komponente (a2) sind Polyethylenglykoldimethacrylate mit 4 bis 10 Ethylenoxideinheiten (d.h. Monomere der oben angegebenen Formel, in denen $R^a$, $R^b$ und $R^c$ jeweils Wasserstoff und x = 4 - 10 ist).
In der Patentliteratur ist eine Vielzahl von Diacrylat- und Dimethacrylat-Monomeren genannte (beispielsweise auch in der DE 39 41 629 A1, insbesondere die Offenbarung im Bereich von Spalte 6, Zeile 15 bis Spalte 8, Zeile 10), die sich zum Einsatz in einer erfindungsgemäßem Zusammensetzung eignen.
Erfindungsgemäßen Zusammensetzungen können als Komponente (a2) auch ein oder mehrere Dimethacrylat-Monomere enthalten, vorzugsweise gewählt aus der Gruppe bestehend aus Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat und Glycerindimethacrylat.
Bisphenol-A-Glycidyl-Methacrylat (Bis-GMA) kann zwar eingesetzt werden. Vorzugsweise enthält eine erfindungsgemäße Zusammensetzung jedoch nicht die Verbindung Bis-GMA. Vorzugsweise ist eine erfindungsgemäße Zusammensetzung frei von sämtlichen Verbindungen mit einem Bisphenol-A-Strukturelement.

Die radikalisch polymerisierbaren Monomere der Komponente (a2), die somit nicht zu Komponente (a1) zählen, können auch Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropytmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Als weiterer Bestandteil können auch lichthärtbare Acrylat- oder Methacrylat-Monomere auf Basis von Polysiloxanen verwendet werden, wie sie beispielsweise in der DE 199 03 177 oder in der DE 44 16 857 beschrieben sind.

Bestandteil (b): Initiatoren und/oder Katalysatoren

Eine erfindungsgemäße Zusammensetzung ist vorzugsweise lichthärtbar und/oder chemisch härtbar. Bevorzugt ist eine erfindungsgemäße Zusammensetzung, wobei Bestandteil (b) einen oder mehrere Lichthärtungsinitiatoren und/oder einen oder mehrere Initiatoren zur chemischen Aushärtung umfasst oder aus diesen besteht.

Die Gesamtmenge des Bestandteils (b) liegt vorzugsweise im Bereich von 0,1 bis 3 Gew.-%, bevorzugt im Bereich von 0,25 bis 1,5 Gew.-%, jeweils bezogen auf die Gesamtmasse der erfindungsgemäßen Zusammensetzung.

Bevorzugte erfindungsgemäße Zusammensetzungen sind chemisch härtbar und umfassen einen oder mehrere Initiatoren für die chemische Aushärtung.

Bevorzugte erfindungsgemäße Zusammensetzungen sind lichthärtbar (photohärtbar) und umfassen einen oder mehrere Lichthärtungsinitiatoren.

Bevorzugte erfindungsgemäße Zusammensetzungen sind dualhärtend.

Dualhärtende erfindungsgemäße Zusammensetzungen umfassen einen oder mehrere Photoinitiatoren (Komponente (b-1) und einen oder mehrere Initiatoren zur chemischen Aushärtung (Komponente (b-2).

Besonders bevorzugte erfindungsgemäße Zusammensetzungen sind solche, wobei Komponente (b) einen oder mehrere radikalbildende Initiatoren und/oder Katalysatoren umfasst, vorzugsweise gewählt aus der Gruppe bestehend aus Photoinitiatoren, Hydroperoxiden und Thiohamstoffderivaten, dabei vorzugsweise mindestens einen Photoinitiator, ein Hydroperoxid und ein Thioharnstoffderivat, da mit solchen Zusammensetzungen besonders gute Ergebnisse im Sinne der vorliegenden Erfindung erzielt wurden.

Komponente (b-1) - Photoinitiatoren

Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben .

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen härtbaren Gemisches bewirken können.

Das Absorptionsmaximum von Campherchinon (CC) liegt bei ca. 470 nm und somit im Bereich des blauen Lichts. Campherchinon (CC) zählt zu den $PI_2$-Initiatoren und wird regelmäßig zusammen mit einem Coinitiator eingesetzt.

Vorzugsweise enthält eine erfindungsgemäße Zusammensetzung Campherchinon (CC).

Ebenfalls bevorzugt ist die Kombination eines alpha-Diketons und eines aromatischen tertiären Amins, weiter bevorzugt ist dabei die Kombination von Campherchinon (CC) und Ethyl-*p-N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in erfindungsgemäßen härtbaren Gemischen wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in den erfindungsgemäßen Gemischen.

Alternativ können auch Boratsalze, wie sie beispielsweise in US 4,772,530, US 4,954,414, US 4,874,450, US 5,055,372 und US 5,057,393 beschrieben sind, als Photo-initiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

<u>Komponente (b-2) - Initiatoren für die chemische Aushärtung</u>

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaftigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Besonders bevorzugte Initiatoren sind Hydroperoxide, dabei insbesondere Cumolhydroperoxid, vorzugsweise in Kombination mit Thioharnstoffderivaten, dabei insbesondere Allylthiohamstoff.

Die Hydroperoxide und die Thioharnstoffderivate werden dabei auf zwei unterschiedliche Komponenten einer erfindungsgemäßen Zusammensetzung aufgeteilt. Beim Mischen der Thiohamstoffderivat-haltigen Komponente mit der hydroperoxidhaltigen Komponente wird durch die Redoxreaktion die radikalische Reaktion initiiert.

In erfindungsgemäßen Zusammensetzungen hat sich das Redoxsystem Cumolhydroperoxid / Allylthiohamstoff als besonders vorteilhaft erwiesen. Dieses Redoxsystem ergab zusammen mit den erfindungsgemäß einzusetzenden Monomeren der Komponente (a1), vorzugsweise zusammen mit der Monomerenkomponente (a2), besonders geeignete erfindungsgemäße Zusammensetzungen welche in besonderem Maße die gewünschten Eigenschaften aufwiesen für ein Wurzelkanalfüll - und/oder -Versiegelungsmaterial.

Dualhärtende Systeme umfassen eine Kombination aus Photoinitiator(en) und Initiator(en) zur chemischen Aushärtung.

In einer ganz besonders bevorzugten Ausgestaltung umfasst eine erfindungsgemäßen Zusammensetzung Campherchinon (CC), Ethyl-*p-N,N*-dimethylaminobenzoat (DABE), Cumolhydroperoxid und Allylthiohamstoff.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 14 95 520, WO 02/092021 oder in WO 02/092023.

Geeignete Malonylsulfamlde sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,8-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfemaphthenat.

<u>Bestandteil (c): Füllstoffkomponente</u>

Eine erfindungsgemäße Zusammensetzung kann vorzugsweise einen Anteil der Füllstoffkomponente (c) von bis zu 85 Gew.-% enthalten, bezogen auf die Gesamtmasse der erfindungsgemäßen Zusammensetzung, wobei die Füllstoffkomponente einen, zwei oder mehrere Füllstoffe umfassen kann.

Die Gesamtmenge der Füllstoffe der Komponente (c) liegt vorzugsweise im Bereich von 35 bis 85 Gew.-%,

bevorzugt im Bereich von 50 bis 80 Gew.-%, besonders bevorzugt im Bereich von 60 bis 75 Gew.-%, jeweils bezogen auf die Gesamtmasse der Zusammensetzung.

Die mittlere Partikelgröße $d_{50}$ der erfindungsgemäß einzusetzenden Füllstoffpartikel einer erfindungsgemäßen Zusammensetzung wird mittels Lichtstreuung (Laserbeugung) bestimmt, vorzugsweise mit einem Partikelgrößenmessgerät Beckman Coulter LS 13320.

Unter Nanopartikeln werden in Zusammenhang mit der vorliegenden Erfindung Partikel mit einer mittleren Partikelgröße von weniger als 200 nm verstanden.

Eine bevorzugte erfindungsgemäße Zusammensetzung enthält zusätzlich zu der Komponente (c1) der röntgenopaken Füllstoffe eine Komponente (c2) der nicht röntgenopaken Füllstoffe, wobei vorzugsweise Komponente (c2) besteht aus oder umfasst:

(c2-a) nicht röntgenopake, nicht agglomerierte nanoskalige Füllstoffe mit einer mittleren Partikelgröße kleiner 200 nm,
und/oder

(c2-b) nicht röntgenopake, agglomerierte Füllstoffpartikel, die vorzugsweise organisch oberflächenmodifiziert, bevorzugt silanisiert, sind.

Die Füllstoffkomponente (c) umfasst in einer bevorzugten Ausgestaltung einen oder mehrere röntgenopake Füllstoffe der Komponente (c1), vorzugsweise mit einer mittleren Partikelgröße im Bereich von 0,4 µm bis 10 µm, und einen oder mehrere Füllstoffe der Komponente (c2-b).

Die Füllstoffkomponente (c) umfasst in einer bevorzugten Ausgestaltung einen oder mehrere röntgenopake Füllstoffe der Komponente (c1), einen oder mehrere Füllstoffe der Komponente (c2-b) und einen oder mehrere Füllstoffe der Komponente (c2-a).

<u>Komponente (c1): Röntgenopake Füllstoffe</u>

Wie oben bereits erwähnt wird die Röntgenopazität üblicherweise in Aluminiumäquivalenten gemessen und in "mm (Al)" angegeben.

Diese Röntgenopazitätsangaben beziehen sich dabei nicht auf den reinen röntgenopaken Füllstoff, sondern auf ein Kompositmaterial, das 75,00 Gew.-% des röntgenopaken Füllstoffs enthält.

Röntgenopake Füllstoffe im Sinne des vorliegenden Textes weisen - gemessen in einer Polymermatrix, welche einen Anteil von 75,00 Gew.-% an röntgenopakem Füllstoff enthält - eine Röntgenopazität auf von zumindest 3 mm Aluminiumäquivalenten gemäß ISO 4049, d.h. von mindestens 3 mm (Al).

Vorzugsweise weisen röntgenopake Füllstoffe eine Röntgenopazität auf von mindestens 3,5 mm (Al), bevorzugt von 4 mm (Al), weiter bevorzugt von mindestens 5 mm (Al) und besonders bevorzugt von mindestens 6 mm (Al).

Um die Röntgenopazität eines Füllstoffs gemäß ISO 4049 zu bestimmen, wird der zu untersuchende Füllstoff, zusammen mit einer für die anschließende Härtung erforderlichen Menge eines oder mehrerer Initiatoren, in eine Monomermatrix eingearbeitet. Nach Härten der Mischung wird die Röntgenopazität (in Aluminiumäquivalenten) des resultierenden Materials, welches einen Anteil von 75,00 Gew.-% des zu untersuchenden Füllstoffs enthält, gemäß ISO 4049 bestimmt. Die Monomer- bzw- Polymermatrix selbst trägt nicht zur Röntgenopazität bei.

Insbesondere beziehen sich die hier angegebenen und gemäß ISO 4049 bestimmten Röntgenopazitätswerte auf ein gehärtetes Kompositmaterial, hergestellt durch Lichthärten der folgenden Zusammensetzung:

24,775 g Bis(methacryloyloxymethyotricyclo[5.2.1.0$^{2,6}$]decan, 0,090 g CC, 0,135 g DABE und 75,00 g zu untersuchender Füllstoff.

Die Füllstoffkomponente (c) einer erfindungsgemäßen Zusammensetzung enthält einen, zwei oder mehrere röntgenopake Füllstoffe als Komponente (c1). Der oder die röntgenopaken Füllstoffe sind vorzugsweise ausgewählt aus der Gruppe bestehend aus Zink, Ytterbium, Yttrium, Zirkonium, Strontium, Caicium, Titan, Wolfram, Tantal, Niob, Barium, Wismut, Molybdän, Lanthan, Cer in Form von Pulvern, Legierungen, Oxiden, Halogeniden, Oxohalogeniden, Sulfaten, Phosphaten, Silikaten, Carbonaten, Wolframaten oder Gläsern und deren Mischungen.

Bevorzugte röntgenopake Füllstoffe sind ausgewählt aus der Gruppe bestehend aus Zink, Ytterbium, Yttrium, Zirkonium, Strontium, Calcium, Titan, Wolfram, Tantal, Niob, Barium, Wismut, Molybdän in Form von Legierungen, Oxiden, Fluoriden, Oxohalogeniden, Sulfaten, Phosphaten, Silikaten, Carbonaten, Wolframaten oder Gläsern und deren Mischungen.

Vorteilhafte röntgenopake Füllstoffe sind dabei $CaWO_4$, $ZrO_2$, Ytterbiumfluorid, Bariumsulfat und/oder röntgenopake Gläser.

Die Gesamtmenge der röntgenopaken Füllstoffe der Komponente (c1) liegt vorzugsweise im Bereich von 30 bis 75 Gew.-%, bevorzugt im Bereich von 40 bis 70 Gew.-%, weiter bevorzugt im Bereich von 50 bis 70 Gew.-%, besonders bevorzugt im Bereich von 55 bis 65 Gew.-%, jeweils bezogen auf die Gesamtmasse der Zusammensetzung.

Die röntgenopaken Füllstoffe der Komponente (c1) werden vorzugsweise in Form von Mikropartikeln mit einer mittleren Partikelgröße im Bereich von 0,4 $\mu$m bis 10 $\mu$m eingesetzt.

Die röntgenopaken Füllstoffe der Komponente (c1) weisen vorzugsweise eine mittlere Partikelgröße im Bereich von 2 $\mu$m bis 8 $\mu$m auf, vorzugsweise eine mittlere Partikelgröße im Bereich von 3 $\mu$m bis 7 $\mu$m.

Die Mikropartikel können eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden.

Eine erfindungsgemäße Zusammensetzung kann Mikropartikel verschiedener Fraktionen enthalten, wobei sich die mittleren Partikelgrößen der Fraktionen unterscheiden, wobei deren mittlere Partikelgrößen um mindestens 0,5 $\mu$m, bevorzugt um mindestens 0,7 $\mu$m, voneinander abweichen.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unter-scheiden.

Zur besseren Einbindung in die Polymermatrix einer erfindungsgemäßen Zusammensetzung können die Mikropartikel organisch oberflächenmodifiziert sein. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders Methacryloxypropyltrimethoxysilan.

In einer besonders bevorzugten erfindungsgemäßen Zusammensetzung wird zumindest ein Teil der Mikropartikel der röntgenopaken Füllstoffe durch organisch oberflächenmodifizierte Partikel, vorzugsweise silanisierte Partikel gebildet und/oder es wird zumindest ein Teil der Mikropartikel der röntgenopaken Füllstoffe durch Dentalglas-Partikel gebildet; vorzugsweise sind zumindest ein Teil der Mikropartikel der röntgenopaken Füllstoffe organisch oberflächenmodifizierte Dentalglas-Partikel, vorzugsweise silanisierte Dentalglas-Partikel.

Die röntgenopaken Füllstoffe der Komponente (c1) können auch in Form von röntgenopaken agglomerierten und/oder röntgenopaken nicht agglomerierten Nanopartikeln eingesetzt werden.

Vorzugsweise ist die mittlere Partikelgröße der röntgenopaken Nanopartikel kleiner als 100 nm und besonders bevorzugt kleiner 60 nm.

In einer bevorzugten Ausgestaltung liegen die röntgenopaken Nanopartikel in nicht agglomerierter Form vor, beispielsweise dispergiert in einem Medium, vorzugsweise in monodisperser Form.

Eine erfindungsgemäße Zusammensetzung kann in einer Ausgestaltung einen oder mehrere röntgenopaken Füllstoffe der Komponente (c1) enthalten, wobei ein Teil der röntgenopaken Füllstoffe in Form von Mikropartikeln (vorzugsweise mit einer mittleren Partikelgröße in dem oben angegebenen Bereich) und ein Teil der röntgenopaken Füllstoffe in Form von Nanopartikeln eingesetzt wird, vorzugsweise in Form von nicht agglomerierten röntgenopaken Nanopartikeln.

Die Materialien für die erfindungsgemäß einzusetzenden röntgenopaken Nanopartikel sind vorzugsweise Oxide oder Mischoxide und bevorzugt ausgewählt aus der Gruppe bestehend aus Oxiden und Mischoxiden der Elemente Yttrium, Strontium, Barium, Zirkonium, Niob, Tantal, Wolfram, Wismut, Molybdän, Zink, Ytterbium, Lanthan, Cer und deren Mischungen. Die bevorzugten oxidischen Nanopartikel sind dabei wie dargelegt nicht agglomeriert.

Um eine gute Einbindung der röntgenopaken Nanopartikel in die Polymermatrix einer erfindungsgemäßen Zusammensetzung zu erreichen, sind die Oberflächen der röntgenopaken Nanopartikel (vorzugsweise der bevorzugten röntgenopaken oxidischen Nanopartikel) organisch modifiziert, d.h. ihre Oberflächen weisen organische Strukturelemente auf. Beispielhaft genannt sei die Oberflächenbehandlung der Füllstoffe mit einem Silan. Als Haftvermittler eignet sich besonders das Methacryloxypropyltrimethoxysilan.

<u>Komponente (c2): nicht röntgenopake Füllstoffe</u>

<u>Komponente (c2-a): nicht röntgenopake, nicht agglomerierte Nanopartikel</u>

Die Füllstoffkomponente (c) kann vorzugsweise einen Füllstoff (c2-a) in Form nicht röntgenopaker, nicht agglomerierter Nanopartikel mit einer mittleren Partikelgröße kleiner 200 nm umfassen.

Vorzugsweise ist dabei die mittlere Partikelgröße kleiner als 100 nm und besonders bevorzugt kleiner 60 nm.

Die nicht röntgenopaken, nicht agglomerierten Nanopartikel der Komponente (c2-a) sind vorzugswiese oberflächenmodifiziert, vorzugsweise organisch oberflächenmodifiziert, bevorzugt silanisiert.

Umfasst eine erfindungsgemäße Zusammensetzung neben den röntgenopaken Füllstoffen der Komponente (c1) zusätzlich nicht agglomerierte Nanopartikel als weiteren Füllstoff der Komponente (c2-a), werden - augenscheinlich bedingt durch eine bessere Raumerfüllung der Füllstoffteilchen insgesamt - die mechanischen Eigenschaften erhöht bei gleichzeitig signifikant reduzierter Löslichkeit.

Die Füllstoffkomponente (c) einer erfindungsgemäßen Zusammensetzung kann einen oder mehrere Füllstoffe der Komponente (c2-a) in einer Menge im Bereich von 0 bis 12 Gew.-% umfassen, vorzugsweise in einer Menge im Bereich von 0 bis 8 Gew.-%, jeweils bezogen auf die Gesamtmasse der Zusammensetzung.

Eine erfindungsgemäße Zusammensetzung umfasst vorzugsweise Komponente (c2-b), die nicht agglomerierte silanisierte Kieselsäure-Nanopartikel umfasst oder aus diesen besteht.

Bevorzugt enthält eine erfindungsgemäße Zusammensetzung nicht agglomerierte silanisierte Kieselsäure-Nanopartikel in einer Menge im Bereich von 2 bis 12 Gew.-%, weiter bevorzugt im Bereich von 3 bis 10 Gew.-%, besonders bevorzugt im Bereich von 4 bis 8 Gew.-%, bezogen auf die Gesamtmasse der Zusammensetzung.

Dabei wird die silanisierte Kieselsäure vorzugsweise in Form von silanisierten Nanopartikeln mit einer mittleren Partikelgröße von kleiner 100 nm, bevorzugt kleiner 60 nm eingesetzt.

In einer bevorzugten Ausgestaltung liegen die nicht agglomerierten nanoskaligen Teilchen beispielsweise dispergiert in einem Medium vor, vorzugsweise in monodisperser Form.

Die Materialien für die erfindungsgemäß einzusetzenden Nanopartikel sind vorzugsweise Oxide oder Mischoxide und bevorzugt ausgewählt aus der Gruppe bestehend aus Oxiden und Mischoxiden der Elemente Silizium und/oder Aluminium. Die bevorzugten oxidischen Nanopartikel sind dabei wie dargelegt nicht agglomeriert.

Um eine gute Einbindung der Nanopartikel in die Polymermatrix einer erfindungsgemäßen Zusammensetzung zu ermöglichen, sind die Oberflächen der Nanopartikel (vorzugsweise der bevorzugten oxidischen Nanopartikel organisch modifiziert, d.h. ihre Oberflächen weisen organische Strukturelemente auf. Beispielhaft genannt sei die Oberflächenbehandlung der Füllstoffe mit einem Silan. Als Haftvermittler eignet sich besonders das Methacryloxypropyltrimethoxysilan.

<u>Komponente (c2-b): nicht röntgenopake, agglomerierte Füllstoffpartikel</u>

Die Füllstoffkomponente (c) umfasst vorzugsweise einen Füllstoff (c2-a) in Form nicht röntgenopaker, agglomerierter Füllstoffpartikel, vorzugsweise in Form agglomerierter Nanopartikel.

Die nicht röntgenopaken, agglomerierten Füllstoffpartikel Komponente (c2-b) sind vorzugsweise agglomerierte organisch oberflächenmodifizierte Nanopartikel, die bevorzugt silanisiert, sind.

Die Materialien für die erfindungsgemäß einzusetzenden nicht röntgenopaken Nanopartikel der Komponente (c2-b) sind vorzugsweise Oxide oder Mischoxide und bevorzugt ausgewählt aus der Gruppe bestehend aus Oxiden und Mischoxiden der Elemente Silizium und/oder Aluminium. Die bevorzugten oxidischen Nanopartikel sind dabei wie dargelegt agglomeriert.

Erfindungsgemäße Zusammensetzungen mit einer Füllstoffkomponente (c) umfassend eine Mischung enthaltend Komponente (c1) und einen oder mehrere Füllstoffe der Komponente (c2-b) zeigten eine für den Einsatz als Wurzelkanalfüllungs- oder versiegelungsmaterial verbesserte Rheologie.

Die Füllstoffkomponente (c) einer erfindungsgemäßen Zusammensetzung kann einen oder mehrere Füllstoffe der Komponente (c2-b) in einer Menge im Bereich von 0 bis 12 Gew.-% umfassen, vorzugsweise in einer Menge im Bereich von 0 bis 8 Gew.-%, jeweils bezogen auf die Gesamtmasse der Zusammensetzung.

Eine erfindungsgemäße Zusammensetzung umfasst vorzugsweise agglomerierte silanisierte Kleselsäure-Nanopartikel der Komponente (c2-b), bevorzugt in einer Menge im Bereich von 1 bis 8 Gew.-%, weiter bevorzugt im Bereich von 2 bis 6 Gew.-%, jeweils bezogen auf die Gesamtmasse der Zusammensetzung.

<u>Weitere optionale Füllstoffe des Bestandteils (c2)</u>

Die Füllstoffkomponente (c2) einer erfindungsgemäßen Zusammensetzung kann ferner weitere nicht röntgenopake Füllstoffe enthalten, die nicht Bestandteil der Komponenten (c2-a) oder (c2-b) sind.

Vorzugsweise sind diese weiteren nicht röntgenopaken Füllstoffe, wenn überhaupt, in einer erfindungsgemäße Zusammensetzung nur in geringer Menge enthalten, vorzugsweise in einer Menge von 0 bis 3 Gew.-%, bevorzugt in einer Menge von 0 bis 2 Gew.-%, weiter bevorzugt in einer Menge von 0 bis 1 Gew.-%, jeweils bezogen auf die Gesamtmasse der Zusammensetzung.

Daneben können z.B. verstärkend wirkende Füllstoff-Materialien, wie Glasfasern, Polyamid- oder Kohlenstofffasern eingesetzt werden. Eine erfindungsgemäße Zusammensetzung kann auch feinteilige Splitter oder Perlpolymerisate enthalten, wobei die Perlpolymerisate Homo- oder Copolymere organischer härtbarer Monomerer sein können.

Bestandteil (d): Molekulargewichtsregler

Eine erfindungsgemäße Zusammensetzung kann einen oder mehrere Molekulargewichtsregler enthalten.

US 4,490,497 A offenbart Zusammensetzungen für chirurgischen Zement, die bei der Herstellung dentaler Prothesen eingesetzt werden können. Eine Flüssigkomponente der Zusammensetzung umfasst als "chain stopping agent" beispielsweise ein doppelt ungesättigtes monocyclisches Terpen oder ein einfach ungesättigtes bicyclisches Terpen. Dort nicht offenbart sind Zusammensetzungen, die zum Füllen und/oder Versiegeln eines Wurzelkanals geeignet sind.

Umfasst eine erfindungsgemäße Zusammensetzung zudem einen Molekulargewichtsregler, kann der Polymerisationsverlauf beeinflusst und Kettenlänge, Vemetzungsgrad des resultierenden Polymerisats sowie Kinetik des Polymerisationsverlaufs in einer Weise geändert werden, dass die gewünschten Eigenschaften weiter verbessert werden. Dabei reduzieren sich die mechanischen Werte deutlich und gleichzeitig werden die Wasseraufnahme und Fließfähigkeit überraschenderweise verbessert.

Die Gesamtmenge des oder der Molekulargewichtsregler der Komponente (d) einer erfindungsgemäßen Zusammensetzung liegt vorzugsweise im Bereich von 0,01 bis 1 Gew.-%, bevorzugt in einer Menge im Bereich von 0,025 bis 0,75 Gew.-%, weiter bevorzugt in einer Menge im Bereich von 0,03 bis 0,50 Gew.-%, jeweils bezogen auf die Gesamtmasse der Zusammensetzung.

Geeignete Molekulargewichtsregler sind beispielsweise diverse Terpene, insbesondere Terpinene ($\alpha$-Terpinen, $\beta$-Terpinen, $\gamma$-Terpinen), Phellandrene ($\alpha$-Phellandren, $\beta$-Phellandren) und Terpinolen (auch $\delta$-Terpinen genannt), 1,4-Cyclohexadien (gegebenenfalls substituiert), 1,3-Cyclohexadien (gegebenenfalls substituiert), 1,4-Dihydronaphtalin, 1,4,5,8-Tetrahydronaphtalin, 2,5-Dihydrofuran oder dimeres Systyrol sowie Linolsäure und $\alpha$-Linolensäure.

Erfindungsgemäß bevorzugte Molekulargewichtsregler sind Verbindungen mit zwei oder mehr Doppelbindungen, vorzugsweise Verbindungen mit zwei oder mehr Doppelbindungen, die durch Abstraktion eines Allyl-H-Atoms ein delokalisiertes Elektronensystem ausbilden können, das sich über 5 oder mehr C-Atome erstreckt, bevorzugt Diene, die durch Abstraktion eines Allyl-H-Atoms ein delokalisiertes Elektronensystem ausbilden können, das sich über 5 C-Atome erstreckt.

Erfindungsgemäß bevorzugt als Bestandteil eines erfindungsgemäßen Wurzelkanalversiegelungs- und/oder Füllungsmaterials sind Molekulargewichtsregler ausgewählt aus der Gruppe der Monoterpene, vorzugsweise aus der Gruppe der Monoterpen-Diene, bevorzugt aus der Gruppe bestehend aus $\alpha$-Terpinen, $\beta$-Terpinen, $\gamma$-Terpinen, $\alpha$-Phellandren, $\beta$-Phellandren und Terpinolen oder sind ausgewählt aus der Gruppe bestehend aus Linolsäure, Linolensäure und sonstigen substituierten oder nicht substituierten Cyclohexadienen.

Erfindungsgemäß besonders bevorzugte Molekulargewichtsregler sind $\gamma$-Terpinen und $\alpha$-Terpinen.

Selbstverständlich können auch Mischungen von Molekulargewichtsreglern eingesetzt werden.

Bestandteil (e): Wirkstoffe

Eine erfindungsgemäße Zusammensetzung kann einen oder mehrere Wirkstoffe enthalten ausgewählt aus der Gruppe bestehend aus remineralisierenden, therapeutischen, devitalisierenden, desinfizierenden, entzündungshemmenden, antibakteriellen und kariostatischen Wirkstoffen.

Die erfindungsgemäßen Zusammensetzungen können therapeutisch wirksame und/oder desinfizierende Wirkstoffe wie Benzalkoniumchlorid, Chlorhexidin, Nanosilber, Nanokupfer und/oder devitalisierende und/oder antibakterielle und/oder entzündungshemmende und/oder kariostatische Zusätze enthalten.

Die erfindungsgemäßen Zusammensetzungen können ferner bioaktive und/oder remineralisiernde Zusätze wie Calciumcarbonat, Calciumhydroxid, Calciumoxid, Tricalciumphosphat, Tetracalciumphosphat, Calciumhydroxylapatit, Calcium/Strontiumapatit oder Fluorapatit enthalten. Es können dabei ferner Zinkoxid sowie ionenabgebende Füllstoffe wie Glasionomerzementgläser zusammen mit bioaktiven und/oder remineralisierenden Zusätzen verwendet werden.

Bestandteil (f): Haftung fördernde Additive

Eine erfindungsgemäße Zusammensetzung kann ein oder mehrere Additive zur Verbesserung der Haftung (sogenannte Haftmonomere) enthalten, insbesondere zur Verbesserung der Haftung an Dentin.

Obwohl die erfindungsgemäßen Zusammensetzungen leicht aus dem Wurzelkanal entfernbar sein müssen, benötigen sie mitunter eine geringe Menge eines haftfördernden Additivs (sogenannte Haftmonomere), das die abschlussdichte Haftung einer erfindungsgemäßen Zusammensetzung am Dentin weiter verbessert.

Eine erfindungsgemäße Zusammensetzung kann zur Verbesserung der Haftung als Bestandteil (f) ein oder mehrere säuregruppenhaltige Acrylate- und/oder MethacrylatMonomere enthalten. Solche säuregruppenhaltigen Monomere können vorzugsweise eine Carbonsäure-, eine Polycarboxylsäure-, eine Phosphorsäure-, eine Phosphonsäure-Polyphosphonsäure-, eine Sulfonsäure- und/oder eine Thiophosphorsäurefunktion aufweisen. Das Monomer kann eine oder eine Vielzahl von Säurefunktionen in einem Molekül enthalten.

Eine bevorzugte erfindungsgemäße Zusammensetzung umfasst ein oder mehrere haftfördernde Additive, ausgewählt aus der Gruppe bestehend aus polymerisierbaren oder nicht polymerisierbaren Säuren, Säureanhydriden und Estern, vorzugsweise aus der Gruppe bestehend aus Phosphorsäuren, Phosphonsäuren, Carbönsäuren und deren Salzen, Carbonsäureestem und Carbonsäureanhydriden,
bevorzugt in einer Menge im Bereich von 0,1 bis 5 Gew.-%, weiter bevorzugt in einer Menge im Bereich von 0,5 bis 2,5 Gew.-%, jeweils bezogen auf die Gesamtmasse der Zusammensetzung.

Vorzugsweise sind die ein oder mehreren haftfördemde Additive der Komponente (f) ausgewählt aus der Gruppe bestehend aus 10-(Meth)acryloyloxydecyldihydrogenphosphat (10-MDP), 2-(Meth)acryloyloxyethyldihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogenphosphat, 2-(Meth)acryloyloxynonyldihydrogenphosphat, 11-(Meth)acryloyloxyundecyldihydrogenphosphat, 20-(Meth)acryloyloxyeicosyldihydrogenphosphat, 1,3-Di(meth)acyloyloxypropyl-2-dihydrogenphosphat, 2-(Meth)acryloyloxyethylphenyldihydrogenphosphat, Di(2-(meth)acyloyloxyethyl)pyrophosphat, Di(2-(meth)acyloyloxypropyl)pyrophosphat, Di(2-(meth)acyloyloxybutyl)pyrophosphat, Di(2-(meth)acyloyloxypentyl)pyrophosphat, das Di(2-(meth)acyloyloxyhexyl)pyrophosphat, Di(2-(meth)acyloyloxydecyl)pyrophosphat, Bis[5-(2-(meth)acryloyloxyethoxycarbonyl)-heptyl]hydrogenphosphat, Mono-, Di- und/oder Triester der Phosphorsäure, welche durch Umsetzung von Hydroxy-C2-C8-alkylmethacrylat (dabei vorzugsweise Hydroxyethylmethacrylat und/oder Hydroxypropylmethacrylat) oder Glyceryldimethacrylat mit Phosphoroxychlorid erhalten werden, Glyceryldimethacrylatphosphat, Pentaerythritottrimethacrylatphosphat, Dipentaerythritolpentaacrylatphosphat, Tetramethacryloxyethylpyrophosphat, 4-(Methacryloyloxyethyl)-trimellitsäure, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat, Methacryloyloxyethylphthalat, Methacryloyloxyethylmaleat, Methacryloyloxyethylsuccinat, 1,3-Glyceroldimethacrylatmaleat und Di-Oxyethoxymethacrylsäureethylendiamintetraessigsäureester.

Die genannten Monomere können einzeln oder in Gemischen eingesetzt werden.

Weitere geeignete säuregruppentragende Monomere sind beispielsweise in der EP 0 980 682 A1 (insbesondere Absätze [0059] bis [0065]) oder der EP 0 948 955 A1 (insbesondere Absätze [0031] bis [0034]) genannt.

Bevorzugte haftfördernde Additive der Komponente (f) sind 10-(Meth)acryloyloxydecyldihydrogenphosphat (10-MDP), Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat.

Ebenfalls bevorzugt sind weitere Phosphorsäureester, insbesondere die Phosphorsäureestem von Hydroxyethyl(meth)acrylat (dabei vorzugsweise Mono- oder Diester), Hydroxypropyl(meth)acrylat (dabei vorzugsweise Mono- oder Diester) und Bisphenol-A-Glycidyl-(meth)acrylat.

Ein im Rahmen der vorliegenden Erfindung besonders wirksames, die Haftung förderndes Additiv, ist das Kondensationsprodukt aus Bis(hydroxymethyl)tricyclo[5.2.1.0.$^{2.6}$]decan mit Maleinsäure, bei dem die beiden Tricyclen über Esterbindungen verknüpft sind. Von den beiden nun endständigen Hydroxylgruppen kann dann eine mit Adipinsäure verestert werden. Diese Verbindung verleiht einer erfindungsgemäßen Zusammensetzung eine hohe Adhäsionskraft an Dentin. Diese Verbindung ist unter dem Namen "Adhesion Resin BL 215 C" bekannt. Eine bevorzugte erfindungsgemäße Zusammensetzung enthält dieses die Haftung fördernde Additiv.

Anstelle der Maleinsäure können auch andere $\alpha$, $\beta$ - ungesättigte Dicarbonsäuren wie Citracon-, Fumar-, Itacon- oder Mesaconsäure verwendet werden.

Bevorzugte erfindungsgemäße Zusammensetzungen umfassen ein oder mehrere haftfördernde Additive des Bestandteils (f), ausgewählt aus der Gruppe bestehend aus

- 10-(Meth)acryloyloxydecyldihydrogenphosphat (10-MDP), Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat,

- weiteren Phosphorsäureestern, dabei bevorzugt den Phosphorsäureestern von Hydroxyethyl(meth)acrylat (dabei vorzugsweise Mono- oder Diester), Hydroxypropyl(meth)acrylat (dabei vorzugsweise Mono- oder Diester) und Bisphenol-A-Glycidyl-(meth)acrylat,

- polymerisierbaren Carbonsäuren, die auf einem ungesättigten Polyesterharz basieren, herstellbar durch folgende Schritte

  (i) Veresterung von Dihydroxymethyltricyclo[5.2.1.0$^{2.8}$]decan mit einer ungesättigten $\alpha$, $\beta$ - ungesättigten Dicar-

bonsäure im molaren Verhältnis 2 : 1, und

(ii) Umsetzung der Hydroxylgruppen des in Schritt (i) gebildeten Polyesters mit einer gesättigten Dicarbonsäure, dabei vorzugsweise Adipinsäure.

Optionale weitere Additive

Eine erfindungsgemäße Zusammensetzung umfasst in manchen Fällen ein oder mehrere weitere Additive.
Diese Additive können verschiedene Funktionen haben. Übliche Additive für den Einsatz in dentalen Zusammensetzungen sind dem Fachmann bekannt, je nach gewünschter Funktion wird er das oder die geeigneten Additive auswählen. Beispielhaft sind im folgenden typische Additive und ihre Funktionen beschrieben.
Lichthärtbare dentale Zusammensetzungen, wie sie erfindungsgemäß bevorzugt sind, enthalten vorzugsweise einen oder mehrere Inhibitoren, auch Stabilisatoren genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie und tert.-Butylhydroxyanisol (BHA), 2,2 Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben
Alternative Inhibitoren sind in der DE 101 19 831 A1 oder in der EP 1 563 821 A1 angegeben
Eine erfindungsgemäß bevorzugte dentale Zusammensetzung umfasst somit als Additiv ein oder mehrere Polymerisationsinhibitoren zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.
Eine erfindungsgemäß bevorzugte dentale Zusammensetzung umfasst als Additiv eine oder mehrere fluoridabgebende Substanzen, dabei vorzugsweise Natriumfluorid und/oder Aminfluoride.
Ferner können ein oder mehrere Tenside Bestandteil einer erfindungsgemäßen Zusammensetzung sein.
UV-Absorber, die beispielsweise durch ihre konjugierten Doppelbindungssytemen und aromatischen Ringe befähigt sind, UV Strahlung zu absorbieren, sind in manchen Fällen Bestandteil einer erfindungsgemäßen Zusammensetzung. Beispiele an UV-Absorbern sind 2-Hydroxy-4-methoxybenzophenon, Salicylsäurephenylester 3-(2'-Hydroxy-5'-methylphenyl)-benzotriazol oder Diethyl-2,5-dihydroxy-terephthalat.
Weitere optionale Additive sind Aromastoffe.
Die Erfindung betrifft zudem ein Verfahren zur Herstellung einer erfindungsgemäßen Zusammensetzung, vorzugsweise in einer der als bevorzugt oder besonders bevorzugt gekennzeichneten Ausgestaltungen, mit folgenden Schritten:

- Mischen der Bestandteile (a), (b) und (c) sowie gegebenenfalls (d), (e) und/oder (f) sowie gegebenenfalls weiterer Additive,
  oder

- Herstellen eines mehrkomponentigen, vorzugsweise zweikomponentigen, Systems umfassend sämtliche der Bestandteile (a), (b), (c), gegebenenfalls (d), (e) und (f) sowie gegebenenfalls weitere Additive,

wobei die Initiatoren und/oder Katalysatoren des Bestandteils (b) so auf separate Komponenten der dentalen Zusammensetzung verteilt sind, dass eine chemische Initiierung durch Vermischen der besagten Komponenten ausgelöst wird.
Unter einem mehrkomponentigen, beispielsweise einem zweikomponentigen, System versteht der Fachmann eine Zusammensetzung aus mehreren, beispielsweise zwei, räumlich getrennten Komponenten. Eine der (beispielsweise zwei) Komponenten enthält dabei ein oder mehrere Monomere, während eine (bzw. die) andere Komponente einen oder mehrere Härter enthält. In den meisten Fällen müssen die mehreren (bzw. beiden) Komponenten vor der Applikation intensiv miteinander vermischt werden.
Offenbart wird auch ein Erzeugnis, erhältlich durch Härten einer erfindungsgemäßen Zusammensetzung, insbesondere in einer der als bevorzugt bzw. besonders bevorzugt gekennzeichneten Ausgestaltungen.
Offenbart wird ferner eine Zusammensetzung, insbesondere in einer der als bevorzugt bzw. besonders bevorzugt gekennzeichneten Ausgestaltungen, als dentales Füllungs- und/oder Versiegelungsmaterial bzw. zur Verwendung als dentales Füllungs- und/oder Versiegelungsmaterial, insbesondere als dentales Füllungs- und/oder Versiegelungsmaterial für einen Wurzelkanal.

Die erfindungsgemäßen Zusammensetzungen können als permanent oder temporär als Wurzelkanalversiegelungs- und/oder Füllmaterialien eingesetzt werden.

Offenbart wird entsprechend auch die Verwendung einer erfindungsgemäßen Zusammensetzung, insbesondere in einer der als bevorzugt bzw. besonders bevorzugt gekennzeichneten Ausgestaltungen, zum temporären oder permanenten Füllen und/oder Versiegeln eines Wurzelkanals.

Die vorliegende Erfindung betrifft eine erfindungsgemäße Zusammensetzung, insbesondere in einer der als bevorzugt bzw. besonders bevorzugt gekennzeichneten Ausgestaltungen, zur Anwendung in einem therapeutischen Verfahren als dentales Füllungs- und/oder Versiegelungsmaterial für einen Wurzelkanal, insbesondere zur Anwendung in einem therapeutischen Verfahren zum temporären oder permanenten Füllen und/oder Versiegeln eines Wurzelkanals.

Offenbart wird auch ein Verfahren zum Behandeln einer Zahnerkrankung, dadurch gekennzeichnet, dass eine erfindungsgemäße Zusammensetzung, vorzugsweise in einer der als bevorzugt angegebenen Ausgestaltungen, als dentales Füllungs- und/oder Versiegelungsmaterial für einen Wurzelkanal eingesetzt wird, insbesondere zur Anwendung in einem therapeutischen Verfahren zum temporären oder permanenten Füllen und/oder Versiegeln eines Wurzelkanals.

Die erfindungsgemäßen Zusammensetzungen können beispielsweise in Applikationskapseln angewendet werden. Die Komponenten werden dann in einem Kapselmischgerät gemischt und über eine Applikationsvorrichtung direkt in den Wurzelkanal eingebracht.

Die erfindungsgemäßen Zusammensetzungen können auch mit Hilfe von Doppelspritzen oder Doppelkammerkartuschen mit einer geeigneten Mischkanüle in den Wurzelkanal appliziert werden.

In einer bevorzugten Ausgestaltung steht die vorliegende Erfindung in einem weiteren Aspekt in Beziehung mit einer mehrkomponentigen Spritze, vorzugsweise einer 2-Komponentenapplikationsspritze, enthaltend eine erfindungsgemäße Zusammensetzung, vorzugsweise in einer der als bevorzugt gekennzeichneten Ausgestaltungen.

Ein typisches Beispiel für eine mehrkomponentige Spritze, die als Wurzelkanalspritze eingesetzt werden kann und die im Rahmen der vorliegenden Erfindung vorzugsweise eingesetzt wird, ist eine 2-Komponentenapplikationsspritze in Form einer Doppelkammerspritze mit einer Mischkanüle, wobei die Mischkanüle vorzugsweise 6 - 16 Mischwendeln aufweist. Bevorzugt weist eine im Rahmen der vorliegenden Erfindung einzusetzende Wurzelkanalspritze, dabei vorzugsweise eine 2-Komponentenapplikationsspritze, einen der Zahnwurzelgeometrie angepassten oder anpassbaren Applikationsaufsatz mit einem Außendurchmesser von 0,3 bis 0,9 mm und einer Länge von 15 bis 50 mm auf.

Die erfindungsgemäßen Zusammensetzungen werden vorzugsweise in Kits zur Versiegelung und Füllung eines Wurzelkanals vermarktet. Ein typisches Kit enthält dann beispielsweise eine mehrkomponentige Spritze, vorzugsweise eine 2-Komponentenapplikationsspritze, sowie gegebenenfalls Applikationsaufsätze, Papierspitzen, Wurzelkanalfüllstifte, die gegebenenfalls oberflächenbeschichtet sind, sowie einen oder mehrere Lentulos.

In einem weiteren Aspekt offenbart wird daher ein Kit zur Versiegelung und/oder Füllung von Wurzelkanälen, enthaltend

- eine erfindungsgemäße Zusammensetzung, vorzugsweise in einer der als bevorzugt gekennzeichneten Ausgestaltungen, in mehrkomponentiger Form, vorzugsweise in zweikomponentiger Form, bevorzugt in Form einer mehrkomponentige Spritze, vorzugsweise einer 2-Komponentenapplikationsspritze,
  und

- optional ein, zwei, drei oder mehr bzw. sämtliche der Bestandteile Applikationsaufsätze, Papierspitzen, Wurzelkanalfüllstifte, Lentulos.

<u>Beispiele</u>

Anhand der folgenden Beispiele wird die Erfindung weiter erläutert. Sofern nicht anders angegeben, beziehen sich alle Angaben auf das Gewicht.

Es werden dabei folgende Bezeichnungen bzw. Abkürzungen verwendet:

CC = Campherchinon

DABE = Ethyl-*p*-*N,N*-dimethylaminobenzoat

PEG-400-DMA = Polyethylenglykol-400-dimethacrylat

TCD-Monomer = Bis(methacryloyloxymethyl)tricyclo[5.2.1.0$^{2.6}$]decan

Nano-$SiO_2$ = silanisierte $SiO_2$-Partikel (40 nm)

Cumolhydroperoxid (88%) = eine Mischung aus 88% Cumolhydroperoxid und 12 % Cumol

GK = hochröntgenopakes, Zr-haltiges Glas ($d_{50}$ = 5 $\mu$m; BET-Oberfläche: 0,5 $m^2/g$)

Silanisierte Kieselsäure = organisch oberflächenmodifizierte agglomerierte Kieselsäure-Partikel (BET-Oberfläche: 160 $m^2/g \pm 25\ m^2/g$)

Haftmonomer = Adhesion Resin BL 215 C

Die beiden jeweiligen Pasten A und B flossen gut aus und waren gut miteinander mischbar.

Die Zusammensetzungen der beiden jeweiligen Pasten (in Gewichtsteilen) sind in den beiden nachfolgenden Tabellen aufgelistet.

Beispiele 1, 2 und 9 sind Vergleichsbeispiele. 3 bis 8 und 10 Die Beispiele (Bsp.) sind erfindungsgemäße Beispiele.

Tabelle 1: Paste A

| | Bsp. 1 | Bsp. 2 | Bsp. 3 | Bsp. 4 | Bsp. 5 | Bsp. 6 | Bsp. 7 | Bsp. 8 | Bsp. 9 | Bsp. 10 |
|---|---|---|---|---|---|---|---|---|---|---|
| Nano-$SiO_2$ | 0,00 | 0,00 | 0,00 | 0,00 | 6,15 | 6,16 | 0,00 | 6,16 | 0,00 | 0,00 |
| TCD-Monomer | 0,00 | 30,34 | 15,17 | 15,14 | 12,32 | 12,30 | 15,17 | 0,00 | 15,17 | 15,17 |
| Verbindung der Formel (2) | 0,00 | 0,00 | 0,00 | 0,00 | 0,00 | 0,00 | 0,00 | 12,30 | 0,00 | 0,00 |
| PEG-400-DMA | 30,34 | 0,00 | 15,17 | 15,14 | 12,32 | 12,30 | 14,17 | 12,30 | 0,00 | 0,00 |
| Triethylenglykol-DMA | 0,00 | 0,00 | 0,00 | 0,00 | 0,00 | 0,00 | 0,00 | 0,00 | 15,17 | 0,00 |
| Tetraethylenglykol-DMA | 0,00 | 0,00 | 0,00 | 0,00 | 0,00 | 0,00 | 0,00 | 0,00 | 0,00 | 15,17 |
| GK | 63,70 | 63,70 | 63,70 | 63,60 | 64,70 | 64,57 | 63,70 | 64,57 | 63,70 | 63,70 |
| Silanisierte Kieselsäure | 5,56 | 5,56 | 5,56 | 5,56 | 4,11 | 4,11 | 5,56 | 4,11 | 5,56 | 5,56 |
| Allylthiohamstoff | 0,28 | 0,28 | 0,28 | 0,28 | 0,28 | 0,28 | 0,28 | 0,28 | 0,28 | 0,28 |
| CC | 0,05 | 0,05 | 0,05 | 0,05 | 0,05 | 0,05 | 0,05 | 0,05 | 0,05 | 0,05 |
| DABE | 0,07 | 0,07 | 0,07 | 0,07 | 0,07 | 0,07 | 0,07 | 0,07 | 0,07 | 0,07 |
| gamma-Terpinen | 0,00 | 0,00 | 0,00 | 0,16 | 0,00 | 0,16 | 0,00 | 0,16 | 0,00 | 0,00 |
| Haftmonomer | 0,00 | 0,00 | 0,00 | 0,00 | 0,00 | 0,00 | 1,00 | 0,00 | 0,00 | 0,00 |

Tabelle 2: Paste B

| | Bsp. 1 | Bsp. 2 | Bsp. 3 | Bsp. 4 | Bsp. 5 | Bsp. 6 | Bsp. 7 | Bsp. 8 | Bsp. 9 | Bsp. 10 |
|---|---|---|---|---|---|---|---|---|---|---|
| Nano-$SiO_2$ | 0,00 | 0,00 | 0,00 | 0,00 | 6,15 | 6,16 | 0,00 | 6,16 | 0,00 | 0,00 |
| TCD-Monomer | 0,00 | 30,34 | 15,17 | 15,17 | 12,32 | 12,32 | 15,17 | 0,00 | 15,17 | 15,17 |
| Verbindung der Formel (2) | 0,00 | 0,00 | 0,00 | 0,00 | 0,00 | 0,00 | 0,00 | 12,32 | 0,00 | 0,00 |
| PEG-400-DMA | 30,34 | 0,00 | 15,17 | 15,17 | 12,32 | 12,32 | 15,17 | 12,32 | 0,00 | 0,00 |
| Triethylenglykol-DMA | 0,00 | 0,00 | 0,00 | 0,00 | 0,00 | 0,00 | 0,00 | 0,00 | 15,17 | 0,00 |
| Tetraethylenglykol-DMA | 0,00 | 0,00 | 0,00 | 0,00 | 0,00 | 0,00 | 0,00 | 0,00 | 0,00 | 15,17 |
| GK | 63,70 | 63,70 | 63,70 | 63,10 | 64,70 | 64,69 | 63,70 | 64,69 | 63,70 | 63,70 |
| Silanisierte Kieselsäure | 5,56 | 5,56 | 5,56 | 5,56 | 4,11 | 4,11 | 5,56 | 4,11 | 5,56 | 5,56 |
| Cumolhydroperoxid (88%) | 0,40 | 0,40 | 0,40 | 0,40 | 0,40 | 0,40 | 0,40 | 0,40 | 0,40 | 0,40 |

Die Ergebnisse der Messungen für die jeweiligen Wurzelkanalversiegelungsmaterialien sind in der nachfolgenden Tabelle aufgelistet.

Als Wurzelkanalversiegelungsmaterialien (Wurzelkanalsealer) sind im Folgenden die unmittelbar nach dem Vermischen der jeweiligen Paste A und Paste B vorliegenden Mischungen zu verstehen. Die beiden Pasten wurden zu gleichen Volumenanteilen miteinander vermischt (1:1 (v/v)).

Tabelle 3: Messergebnisse

| | Bsp. 1 | Bsp. 2 | Bsp. 3 | Bsp. 4 | Bsp. 5 | Bsp. 6 | Bsp. 7 | Bsp. 8 | Bsp.9 | Bsp.10 |
|---|---|---|---|---|---|---|---|---|---|---|
| Biegefestigkeit **[MPa]** | 3,6 | 12,7 | 5,7 | 1,1 | 26,4 | 6,5 | 5,6 | 7,1 | | |
| E-Modul [MPa] | 93 | 255 | 117 | 15 | 600 | 102 | 123 | 105 | | |
| Wasseraufnahme [µg/mm$^3$] | 227 | 4 | 23 | 15 | 25 | 16 | 25 | 14 | | |
| Wasseraufnahme | 9,85% | 0,18% | 1,01% | 0,66% | 1,01% | 0,65% | 1,10% | 0,57% | | |
| Löslichkeit [µg/mm$^3$] | 8 | 2 | 5 | 4 | 2 | 2 | 5 | 2 | | |
| Löslichkeit | 0,35% | 0,09% | 0,24% | 0,19% | 0,07% | 0,07% | 0,24% | 0,07% | | |
| Flow (ISO 6876) | 17 mm | 32 mm | 27 mm | 30 mm | 30 mm | 30 mm | 28 mm | 29 mm | | |
| Rönt-genopazität (Al) | 6,20 mm | 6,30 mm | 6,25 mm | 6,20 mm | 6,35 mm | 6,35 mm | 6,25 mm | 6,35 mm | | |
| Abbindezeit | 21 min | 19 min | 20 min | 22 min | 21 min | 23 min | 19 min | 22 min | | |
| Verarbeitungszeit | 42 min | 39 min | 40 min | 42 min | 40 min | 44 min | 39 min | 43 min | | |
| Dentinhaftung | | | 1,4 MPa | | | | 3,3 MPa | | 4,0 MPa | 3,6 MPa |

Ergebnisse:

Das nicht erfindungsgemäße Beispiel 1 weist sehr hohe Werte für die Wasseraufnahme und die Löslichkeit auf und ist daher für die Verwendung zum Versiegeln und Füllen eines Wurzelkanals nicht geeignet.

Die Ergebnisse zeigen für Beispiel 2 dagegen überraschenderweise eine ungewöhnlich geringe Wasseraufnahme sowie eine ungewöhnlich geringe Löslichkeit. Darüberhinaus ist die Fließfähigkeit des Materials ganz ausgezeichnet und die mechanischen Werte akzeptabel.

Hinsichtlich der erfindungsgemäßen Zusammensetzung gemäß Beispiel 3 kann festgestellt werden, dass durch die Kombination der Monomere der Komponenten (a1) und (a2) insbesondere der Wert für die Wasseraufnahme drastisch verbessert wird gegenüber Vergleichsbeispiel 1.

In der erfindungsgemäßen Zusammensetzung gemäß Beispiel 4 wurde durch Zugabe eines Molekulargewichtsreglers (γ-Terpinen) der Polymerisationsverlauf beeinflusst und Kettenlänge, Vemetzungsgrad des resultierenden Polymerisats sowie Kinetik des Polymerisationsverlaufs in einer Weise geändert, dass einzelne der gewünschten Eigenschaften weiter verbessert werden. Die Ergebnisse zeigen, dass die mechanischen Werte deutlich reduziert werden. Die Wasseraufnahme und Fließfähigkeit werden überraschenderweise verbessert.

In Beispiel 5 enthielt die erfindungsgemäße Zusammensetzung zusätzlich nicht agglomerierte, nanoskalige Partikel als weiterer Füllstoff (Komponente (c1)). Augenscheinlich bedingt durch eine bessere Raumerfüllung der Füllstoffteilchen werden die mechanischen Werte angehoben bei gleichzeitig stark reduzierter Löslichkeit.

Die erfindungsgemäße Zusammensetzung gemäß Beispiel 6 umfasst sowohl einen Molekulargewichtsregler (Komponente (d), hier gamma-Terpinen) und einen nanoskaligen Füllstoff (Komponente (c1)). Die mechanischen Werte konnten so noch weiter optimiert werden. Der Wert der Löslichkeit konnte extrem niedrig eingestellt werden, die Wasseraufnahme war ebenfalls äußerst gering bei einem guten Fließverhalten.

Ferner zeigt die erfindungsgemäße Zusammensetzung gemäß Beispiel 8, dass durch Einsatz der erfindungsgemäßen Monomere als Komponente (a1) die Eigenschaften noch weiter verbessert werden können.

Eine erfindungsgemäße Zusammensetzung, wie insbesondere die erfindungsgemäßen Beispiele 3, 6 und 8 zeigen, weist im ausgehärteten Zustand eine hinreichende Festigkeit auf und lässt sich dennoch ohne große Mühe mit einem zahnärztlichen Instrument, wie beispielsweise einer Feile, aus dem Wurzelkanal entfernen. Die Röntgensichtbarkeit mit einem Wert von 6,25 - 6,35 mm Aluminium ist ausgezeichnet für eine Diagnose im Wurzelkanal.

Nach Wasserlagerung der erfindungsgemäßen Proben wurden bei den mikroskopischen Untersuchungen keine Randspalten zwischen der Füllung und der Wurzelkanalwand beobachtet.

Vergleicht man die für einen Wurzelkanalversiegelungs- und/oder -füllmaterial auf Methacrylatharzbasis publizierten Werte der Wasseraufnahme und Löslichkeit ("Water sorption and solubility of Methacrylate resin based root canal sealers", Journal of Endodontics, 33, 8, 990-994, 2007) mit denen der erfindungsgemäßen Zusammensetzungen, so kann folgendes festgestellt werden:

Epiphany® (Pentron) hat eine Wasseraufnahme von 8% und EndoREZ® (Ultradent) eine Wasseraufnahme von 3%.

Epiphany® (Pentron) und EndoREZ® (Ultradent) haben jeweils eine Löslichkeit von 3,5%.

Die erfindungsgemäßen Zusammensetzungen hingegen zeigen sehr viel geringere, d.h. sehr viel bessere Werte, für die Wasseraufnahme und die Löslichkeit.

Dabei sei betont, dass dabei die Bestimmung der Werte für die Wasseraufnahme der erfindungsgemäßen Zusammensetzungen unter noch anspruchsvolleren Bedingungen erfolgte als die der publizierten Daten aus dem Stand der Technik (7 Tage vs. 24h Wasserlagerung).

Die Dentinhaftung wurde insbesondere für die Beispiele 3, 9 und 10 bestimmt Die Zusammensetzungen gemäß diesen Beispielen unterscheiden sich lediglich durch den Typ des jeweils eingesetzten Polyethylenglykol-Dimethacrylats. In den Untersuchungen hat sich bestätigt, dass bei Einsatz von Polyethylenglykoldimethacrylaten mit 4 Ethylenoxideinheiten (Tetraethylenglykol-DMA) bzw. ca. 9 Ethylenoxideinheiten (PEG-400-DMA) Dentinhaftungswerte erreicht werden, die für den Einsatz der jeweiligen Zusammensetzung als Wurzelkanalfüllmaterial bevorzugt sind.

Messmethoden:

Zur Bestimmung der mechanischen Werte (Biegefestigkeit und E-Modul) wurde das zu untersuchende Material ausschließlich chemisch gehärtet.

Biegefestigkeit

Die Biegefestigkeit wurde gemäß ISO 4049 bestimmt. Dazu wurden die jeweiligen Wurzelkanalversiegelungsmaterialien (Wurzelkanalsealer) luftblasenfrei in entsprechende Teflonformen eingefüllt, mit Folie und Glasplatte bedeckt

und mit einer Schraubzwinge wurden die Überschüsse herausgepresst. Die Probekörper wurden 24 Stunden bei 37°C im Wasserbad ausgehärtet.

Die Maße des Probekörpers (2 mm x 2 mm) wurden in der Mitte mit einer Messgenauigkeit von 0,01 mm gemessen. Anschließend wurden die Probekörper in einer Universalprüfmaschine Zwick Z005 (Zwick GmbH, Ulm, Deutschland) mit einer Vorschubgeschwindigkeit von 0,75 mm/min bis zum Bruch belastet.

Die Biegefestigkeit, $\sigma$, wird in Megapascal nach folgender Gleichung berechnet:

$$\sigma = \frac{3 \cdot F \cdot l}{2 \cdot b \cdot h^2}$$

Dabei ist

F die maximale auf den Probekörper ausgeübte Kraft, in Newton;
/ der Abstand zwischen den Auflagen (20 mm)
b die Breite in der Mitte des Probekörpers unmittelbar vor der Prüfung gemessen, in Millimeter;
h die Höhe in der Mitte des Probekörpers unmittelbar vor der Prüfung gemessen, in Millimeter.

E-Modul

Der E-Modul wurde aus den Messungen der Biegefestigkeiten durch Berechnung der Steigung im linearen Bereich ermittelt.

Wasseraufnahme und Löslichkeit

Wasseraufnahme und Löslichkeit wurden analog zu ISO 4049 bestimmt. Dazu wurden die Wurzelkanalsealer luftblasenfrei in entsprechende Teflonformen eingefüllt, mit Folien und Glasplatten bedeckt und mit einer Schraubzwinge wurden die Überschüsse herausgepresst. Die Probekörper mit einem Durchmesser von 15,0 $\pm$ 0,1 mm und einer Höhe von 1,0 $\pm$ 0,1 mm wurden segmentweise lichtgehärtet. Anschließend wurden die Probekörper in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_1$, erreicht war.

Nach dem vollständigen Trocknen wurde der Durchmesser zweimal rechtwinklig zueinander mit einer Messgenauigkeit von 0,01 mm gemessen und daraus der mittlere Durchmesser berechnet. Die Dicke des Probekörpers wurde in der Mitte und an vier in gleichem Abstand liegenden Stellen des Randes auf 0,01 mm gemessen. Aus dem mittleren Durchmesser und der mittleren Dicke wurde das Volumen, V, berechnet.

Anschließend wurden die Probekörper für 7 Tage in Wasser bei 37°C gelagert Danach wurden die Probekörper herausgenommen, mit Wasser abgespült und abgetupft, bis auf der Oberfläche keine Feuchtigkeit mehr sichtbar war. Die Probekörper wurden 15 s in der Luft hin- und hergeschwenkt und 1 min nach dem Herausnehmen aus dem Wasser gewogen. Diese Masse wird als $m_2$ angegeben.

Anschließend wurden die Probekörper erneut in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_3$, erreicht war.

Die Wasseraufnahme, $W_{sp}$, wurde nach folgender Gleichung berechnet:

$$W_{sp} = \frac{m_2 - m_3}{V}$$

Dabei ist

$m_2$ die Masse des Probekörpers nach Wasserlagerung für 7 Tage in $\mu$g;
$m_3$ die Masse des wieder getrockneten Probekörpers in $\mu$g;
V das Volumen des Probekörpers in $mm^3$

Die Löslichkeit, $W_{sl}$, wurde nach folgender Gleichung berechnet:

$$W_{sl} = \frac{m_1 - m_3}{V}$$

Dabei ist

$m_1$ die Masse des getrockneten Probekörpers vor der Wasserlagerung in $\mu$g
$m_3$ die Masse des wieder getrockneten Probekörpers in $\mu$g;
V das Volumen des Probekörpers in $mm^3$

Fließfähigkeit (Flow)

Die Fließfähigkeit (Flow) wurde gemäß ISO 6876 bestimmt. Auf eine Glasplatte (40 mm x 40 mm) wurden 0,05 ml des angemischten Wurzelkanalsealers appliziert. 3 min nach Mischbeginn wurden eine zweite Glasplatte (40 mm x 40 mm; 20 g) und eine Gewicht (100 g) aufgelegt. 10 min nach Mischbeginn wurde das Gewicht entfernt und der kleinste sowie der größte Durchmesser gemessen und daraus der mittlere Durchmesser berechnet.
Die Fließfähigkeit F ergibt sich als Mittelwert der mittleren Durchmesser von jeweils drei Einzelbestimmungen.

Röntgenopazität

Zur Bestimmung der Röntgenopazität wurden Probekörper mit einem Durchmesser von 15 mm und einer Höhe von 2 mm der Wurzelkanalsealer hergestellt. Von den Probekörpem wurden jeweils zusammen mit einer Aluminiumtreppe Röntgenaufnahmen angefertigt (7 mA; 60 kV; 0,04 s).
Zur Bestimmung wurden die Höhen der Aluminiumtreppenstufen und die Dicke der Probekörper auf 0,01 mm genau bestimmt sowie die Grauwerte für die Stufen der Aluminiumtreppe und für die Probekörper bestimmt. Aus diesen Werten wurde durch lineare Regression die Röntgenopazität in Aluminiumäquivalenten berechnet.

Verarbeitungszeit

Die Verarbeitungszeit wurde am Rheometer Physica MCR 301 (Anton Paar GmbH, Graz, Österreich) bestimmt. Dazu wurde bei 23°C in einer Oszillationsmessung ($\gamma$ = 1%, f = 4 Hz) die Viskosität in Abhängigkeit von der Zeit aufgenommen. Der Wurzelkanalsealer wurde angemischt und auf die Messplatte eines Platte/Platte-Systems aufgebracht (d = 12 mm, Spalt = 1 mm). Die Zeit wurde mit Mischbeginn gestartet. Als Verarbeitungszeit wurde der Schnittpunkt der Geraden des noch nicht ausgehärtetem Materials mit der Tangente am steilsten Punkt der Aushärtereaktion ausgewertet.

Abbindezeit

Die Abbindezeit wurde am Rheometer Physica MCR 301 (Anton Paar GmbH, Graz, Österreich) bestimmt. Dazu wurde bei 37°C in einer Oszillationsmessung ($\gamma$ = 1%, f = 4 Hz) die Viskosität in Abhängigkeit von der Zeit aufgenommen. Der Wurzelkanalsealer wurde angemischt und auf die Messplatte eines Platte/Platte-Systems aufgebracht (D = 12 mm, Spalt = 1 mm). Die Zeit wurde mit Mischbeginn gestartet. Als Abbindezeit wurde die Zeit vom Mischbeginn bis zum erreichen einer Viskosität von $10^4$ Pa•s ausgewertet.

Dentinhaftung

Die Dentinhaftung wurde im Scherversuch an Rinderdentin bestimmt. Dazu wurde die Dentinoberfläche zunächst mit 180er SiC-Schleifpapier und anschließend mit 1000er SiC-Schleifpapier glatt geschliffen. Die Oberfläche wurde mit Papier abgetupft bis gerade eben keine Feuchtigkeit mehr sichtbar war. Anschließend wurde der Wurzelkanalsealer in einem Silikonring (d = 5 mm) auf die Oberfläche appliziert und für 24 Stunden bei 37°C im Wasserbad ausgehärtet. Vor der Prüfung wurden die Silikonringe vorsichtig entfernt. In einer Universalprüfmaschine Zwick Z005 (Zwick GmbH, Ulm, Deutschland) wurden die Probekörper mit einer Vorschubgeschwindigkeit von 1 mm/min bis zum Bruch belastet. Anschließend wurde der Durchmesser der Haftfläche des Probekörpers zweimal rechtwinklig zueinander mit einer Messgenauigkeit von 0,01 mm gemessen und daraus der mittlere Durchmesser berechnet.
Die Haftung, $\tau_{Dentin}$, wird in Megapascal nach folgender Gleichung berechnet:

$$\tau_{Dentin} = \frac{F}{\pi \cdot \left(\frac{d_1+d_2}{4}\right)^2}$$

Dabei ist

F die maximale auf den Probekörper ausgeübte Kraft, in Newton;
$d_1$ und $d_2$ die rechtwinklig zueinander gemessenen Durchmesser des Probekörpers, in Millimeter

Synthese der Verbindung der Formel (2)

0,95 g (4,84 mmol) 3(4), 8(9)-Bis(hydroxymethyl)tricyclo[5.2.1.0$^{2,6}$]decan wurden in 10 mL Toluol gelöst und mit 0,04 g BHT und 0,103 g der Katalysatorlösung (0,50 g Dibutylzinn(II)dilaurat gelöst in 9,50 g Toluol) versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 72 Stunden wurden weitere 0,102 g Katalysatorlösung zugesetzt und weiter erhitzt, bis keine Isocyanatbande mehr detektiert wurde. Das Lösungsmittel wurde am Rotationsverdampfer entfernt. Das Allophanat der Formel (2) wurde in einer Ausbeute von 3,83 g (4,69 mmol, 97%) als leicht gelbliches Öl erhalten.

Die Beispiele 2 bis 7 wurden unter Austausch des dort jeweils eingesetzten TCD-Monomeren gegen die Verbindung der Formel (2) wiederholt; diese weiteren Beispiele werden als Beispiele S2 bis S7 bezeichnet. Es wurden sämtliche in den Beispielen 2 bis 7 bestimmten Parameter auch für die Beispiele S2 bis S7 bestimmt. Die bestimmten Parameterwerte für die Beispiele S2 bis S7 sind ähnlich zu denen aus den Beispielen 2 bis 7 und übertreffen diese teilweise. Dies zeigt neben Beispiel 8, dass die Verbindung der Formel (2), welche repräsentativ für die erfindungsgemäß einzusetzenden Verbindungen steht, hervorragend für den Einsatz in erfindungsgemäßen Kompositmaterialien geeignet ist.

## Patentansprüche

**1.** Härtbare dentale Zusammensetzung zur Anwendung in einem therapeutischen Verfahren als dentales Füllungs- und/oder Versiegelungsmaterial für einen Wurzelkanal, bestehend aus oder umfassend:

(a) eine Monomerenkomponente umfassend oder bestehend aus

(a1) ein, zwei oder mehr polymerisierbare Monomere mit einer Molmasse von weniger als 4000 g/mol ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,
- b ist eine natürliche Zahl ausgewählt aus der Gruppe der natürlichen Zahlen 1, 2, 3 und 4,
- jedes Z bedeutet ein Strukturelement, das unabhängig von etwaigen weiteren Strukturelementen Z ausgewählt ist aus der Gruppe bestehend aus

$-O-(C{=}O)-CH{=}CH_2$, $-O-(C{=}O)-C(CH_3){=}CH_2$,

$-(C{=}O)-CH{=}CH_2$, $-(C{=}O)-C(CH_3){=}CH_2$,

$-CH{=}CH_2$, $-C(CH_3){=}CH_2$ und $-O-CH{=}CH_2$,

- jeder Index e ist eine natürliche Zahl, die unabhängig von etwaigen weiteren Indizes e ausgewählt ist aus der Gruppe der natürlichen Zahlen 1, 2, 3 und 4,
- jeder Index x bedeutet unabhängig von etwaigen weiteren Indizes x 0 oder 1,
- jedes Y bedeutet in der Struktur $Q(Y_xZ_e)_b$ bei x = 1 ein Strukturelement, welches das polyalicyclische

Strukturelement Q mit e Strukturelementen Z verbindet, wobei jedes Y unabhängig von etwaigen weiterpen Strukturelementen Y gewählt ist,

(b) ein oder mehrere Initiatoren und/oder Katalysatoren, und
(c) eine Füllstoffkomponente bestehend aus oder umfassend

(c1) einen, zwei oder mehrere röntgenopake Füllstoffe, sowie gegebenenfalls ein oder mehrere sonstige Additive

wobei die Monomerenkomponente (a)

(a2) ein, zwei oder mehr weitere polymerisierbare Monomere aus der Gruppe bestehend aus Acrylaten und Methacrylaten mit einem Polyether-Strukturelement enthält, wobei keines dieser weiteren polymerisierbaren Monomere eines der Struktur $Q(Y_x(Z_e)_b$ gemäß der Definition zu (a1) ist,

wobei das Verhältnis der Gesamtmasse der (a1) polymerisierbaren Monomere gemäß der Definition zu (a1) zur Gesamtmasse der (a2) ein, zwei oder mehr weiteren polymerisierbaren Monomeren aus der Gruppe bestehend aus Acrylaten und Methacrylaten mit einem Polyether-Strukturelement, wobei keines der weiteren polymerisierbaren Monomere eines der Struktur $Q(Y_xZ_e)_b$ gemäß der Definition zu (a1) ist im Bereich von 3 : 1 bis 1 : 3 liegt,
wobei die Monomerenkomponente (a2) ein oder mehrere Polyethylenglykoldi(meth)acrylate mit 4 bis 10 Ethylenoxideinheiten enthält oder aus diesen besteht.

**2.** Zusammensetzung nach Anspruch 1, wobei das Verhältnis der Gesamtmasse der (a1) polymerisierbaren Monomere gemäß der Definition in Anspruch 1 zu (a1) zur Gesamtmasse der (a2) ein, zwei oder mehr weiteren polymerisierbaren Monomeren aus der Gruppe bestehend aus Acrylaten und Methacrylaten mit einem Polyether-Strukturelement, wobei keines der weiteren polymerisierbaren Monomere eines der Struktur $Q(Y_xZ_e)_b$ gemäß der Definition in Anspruch 1 zu (a1) ist, im Bereich von 2 : 1 bis 1 : 2, bevorzugt im Bereich von 3 : 2 bis 2 : 3 liegt.

**3.** Zusammensetzung nach einem der vorangehenden Ansprüche, wobei die Monomerenkomponente (a),

- als Komponente (a1) eine Gesamtmenge im Bereich von 5 bis 48,5 Gew.-%, vorzugsweise von 8 bis 30 Gew.-%, bevorzugt von 10 bis 20 Gew.-%, polymerisierbare Monomere mit einer Molmasse von weniger als 4000 g/mol umfasst, ausgewählt aus der Gruppe bestehend aus Verbindungen (Monomere) der Struktur $Q(Y_xZ_e)_b$ gemäß der Definition in Anspruch 1 zu (a1),
und/oder
- als Komponente (a2) eine Gesamtmenge im Bereich von 5 bis 48,5 Gew.-%, vorzugsweise von 8 bis 30 Gew.-%, bevorzugt von 10 bis 20 Gew.-% an ein, zwei oder mehr weiteren polymerisierbaren Monomeren umfasst, ausgewählt aus der Gruppe bestehend aus Acrylaten und Methacrylaten mit einem Polyether-Strukturelement, vorzugsweise der Gruppe der Methacrylate mit einem Polyether-Strukturelement, wobei keines der weiteren polymerisierbaren Monomere eines der Struktur $Q(Y_xZ_e)_b$ gemäß der Definition in Anspruch 1 zu (a1) ist, jeweils bezogen auf die Gesamtmasse der Zusammensetzung.

**4.** Zusammensetzung nach einem der vorangehenden Ansprüche, umfassend als Komponente (c) eine Gesamtmerige an Füllstoffen im Bereich von 35 bis 85 Gew.-%, vorzugsweise im Bereich von 50 bis 80 Gew.-%, bevorzugt im Bereich von 60 bis 75 Gew.-%, jeweils bezogen auf die Gesamtmasse der Zusammensetzung.

**5.** Zusammensetzung nach einem der vorangehenden Ansprüche, **dadurch gekennzeichnet, dass** ein, zwei oder mehr Verbindungen der Struktur $Q(Y_xZ_e)_b$ einen Tricyclo[5.2.1.0$^{2,6}$]-decan-Rest oder einen Tricyclo[3.3.1.1$^{3,7}$]decan-Rest aufweisen und Z vorzugsweise ausgewählt ist aus der Gruppe bestehend aus $-O-(C=O)-CH=CH_2$ und $-O-(C=O)-C(CH_3)=CH_2$, bevorzugt bedeutet Z die Gruppe $-O-(C=O)-C(CH_3)=CH_2$.

**6.** Zusammensetzung nach einem der vorangehenden Ansprüche, **dadurch gekennzeichnet, dass** Komponente (a1) umfasst oder besteht aus Bis(methacryloyloxymethyl)tricyclo[5.2.1.0$^{2,6}$]decan und/oder Bis(acryloyloxymethyl)tricyclo [5.2.1.0$^{2,6}$]decan.

**7.** Zusammensetzung nach einem der vorangehenden Ansprüche, **dadurch gekennzeichnet, dass** diese als Additiv einen oder mehrere Molekulargewichtsregler als Komponente (d) enthält, vorzugsweise ausgewählt aus der Gruppe bestehend aus Dienen, die durch Abstraktion eines Allyl-H-Atoms ein delokalisiertes Elektronensystem ausbilden

können, das sich über 5 C-Atome erstreckt.

**8.** Zusammensetzung nach einem der vorhergehenden Ansprüche, wobei die Füllstoffkomponente (c) zusätzlich umfasst

(c2) einen oder mehrere nicht röntgenopake Füllstoffe.

**9.** Zusammensetzung nach einem der vorangehenden Ansprüche, **dadurch gekennzeichnet, dass** diese ein oder mehrere haftfördernde Additive als Komponente (f) enthalten, vorzugsweise ausgewählt aus der Gruppe bestehend aus polymerisierbaren oder nicht polymerisierbaren Säuren, Säureanhydriden und Estern, vorzugsweise aus der Gruppe bestehend aus Phosphorsäuren, Phosphonsäuren, Carbonsäuren und deren Salzen, Carbonsäureestern und Carbonsäureanhydriden.

**10.** Zusammensetzung nach einem der Ansprüche 1 bis 9 zur Anwendung in einem therapeutischen Verfahren als dentales Füllungs- und/oder Versiegelungsmaterial für einen Wurzelkanal, zur Anwendung in einem therapeutischen Verfahren zum temporären oder permanenten Füllen und/oder Versiegeln eines Wurzelkanals.

## Claims

**1.** Curable dental composition for use in a therapeutic procedure as a dental filling and/or sealing material for a root canal, consisting of or comprising:

(a) a monomer component comprising or consisting of

(a1) one, two or more polymerisable monomers with a molar mass of less than 4,000 g/mol selected from the group consisting of compounds (monomers) of the structure $Q(Y_xZ_e)_b$, wherein:

- Q denotes a saturated or olefinically unsaturated polyalicyclic structure element selected from the group consisting of bicyclic, tricyclic, tetracyclic, pentacyclic and hexacyclic hydrocarbon radicals, wherein optionally one, two or more of the hydrogen atoms of this polyalicyclic structure element Q which are not replaced by substituents $Y_xZ_e$ are replaced by alkyl groups (in this context preferably C1-C4-alkyl), alkoxy groups (in this context preferably C1-C4-alkoxy), halogen atoms (in this context preferably F) or trifluoromethyl groups,
- b is a positive integer selected from the group of the positive integers 1, 2, 3 and 4,
- each Z denotes a structure element which independently of any further structure elements Z is selected from the group consisting of

$-O-(C=O)-CH=CH_2$, $-O-(C=O)-C(CH_3)=CH_2$,

$-(C=O)-CH=CH_2$, $-(C=O)-C(CH_3)=CH_2$,

$-CH=CH_2$, $-C(CH_3)=CH_2$ and $-O-CH=CH_2$,

- each index e is a positive integer which independently of any further indices e is selected from the group consisting of the positive integers 1, 2, 3 and 4,
- each index x independently of any further indices x denotes 0 or 1,
- each Y in the structure $Q(Y_xZ_e)_b$ where x = 1 denotes a structure element which bonds the polyalicyclic structure element Q with e structure elements Z, wherein each Y is selected independently of any further structure elements Y,

(b) one or more initiators and/or catalysts, and
(c) a filler component consisting of or comprising

(c1) one, two or more radiopaque fillers,

and optionally one or more other additives
wherein the monomer component (a) comprises

(a2) one, two or more further polymerisable monomers from the group consisting of acrylates and methacrylates having a polyether structure element, wherein none of these further polymerisable monomers is one of the structure $Q(Y_xZ_e)_b$ according to the definition of (a1),

wherein the ratio of the total weight of the (a1) polymerisable monomers according to the definition of (a1) to the total weight of the (a2) one, two or more further polymerisable monomers from the group consisting of acrylates and methacrylates having a polyether structure element, wherein none of the further polymerisable monomers is one of the structure $Q(Y_xZ_e)_b$ according to the definition of (a1), is in the range of from 3 : 1 to 1 : 3,
wherein the monomer component (a2) comprises one or more polyethylene glycol di(meth)acrylates having 4 to 10 ethylene oxide units or consists of these.

**2.** Composition according to claim 1, wherein the ratio of the total weight of the (a1) polymerisable monomers according to the definition in claim 1 of (a1) to the total weight of the (a2) one, two or more further polymerisable monomers from the group consisting of acrylates and methacrylates having a polyether structure element, wherein none of the further polymerisable monomers is one of the structure $Q(Y_xZ_e)_b$ according to the definition in claim 1 of (a1), is in the range of from 2 : 1 to 1 : 2, preferably in the range of from 3 : 2 to 2 : 3.

**3.** Composition according to one of the preceding claims, wherein the monomer component (a)

- comprises as component (a1) a total amount in the range of from 5 to 48.5 wt.%, by preference from 8 to 30 wt.%, preferably from 10 to 20 wt.%, of polymerisable monomers with a molar mass of less than 4,000 g/mol, selected from the group consisting of compounds (monomers) of the structure $Q(Y_xZ_e)_b$ according to the definition in claim 1 of (a1),
and/or
- comprises as component (a2) a total amount in the range of from 5 to 48.5 wt.%, by preference from 8 to 30 wt.%, preferably from 10 to 20 wt.% of one, two or more further polymerisable monomers selected from the group consisting of acrylates and methacrylates having a polyether structure element, preferably from the group of methacrylates having a polyether structure element, wherein none of the further polymerisable monomers is one of the structure $Q(Y_xZ_e)_b$ according to the definition in claim 1 of (a1),

in each case based on the total weight of the composition.

**4.** Composition according to one of the preceding claims, comprising as component (c) a total amount of fillers in the range of from 35 to 85 wt.%, by preference in the range of from 50 to 80 wt.%, preferably in the range of from 60 to 75 wt.%, in each case based on the total weight of the composition.

**5.** Composition according to one of the preceding claims, **characterised in that** one, two or more compounds of the structure $Q(Y_xZ_e)_b$ contain a tricyclo[5.2.1.0$^{2,6}$]decane radical or a tricyclo[3.3.1.1$^{3,7}$]decane radical and Z is preferably selected from the group consisting of $-O-(C=O)-CH=CH_2$ and $-O-(C=O)-C(CH_3)=CH_2$, preferably Z denotes the group $-O-(C=O)-C(CH_3)=CH_2$.

**6.** Composition according to one of the preceding claims, **characterised in that** component (a1) comprises or consists of bis(methacryloyloxymethyl)tricyclo[5.2.1.0$^{2,6}$]decane and/or bis(acryloyloxymethyl)tricyclo[5.2.1.0$^{2,6}$]decane.

**7.** Composition according to one of the preceding claims, **characterised in that** this comprises as an additive one or more molecular weight regulators as component (d), preferably selected from the group consisting of dienes which can form a delocalised electron system which extends over 5 C atoms by abstraction of an allyl-H atom.

**8.** Composition according to one of the preceding claims, wherein the filler component (c) additionally comprises

(c2) one or more non-radiopaque fillers.

**9.** Composition according to one of the preceding claims, **characterised in that** this comprises one or more adhesion-promoting additives as component (f), preferably selected from the group consisting of polymerisable or non-polymerisable acids, acid anhydrides and esters, preferably from the group consisting of phosphoric acids, phosphonic acids, carboxylic acids and salts thereof, carboxylic acid esters and carboxylic acid anhydrides.

**10.** Composition according to one of claims 1 to 9 for use in a therapeutic procedure as a dental filling and/or sealing

material for a root canal, for use in a therapeutic procedure for temporary or permanent filling and/or sealing of a root canal.

## Revendications

**1.** Composition dentaire durcissable pour son utilisation dans un procédé thérapeutique comme matériau de comblement et/ou de scellement d'un canal radiculaire, consistant en ou comprenant :

(a) un composant monomère comprenant ou consistant en

(a1) un, deux ou plusieurs monomères polymérisables avec une masse molaire inférieure à 4000 g/mole choisi dans le groupe consistant en composés (monomères) de structure $Q(Y_xZ_e)_b$, dans laquelle :

- Q désigne un élément structural polyalicyclique saturé ou oléfiniquement insaturé choisi dans le groupe consistant en radicaux hydrocarbures bicycliques, tricycliques, tétracycliques, pentacycliques et hexacycliques, dans laquelle éventuellement un, deux ou plusieurs des atomes d'hydrogène non substitués par le substituant $Y_xZ_e$ de cet élément structural polyalicyclique Q sont substitués par des groupes alkyle (dont de préférence un groupe alkyle en C1 à C4), des groupes alcoxy (dont de préférence un groupe alcoxy en C1 à C4), des atomes d'halogène (dont de préférence F) ou des groupes trifluorométhyle,
- b est un nombre entier naturel choisi dans le groupe des nombres entiers naturels 1, 2, 3 et 4,
- chaque Z désigne un élément structural qui est choisi indépendamment de quelconques autres éléments structuraux Z dans le groupe consistant en

$-O-(C{=}O)-CH{=}CH_2$, $-O-(C{=}O)-C(CH_3){=}CH_2$,

$-(C{=}O)-CH{=}CH_2$, $-(C{=}O)\ -C(CH_3){=}CH_2$,

$-CH{=}CH_2$, $-C(CH3){=}CH_2$ et $-O-CH{=}CH_2$,

- chaque indice e est un nombre entier naturel qui est choisi indépendamment d'un quelconque autre indice e dans le groupe des nombres entiers naturels 1, 2, 3 et 4,
- chaque indice x désigne, indépendamment d'un quelconque autre indice x, 0 ou 1,
- chaque Y désigne, dans la structure $Q(Y_xZ_e)_b$ où x = 1, un élément structural qui relie l'élément structural polyalicyclique Q à e éléments structuraux Z, dans laquelle chaque Y est choisi indépendamment de quelconques autres éléments structuraux Y,

(b) un ou plusieurs initiateurs et/ou catalyseurs, et
(c) un composant de comblement consistant en ou comprenant

(c1) un, deux ou plusieurs agents de comblement radio-opaques et éventuellement un ou plusieurs autres additifs,

dans laquelle le composant monomère (a) contient

(a2) un, deux ou plusieurs autres monomères polymérisables du groupe consistant en acrylates et méthacrylates avec un élément structural polyéther, dans laquelle aucun de ces autres monomères polymérisables est l'un de la structure $Q(Y_xZ_e)_b$ selon la définition de (a1),

dans laquelle le rapport de la masse totale des (a1) monomères polymérisables selon la définition de (a1) à la masse totale des (a2) un, deux ou plusieurs autres monomères polymérisables du groupe consistant en acrylates et méthacrylates avec un élément structural de polyéther, dans laquelle aucun des autres monomères polymérisables n'est l'un de la structure $Q(Y_xZ_e)_b$ selon la définition de (a1), se situe dans la plage de 3:1 à 1:3,
dans laquelle le composant monomère (a2) contient ou consiste en un ou plusieurs polyéthylène glycol-di(méth)acrylate avec 4 à 10 motifs d'oxyde d'éthylène.

**2.** Composition selon la revendication 1, dans lequel le rapport de la masse totale des monomères polymérisables

(a1) selon la définition de la revendication 1 pour (a1) à la masse totale des un, deux ou plusieurs autres monomères polymérisables (a2) du groupe consistant en acrylates et méthacrylates avec un élément structural de polyéther, dans laquelle aucun des autres monomères polymérisables n'est l'un de la structure $Q(Y_xZ_e)_b$ selon la définition de la revendication 1 pour (a1) se situe dans la plage de 2:1 à 1:2, de manière préférée dans la plage de 3:2 à 2:3.

**3.** Composition selon l'une des revendications précédentes, dans laquelle le composant monomère (a),

- comprend, comme composant (a1), une quantité totale dans la plage de 5 à 48,5 % en poids, de préférence de 8 à 30 % en poids, de manière préférée de 10 à 20 % en poids de monomères polymérisables avec une masse molaire inférieure à 4000 g/mole, choisis dans le groupe consistant en composés (monomères) de la structure $Q(Y_xZ_e)_b$ selon la définition de la revendication 1 pour (a1),
et/ou
- comprend, comme composant (a2), une quantité totale dans la plage de 5 à 48,5 % en poids, de préférence de 8 à 30 % en poids, de manière préférée de 10 à 20 % en poids d'un, deux ou plusieurs autres monomères polymérisables choisis dans le groupe consistant en acrylates et méthacrylates avec un élément structural polyéther, de préférence du groupe des méthacrylates avec un élément structural polyéther, dans laquelle aucun des autres monomères polymérisables n'est l'un de la structure $Q(Y_xZ_e)_b$ selon la définition de la revendication 1 pour (a1), respectivement par rapport à la masse totale de la composition.

**4.** Composition selon l'une des revendications précédentes, comprenant, comme composant (c), une quantité totale d'agents de comblement dans la plage de 35 à 85 % en poids, de préférence dans la plage de 50 à 80 % en poids, de manière préférée dans la plage de 60 à 75 % en poids, respectivement par rapport à la masse totale de la composition.

**5.** Composition selon l'une des revendications précédentes, **caractérisée en ce qu'**un, deux ou plusieurs composés de la structure $Q(Y_xZ_e)_b$ présentent un radical tricyclo [5.2.1.0$^{2,6}$] décane ou un radical tricyclo[3.3.1.1$^{3,7}$]décane et Z est choisi de préférence dans le groupe consistant en -O-(C=O) -CH=$CH_2$ et -O-(C=O)-C($CH_3$)=$CH_2$, de manière préférée Z désigne le groupe -O-(C=O) -C($CH_3$)=$CH_2$.

**6.** Composition selon l'une des revendications précédentes, **caractérisée en ce que** le composant (a1) comprend ou consiste en bis(méthacryloyloxyméthyl) tricyclo[5.2.1.0$^{2,6}$]décane et/ou en bis(acryloyloxyméthyl) tricyclo[5.2.1.0$^{2,6}$]décane.

**7.** Composition selon l'une des revendications précédentes, **caractérisée en ce que** celle-ci contient, comme additif, un ou plusieurs régulateurs du poids moléculaire comme composants (d), de préférence choisis dans le groupe consistant en diènes, qui peuvent former, par abstraction d'un atome d'allyle-H, un système d'électrons délocalisé qui s'étend sur 5 atomes de C.

**8.** Composition selon l'une des revendications précédentes, dans laquelle le composant de comblement (c) comprend en outre

(c2) un ou plusieurs agents de comblement non radio-opaques.

**9.** Composition selon l'une des revendications précédentes, **caractérisée en ce que** celle-ci contient un ou plusieurs additifs favorisant l'adhérence comme composants (f), choisis de préférence dans le groupe consistant en acides, anhydrides acides et esters polymérisables ou non polymérisables, de préférence dans le groupe consistant en acides phorphoriques, acides phosphoniques, acides carboxyliques et leurs sels, esters d'acides carboxyliques et anhydrides d'acides carboxyliques.

**10.** Composition selon l'une des revendications 1 à 9, pour son utilisation dans un procédé thérapeutique comme matériau de comblement et/ou de scellement dentaire pour un canal radiculaire, pour son utilisation dans un procédé thérapeuthique pour le comblement provisoire et/ou permanent et le scellement d'un canal radiculaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 2420351 C3
- DE 102007029640 A1
- US 20090131552 A
- DE 102005041115 A1
- DE 69518030 T2
- US 6734223 B2
- US 20080287566 A1
- EP 1547571 B1
- DE 60125374 T2
- US 7320598 B2
- US 6811400 B2
- US 6500004 B2
- US 6652282 B2
- US 20080200586 A
- US 5646197 A
- US 4449938 A
- EP 0864312 A
- DE 1139940 A
- US 20080206716 A1
- WO 2008100451 A2
- WO 2008100452 A2
- US 20080299093 A1
- DE 2931926
- DE 2816823
- DE 2406557
- DE 3522006 A1
- DE 3522005 A1
- DE 3703080 A1
- DE 3703130 A1
- DE 3703120 A1
- DE 102005053775 A1
- DE 102007034457 A1
- DE 102006060983 A1
- WO 03035013 A1
- DE 60216951 T2
- EP 1238993 A
- EP 0209700 A2
- DE 3522005
- EP 0000194 A1
- US 4160080 A
- EP 0682012 B1
- EP 1727846 B1
- EP 0712840 B1
- EP 0867457 B1
- DE 102007040240 A1
- EP 1645582 A1
- DE 102007040239 A1
- DE 102004060285 A1
- WO 2006063891 A1
- US 6670499 B1
- US 6670499 B
- JP 7206740 A
- EP 1112995 B1
- EP 0049631 B1
- DE 10352260 B3
- EP 0023686 B1
- DE 3338077 A1
- EP 0143613 B1
- WO 2009065873 A2
- DE 3941629 A1
- DE 19903177
- DE 4416857
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- US 4772530 A
- US 4954414 A
- US 4874450 A
- US 5055372 A
- US 5057393 A
- EP 1720506 A
- EP 1839640 A
- DE 1495520
- WO 02092021 A
- WO 02092023 A
- EP 0059451 A

- US 4490497 A
- EP 0980682 A1
- EP 0948955 A1
- EP 0783880 B1
- DE 10119831 A1
- EP 1563821 A1


**In der Beschreibung aufgeführte Nicht-Patentliteratur**


- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II
- **WASSERAUFNAHME ; LÖSLICHKEIT.** Water sorption and solubility of Methacrylate resin based root canal sealers. *Journal of Endodontics,* 2007, vol. 33 (8), 990-994